# EUROPEAN PATENT SPECIFICATION

(11) **EP 2 766 017 B1**
(45) Date of publication and mention of the grant of the patent: **01.07.2020**
(21) Application number: 12781519.9
(22) Date of filing: 04.10.2012
(51) Int. Cl.: A61K 31/44, A61P 9/10, A61P 25/00

(54) **METHODS FOR TREATING A STROKE-RELATED SENSORIMOTOR IMPAIRMENT USING AMINOPYRIDINES**
VERFAHREN ZUR BEHANDLUNG VON DURCH SCHLAGANFALL VERMITTELTEN SENSOMOTORISCHEN STÖRUNGEN MIT AMINOPYRIDINEN
PROCÉDÉS DE TRAITEMENT D'UNE DÉFICIENCE SENSORIMOTRICE LIÉE À UN ACCIDENT VASCULAIRE CÉRÉBRAL À L'AIDE D'AMINOPYRIDINES

(30) Priority: 04.10.2011 US 201161543150 P; 27.01.2012 US 201261591833 P; 18.05.2012 US 201261648695 P
(43) Date of publication of application: 20.08.2014
(73) Proprietor: Acorda Therapeutics, Inc., Ardsley, NY 10502 (US)
(72) Inventor: BLIGHT, Andrew, R., Mahopac, NY 10541 (US); CAGGIANO, Anthony, O., Larchmont, NY 10538 (US); PARRY, Tom, J., Hellertown, PA 18055 (US); IACI, Jennifer, F., Boonton, NJ 07005 (US)
(74) Representative: Carpmaels & Ransford LLP
(86) International application number: PCT/US2012/058607
(87) International publication number: WO 2013/052575

(56) References cited:
- WO-A1-89/09600
- WO-A1-2012/103471
- WO-A2-02/064126
- KR-B1- 100 951 540
- US-A1- 2002 022 587
- QUAN LILLY ET AL: "Selective effects of subarachnoid hemorrhage on cerebral vascular responses to 4-aminopyridine in rats", STROKE, vol. 31, no. 10, October 2000 (2000-10), pages 2460-2465, ISSN: 0039-2499
- UCHIHASHI YOSHITAKA ET AL: "Na+ channel block prevents the ischemia-induced release of norepinephrine from spinal cord slices", EUROPEAN JOURNAL OF PHARMACOLOGY, vol. 346, no. 2-3, 10 April 1998 (1998-04-10), pages 145-150, ISSN: 0014-2999

## Description

### 1. FIELD OF INVENTION

The invention relates to treatment of impairments, in particular, sensorimotor impairments, associated with stroke.

### 2. BACKGROUND

Central nervous system (CNS) injuries are a serious health problem. CNS injuries generally heal incompletely leaving the subject with some degree of permanent dysfunction. The residual dysfunction may include motor, sensory, cognitive, emotional and autonomic abnormalities.

A key category of CNS injury comprises brain injury consequent to stroke. Stroke is the third-leading cause of death and the main cause of disability in the western world. Stroke, therefore, presents a large socioeconomic burden. The etiology of a stroke can be either ischemic, which is the case in the majority of strokes, or hemorrhagic. An ischemic stroke can be caused by a clot that forms elsewhere in the body and travels via the bloodstream to the brain (embolic stroke) or by a blood clot that forms inside the artery of the brain (thrombotic stroke). After massive cell death in the immediate infarct core due to lack of glucose and oxygen, the infarct area expands for days, owing to secondary mechanisms such as glutamate excitotoxicity, apoptotic mechanisms, and generation of free radicals. Following neural injuries (e.g. an ischemic event) animals and people may recover function over several days, weeks and months without any therapeutic intervention. All too often, however, this recovery is only partial and animals and people suffer from long-term or permanent disability which may include motor, sensory and cognitive deficits. The motor, sensory and cognitive impairments due to stroke can have significant impact on activities of daily living and quality of life. Stroke survivors are often left with permanent neurological deficits, with an estimated 15-30% of stroke survivors becoming permanently disabled (Roger et al., Circulation 2012; 125:22-e220).

Risk factors that increase the likelihood of an individual having a stroke are well known. These include, and are not limited to, risk factors that cannot be changed: advanced age, heredity, race, gender, prior history of stroke or heart attack; and risk factors that can be changed, treated or controlled: high blood pressure, cigarette smoking, diabetes mellitus, carotid or other artery disease, atrial fibrillation, other heart disease, sickle cell disease, high blood cholesterol, poor diet, and physical inactivity and obesity.

To date, the direct pharmacotherapy of ischemic stroke is confined to drugs administered in the acute phase following a stroke. The acute phase ranges from the time of onset of the injury (e.g., stroke) to approximately six hours post-injury. There is currently no pharmacotherapy for hemorrhagic stroke.

Other than tissue plasminogen activator ("tPA") and certain mechanical clot retrieval devices suitable for acute use (see Eesa et al., 2011, Expert Rev Neurother. 11(8):1125-1139), presently there is no therapy approved in the U.S. for the treatment of stroke. After the available treatments, patients often remain with some level of dysfunction that at best may improve somewhat endogenously for approximately 60 days and then very slightly for up to a year or more. This recovery may only be augmented by physical therapy. Unfortunately, many patients are left with permanent disability with little hope for improvement.

Treatment of acute stroke is accomplished by restoring blood flow in the occluded vessel through the time-sensitive use of thrombolytics, specifically tPA. tPA disrupts the blood clot in the artery feeding blood to the brain, facilitating restoration of blood flow and oxygenation to the brain. However, only a small percentage of stroke patients receive successful tPA therapy: tPA is currently only FDA approved for use within 3 hours of the onset of stroke symptoms and is only given to about 3 percent of individuals with stroke. Many patients are not candidates for tPA therapy, do not arrive to the hospital in time for tPA, or have multiple small infarcts over time that cannot be treated with tPA. Furthermore, even those patients that are successfully treated with tPA often have some degree of cellular damage to the brain.

tPA is a serine protease that converts plasminogen to plasmin. Plasmin then breaks fibrin which is a component of the clots that occlude the vessels in the brain and cause strokes. Ideally, tPA is administered within the first three hours post-occlusion, but may be administered by some clinicians as late as six hours post-occlusion. Unfortunately, the vast majority of patients who experience a stroke fail to reach the hospital in time to be considered for this treatment. For those patients who arrive at the hospital within the efficacious temporal window, tPA is administered in an attempt to reverse the occlusion of blood flow, restore oxygenation of the brain and limit the extent of lost brain structure. However, there are some significant contraindications that limit the use of tPA. Patients receiving tPA after 3 hours are at an increased risk of serious bleeding while the effectiveness of tPA is diminishing. For such reasons, tPA is limited to administration during the acute phase in order to achieve any therapeutic efficacy.

To date, no other drugs have been approved by the FDA for the treatment of stroke. Current experimental therapies such as arterially delivered pro-urokinase are under investigation as potential means for disrupting clots and restoring blood flow. The scientific literature has, however, described agents that have proven beneficial for protecting brain matter and restoring function in experimental animal models of stroke. Most of these agents focus on reducing acute cell death, inflammation, and apoptosis and must, therefore, be delivered within hours (some up to 24 hours) after the ischemic event.

Aspirin (ASA) is also recommended by several organizations when individuals are suffering stroke symptoms. Some other anti-platelet therapies are used to help reduce the likelihood of stroke.

Heretofore, it is generally accepted that treatment for stroke is required acutely (Abe et al., 2008, J Cereb Blood Flow Metab. Jul 23, Epub ahead of print, Sun et al., 2008, Stroke Jul 10, Epub ahead of print; Dohare et al., 2008, Behav Brain Res. 193(2):289-97; Belayev et al., 2001, Stroke 32(2):553-60). With few exceptions, for example with the exception of glial growth factor 2 (GGF2) (see Iaci et al., 2010, Neuropharmacology 59:640-649), agents have not been shown to limit damage to the brain, restore function or enhance recovery following stroke when administered after a lag time of several hours, and at most, in some experimental animal models, about one day following stroke.

After an acute occlusion, there is often a localized area of destroyed brain matter that is surrounded by a penumbral zone that will die within hours if circulation is not restored. The time to death of this penumbral zone can be extended by a few hours in experimental models with neuroprotectants, such as NMDA antagonists, calcium channel blockers, radical scavengers and trapping agents, anti-apoptotics, caspase inhibitors, parp inhibitors, etc. After 24 to 48 hours, however, there is little hope for protecting cells from necrotic death and, while apoptotic death continues for several days, the therapeutic window for anti-apoptotic therapies has not proven to be much wider than acute protective therapies (Schulz et al., 1998, Cell Death Differ. 5(10):847-57; Komjati et al., 2004, Int J Mol Med. 13(3):373-82).

The permanent sensorimotor deficits in the individuals who survive a stroke are usually only partially addressed by rehabilitation with physical therapy. Despite this there has been little attention to the potential for pharmacological intervention to treat permanent functional deficits in such patients. This may be due to the generally held belief that not much can be done to replace nerve cells and circuits that have been lost as a result of stroke.

### Potassium Channel Blockers

An exemplary property of certain aminopyridines is that they are potassium channel blockers. 4-aminopyridine (4-AP) is an example of an aminopyridine with such potassium channel blocking properties. At 4-AP plasma concentrations obtained in clinical studies, which are typically <1 microM (94 ng/mL⁻¹), the potassium channel blocking activity of 4-AP appears to be selective for certain types of these channels. Interestingly, at high concentration (such as at millimolar concentrations) 4-AP is a broad-spectrum blocker of potassium channels. The clinical neurologic effects of 4-AP are consistent with the molecular mechanism of potassium channel blockade.

Studies of 4-aminopyridine (dalfampridine, fampridine) have been conducted using intravenous (i.v.) administration and immediate-release (IR) oral capsule formulations in addition to controlled-release or sustained-release formulations. Administration of IR capsules resulted in rapid and short-lasting peaks of 4-aminopyridine in the plasma. Early pharmacokinetic studies were conducted using an immediate release (IR) formulation for oral administration, which consisted of 4-aminopyridine powder in a gelatin-based capsule or oral solution. Administration resulted in rapidly changing 4-aminopyridine plasma levels that were not well tolerated. A sustained-release matrix tablet (known as Fampridine-SR or AMPYRA®, Acorda Therapeutics, Hawthorne, NY) was then developed. The sustained release matrix tablet showed improved stability and an appropriate pharmacokinetic profile for twice-daily dosing. Sustained release compositions of 4-aminopyridine and related use of such compositions are set forth, e.g., in US Patent 5,370,879, US Patent 5,540,938; US Patent 8,007,826; and US Patent Publication US2005-0228030. For example, suitable formulations, methods of manufacture, pharmacokinetic characteristics of sustained release aminopyridine compositions and methods of treating various neurological disorders are further described in U.S. Patent No. 8,007,826 entitled "Sustained Release Aminopyridine Composition" issued on August 30, 2011; and U.S. Patent Publication No. 2005-0228030 entitled "Methods of Using Sustained Release Aminopyridine Compositions" published on October 13, 2005.

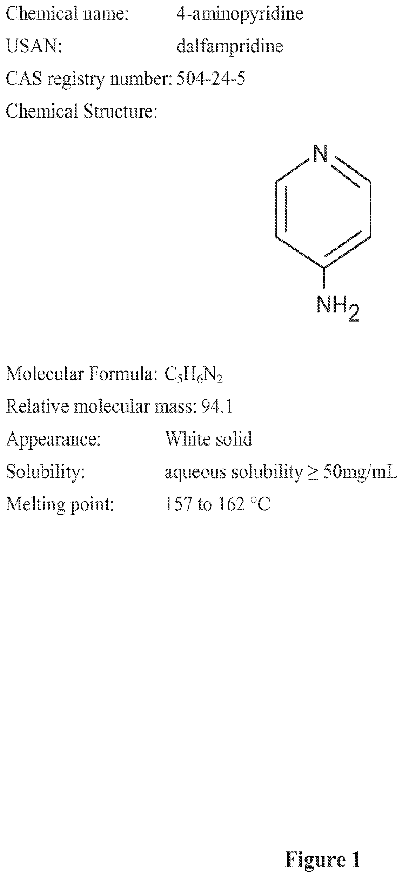

The compound 4-aminopyridine is a potassium (K+) channel blocker approved by the U.S. Food and Drug Administration as a treatment for patients with MS. As set forth in Figure 1, dalfampridine is the United States Adopted Name (USAN) for the chemical 4-aminopyridine (4AP), which has a molecular formula of C₅H₆N₂ and molecular weight of 94.1; the former USAN name for this compound was fampridine (which remains the International Nonproprietary Name). The terms "dalfampridine", "fampridine" and "4-aminopyridine" will be used throughout this specification to refer to the active drug substance.

International Publication No. WO 89/09600 discloses use of a combination of a potassium channel blocker and choline or a source of choline to treat certain diseases including "post-stroke or post-toxic syndromes affecting memory or cognition" (see p. 6). Quan et al., 2000, Stroke 31:2460-2465 describes the use of pharmacological inhibitors to assess the influence of subarachnoid haemorrhage on function of 2 types of K+ channel in regulation of basilar artery diameter in vivo and membrane potential (Eₘ) in vitro. Uchihashi et al., 1998, European Journal of Pharmacology 346:145-150 describes the [Ca²⁺]ₒ-independent effect of ischemia on norepinephrine release and its antagonism by tetrodotoxin and low temperature (10°C). US 2002/0022587 A1 describes methods for preventing damage to excitable cells following ischemia by administering to a patient, who is undergoing or who has undergone an ischemic event, an effective amount of a compound which increases a transient potassium (K+) conductance in the excitable cells of the patient. WO 02/064126 A2 describes N-(pyridinyl)-1H-indol-1-amines providing a unique combination of blocking properties for both the potassium and sodium channels that are useful for the treatment of demyelinating diseases and conditions such as multiple sclerosis, spinal cord injury, traumatic brain injury and stroke and also for stroke rehabilitation, the treatment of bladder irritation and dysfunction and the treatment of neuropathic pain and chemokine-induced pain.

There is long-standing unmet need in the art to effectively treat impairments induced by a stroke, e.g., sensorimotor impairments. In particular, there is a need for such treatment in delayed, non-acute situations that are more than hours, days or weeks following an acute injury. In addition to treatment outside of the acute period, there is a significant unmet medical need for any therapy that can be delivered in the chronic phase that will improve sensory, motor, cognitive, emotional or autonomic function.

### 3. BRIEF SUMMARY OF THE INVENTION

The invention relates to an aminopyridine or a pharmaceutically acceptable salt thereof for use in treating a stroke-related sensorimotor impairment in a human patient who has suffered a stroke, wherein the aminopyridine has the following structure: 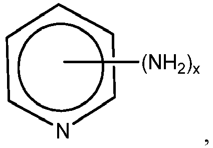 wherein x is 1 or 2.

Disclosedherein are methods for treatment of a patient who has suffered a stroke by administering a therapeutically effective amount of an aminopyridine or a pharmaceutically acceptable salt thereof. Alsodisclosed herein is treatment of a stroke-related impairment in a patient who had a stroke. In particular, disclosed herein is treatment that causes improvement in one or more sensorimotor impairments related to or induced by a stroke. In particular, the use of aminopyridines in such treatments is disclosed. In one embodiment, one or more aminopyridines are used in the methods disclosed herein. In one embodiment, the aminopyridine is a mono- or di-aminopyridine. In some embodiments, the mono- aminopyridine is 3-aminopyridine or 4-aminopyridine. In one embodiment, the di- aminopyridine is 3,4-diaminopyridine.

In certain embodiments, the patient treated in accordance with the methods described herein is a mammal. In a preferred embodiment, the patient treated in accordance with the methods described herein is a human. In certain embodiments, the stroke treated in accordance with the invention is an ischemic stroke. Subtypes of ischemic stroke that can be treated in accordance with the invention include, without limitation, large-artery atherosclerosis (embolus/thrombosis), cardioembolism (cardioembolic stroke), small-vessel occlusion (lacunar stroke), as well as stroke of other determined or undetermined etiology. In certain embodiments, the stroke treated in accordance with the invention is associated with non-atherosclerotic vasculopathies, hypercoagulable states or hematologic disorders. In another embodiment, the stroke treated in accordance with the invention is a hemorrhagic stroke. Subtypes of hemorrhagic stroke that can be treated in accordance with the invention include, without limitation, subarachnoid hemorrhage and intracerebral hemorrhage.

In some embodiments, an aminopyridine or a pharmaceutically acceptable salt thereof is administered in a sustained release composition. In other embodiments, an aminopyridine or a pharmaceutically acceptable salt thereof is administered in an immediate release composition. Described herein is a method comprising administering an aminopyridine or a pharmaceutically acceptable salt thereof once daily, twice daily or thrice daily. In a specific embodiment, an aminopyridine (e.g., 4-AP) or a pharmaceutically acceptable salt thereof is in a sustained release composition, and is administered once or twice daily, preferably orally. In another specific embodiment, an aminopyridine (e.g., 4-AP) or a pharmaceutically acceptable salt thereof is in an immediate release composition, and is administered three times or more than three times daily, preferably orally.

In specific embodiments, an aminopyridine itself, and not a pharmaceutically acceptable salt thereof, is used in any of the methods described herein.

In certain embodiments, an aminopyridine or a pharmaceutically acceptable salt thereof, preferably a therapeutically effective amount of the aminopyridine or salt, is administered to the patient orally, intravenously, intramuscularly or subcutaneously. In one embodiment, an aminopyridine or a pharmaceutically acceptable salt thereof is administered to the patient orally. In some of the embodiments wherein an aminopyridine or a pharmaceutically acceptable salt thereof is administered orally, it is formulated in a form of a tablet, a pill or a capsule. In one embodiment, an aminopyridine or a pharmaceutically acceptable salt thereof is administered to the patient intravenously.

In a specific embodiment, an aminopyridine or a pharmaceutically acceptable salt, preferably a therapeutically effective amount of the aminopyridine or salt, is administered to the patient orally, in a sustained release composition b.i.d. (i.e., twice daily). In certain embodiments, twice daily administration comprises administration of an aminopyridine or a pharmaceutically acceptable salt thereof every 12 hours. In a specific embodiment, an aminopyridine or a pharmaceutically acceptable salt thereof in a sustained release composition provides a Tₘₐₓ of about 2 hours to about 6 hours in a human. In another specific embodiment, an aminopyridine or a pharmaceutically acceptable salt thereof, preferably a therapeutically effective amount of the aminopyridine or salt, is administered to the patient orally, in a sustained release composition once daily.

In certain embodiments, an aminopyridine or a pharmaceutically acceptable salt thereof is administered in an amount in the range between about 4 mg and about 17.5 mg, or from 4 mg to 17.5 mg, (e.g., about 4, 5, 6, 7, 7.5, 8, 9, 10, 11, 12, 12.5, 13, 14, 15, 16, 17, or 17.5 mg), which in specific embodiments, is once or twice daily, preferably in a sustained release composition. In certain embodiments, an aminopyridine or a pharmaceutically acceptable salt thereof is administered in an amount in the range between about 8 mg and about 30 mg, or from 8 mg to 30 mg, (e.g., about 8, 9, 10, 11, 12, 12.5, 13, 14, 15, 16, 17, 17.5, 18, 19, 20, 21, 22, 23, 24, 25, 26, 27, 28, 29 or 30 mg), which in specific embodiments, is once or twice daily, preferably in a sustained release composition. In some embodiments, an aminopyridine or a pharmaceutically acceptable salt thereof is administered in an amount in the range from 4 mg to 40 mg. In some embodiments, an aminopyridine or a pharmaceutically acceptable salt thereof is administered in an amount between about 5 mg and 15 mg, 5 mg and 10 mg, 5 mg and 7.5 mg, or 7.5 mg and 10 mg twice daily, preferably in a sustained release composition, or from about 5 mg to 15 mg, 5 mg to 10 mg, 5 mg to 7.5 mg, or 7.5 mg to 10 mg twice daily, preferably in a sustained release composition. In one embodiment, an aminopyridine or a pharmaceutically acceptable salt thereof is administered at a dose of 5 mg twice daily, preferably in a sustained release composition. In another embodiment, an aminopyridine or a pharmaceutically acceptable salt thereof is administered at a dose of 10 mg twice daily, preferably in a sustained release composition. In another embodiment, an aminopyridine or a pharmaceutically acceptable salt thereof is administered at a dose of 10 mg once daily, preferably in a sustained release composition. In some of these embodiments, the aminopyridine is 4-aminopyridine. In other embodiments, an aminopyridine or a pharmaceutically acceptable salt thereof is administered between about 8 mg and 30 mg, 8 mg and 20 mg, 10 mg and 15 mg, or 10 mg and 20 mg once daily (e.g., in a sustained release composition).

Any of the dosages and dosage regimens described in this application can serve as the therapeutically effective amount of aminopyridine or pharmaceutically acceptable salt thereof used in the methods described herein.

Also described herein is a method comprising administering an aminopyridine or a pharmaceutically acceptable salt thereof during the early chronic phase and/or during the stable chronic phase following a stroke. Also described is a method comprising administering an aminopyridine or a pharmaceutically acceptable salt thereof during the acute phase following a stroke. In certain embodiments, treatment is initiated during the acute phase, and continues during the early chronic phase and/or the stable chronic phase post-stroke. In some embodiments, treatment is initiated during the early chronic phase and continues during the stable chronic phase. In one embodiment, treatment is initiated during the stable chronic phase. In specific embodiments, treatment is initiated during the period post-stroke when spontaneous recovery of sensorimotor functions is expected or observed in a patient. In other specific embodiments, treatment is initiated during the period post-stroke when little or no measureable spontaneous recovery of, or improvement in, sensorimotor functions is expected or observed in a patient. In some embodiments, treatment is initiated during the period post-stroke when spontaneous recovery of sensorimotor functions is expected or observed in a patient, but continues for any period of time beyond such period (e.g., continues for 6 months, 1 year, 5 years, 10 years, 20 years beyond such period, or continues for the lifetime of the treated patient).

In certain embodiments, treatment in accordance with the invention begins at or after 1, 2, 3, 4, 5, 6, 7, 8, 9, 10, 11, 12, 13, 14, 15, 16, 17, 18, 19, 20, 21, 22, 23, 24, 25, 26, 27 or 28 days; 1, 2, 3, 4, 5, 6, 7, 8, 9, 10, 11, or 12 weeks; or 1, 2, 3, 4, 5, 6, 7, 8, 9, 10, 11, 12 months post-stroke. In certain embodiments, treatment in accordance with the invention continues for more than 1, 2, 3, 4, 5, 6, 7, 8, 9, 10, 11, or 12 weeks; 1, 2, 3, 4, 5, 6, 7, 8, 9, 10, 11, or 12 months; or 1, 2, 3, 4, 5, 6, 7, 8, 9, or 10 years since the commencement of treatment. In some embodiment, treatment in accordance with the invention begins any time post-stroke.

In one embodiment, the step of administering of an aminopyridine or a pharmaceutically acceptable salt thereof, preferably a therapeutically effective amount of the aminopyridine or salt, begins at least 4, 5, 6, 7, 8, 9, 10, 11 or 12 months from the time the patient had a stroke. In one embodiment, the step of administering of an aminopyridine or a pharmaceutically acceptable salt thereof, preferably a therapeutically effective amount of the aminopyridine or salt, begins more than 4, 5, 6, 7, 8, 9, 10, 11 or 12 months from the time the patient had a stroke. In one embodiment, the step of administering of an aminopyridine or a pharmaceutically acceptable salt thereof, preferably a therapeutically effective amount of the aminopyridine or salt, begins at least 8 weeks from the time the patient had a stroke. In one embodiment, the step of administering of an aminopyridine or a pharmaceutically acceptable salt thereof, preferably a therapeutically effective amount of the aminopyridine or salt, begins at least 4 weeks from the time the patient had a stroke. In another embodiment, the step of administering of an aminopyridine or a pharmaceutically acceptable salt thereof, preferably a therapeutically effective amount of the aminopyridine or salt, begins at least 1 week from the time the patient had a stroke. In yet another embodiment, the step of administering of an aminopyridine or a pharmaceutically acceptable salt thereof, preferably a therapeutically effective amount of the aminopyridine or salt, begins between 2 and 7 days from the time the patient had a stroke. In some embodiments, the step of administering of an aminopyridine or a pharmaceutically acceptable salt thereof, preferably a therapeutically effective amount of the aminopyridine or salt, begins at least 1 week, 2 weeks, 3 weeks, 4 weeks, 5 weeks, 6 weeks, 7 weeks, or 8 weeks from the time the patient had a stroke. In one embodiment, the step of administering of an aminopyridine or a pharmaceutically acceptable salt thereof, preferably a therapeutically effective amount of the aminopyridine or salt, begins at least 4 months from the time the patient had a stroke. In one embodiment, the step of administering of an aminopyridine or a pharmaceutically acceptable salt thereof, preferably a therapeutically effective amount of the aminopyridine or salt, begins at least 6 months from the time the patient had a stroke. In one embodiment, the step of administering of an aminopyridine or a pharmaceutically acceptable salt thereof, preferably a therapeutically effective amount of the aminopyridine or salt, begins at least 8 months from the time the patient had a stroke. In one embodiment, the step of administering of an aminopyridine or a pharmaceutically acceptable salt thereof, preferably a therapeutically effective amount of the aminopyridine or salt, begins at least 12 months from the time the patient had a stroke. In one embodiment, the step of administering of an aminopyridine or a pharmaceutically acceptable salt thereof, preferably a therapeutically effective amount of the aminopyridine or salt, begins more than 4 months from the time the patient had a stroke. In one embodiment, the step of administering of an aminopyridine or a pharmaceutically acceptable salt thereof, preferably a therapeutically effective amount of the aminopyridine or salt, begins more than 6 months from the time the patient had a stroke.

In particular embodiments, the step of administering of an aminopyridine or a pharmaceutically acceptable salt thereof, preferably a therapeutically effective amount of the aminopyridine or salt, begins at least 3, 6, 7, 8, 9, 10, 11, 12, 13, 14, 15, 16, 17, 18, 19, 20, 21, 22, 23, 24 or 48 hours from the time the patient had a stroke. In one embodiment, the step of administering of an aminopyridine or a pharmaceutically acceptable salt thereof, preferably a therapeutically effective amount of the aminopyridine or salt, begins at least 6 hours from the time the patient had a stroke. In one embodiment, the step of administering of an aminopyridine or a pharmaceutically acceptable salt thereof, preferably a therapeutically effective amount of the aminopyridine or salt, begins at least 12 hours from the time the patient had a stroke. In another embodiment, the step of administering of an aminopyridine or a pharmaceutically acceptable salt thereof, preferably a therapeutically effective amount of the aminopyridine or salt, begins at least 24 hours from the time the patient had a stroke. In another embodiment, the step of administering of an aminopyridine or a pharmaceutically acceptable salt thereof, preferably a therapeutically effective amount of the aminopyridine or salt, begins at least 48 hours from the time the patient had a stroke. In one embodiment, the step of administering of an aminopyridine or a pharmaceutically acceptable salt thereof, preferably a therapeutically effective amount of the aminopyridine or salt, begins more than 6 hours from the time the patient had a stroke. In another embodiment, the step of administering of an aminopyridine or a pharmaceutically acceptable salt thereof, preferably a therapeutically effective amount of the aminopyridine or salt, begins more than 24 hours from the time the patient had a stroke.

In another embodiment, the step of administering of an aminopyridine or a pharmaceutically acceptable salt thereof, preferably a therapeutically effective amount of the aminopyridine or salt, begins immediately following a stroke or within 1 hour, 2 hours, 3 hours, 4 hours, 6 hours, 8 hours, 10 hours, 12 hours, 16 hours, 20 hours, 24 hours, 36 hours or 48 hours from the time the patient had a stroke. In specific embodiments, the step of administering of an aminopyridine or a pharmaceutically acceptable salt thereof, preferably a therapeutically effective amount of the aminopyridine or salt, begins within 1 day or within 2 days from the time the patient had a stroke. In certain embodiments, treatment in accordance with the invention begins immediately after the stroke or within 1, 2, 3, 4, 5, 6, 7, 8, 9, 10, 11, 12, 13, 14, 15, 16, 17, 18, 19, 20, 21, 22, 23, 24, 36 or 48 hours following a stroke, and continues for at least 1, 2, 3, 4, 5, 6, 7, 8, 9, 10, 11, or 12 weeks; 1, 2, 3, 4, 5, 6, 7, 8, 9, 10, 11, or 12 months; or 1, 2, 3, 4, 5, 6, 7, 8, 9, or 10 years since the commencement of treatment.

Described herein are methods for treating one or more sensorimotor impairments in a patient who has suffered a stroke. Also described herein are methods for treating one or more motor or sensory impairments in a patient who has suffered a stroke. Sensorimotor impairments treated in accordance with the methods described herein include, without limitation: ataxia, global body control impairments, coordination or balance impairments, impairment in body sense, endurance impairment, impairment in hand function, fine hand coordination loss or impairment, hyperreflexia, impairment in grip strength, impairment in hand strength, impairment in manual dexterity, muscle weakness, muscle tone impairment, range of motion impairment, spasticity, strength impairment/weakness, tremor, impairment in limb function, upper extremity function impairment, lower extremity function impairment, impairment in lower extremity muscle strength, walking impairments (e.g., decreased walking speed or abnormal gait), speech impairments (e.g., dysarthria), impairment in jaw function, impairment in chewing, and impairment in jaw articulation.

In one embodiment, the sensorimotor impairment treated in accordance with the invention is an impairment in walking such as a decreased walking speed. In one embodiment, the sensorimotor impairment treated in accordance with the methods described herein is an impairment in proprioception. In other embodiments, the sensorimotor impairment treated in accordance with the invention is an impairment in global body control or body sense. In another embodiment, the sensorimotor impairment treated in accordance with the invention is an impairment in limb function (e.g., an impairment in lower extremity function, an impairment in lower extremity muscle strength, or an impairment in upper extremity function). In one embodiment, the sensorimotor impairment treated in accordance with the invention is an impairment in lower extremity function and/or lower extremity muscle strength. In one embodiment, the sensorimotor impairment treated in accordance with the invention is an impairment in upper extremity function. In one embodiment, the sensorimotor impairment treated in accordance with the invention is an impairment in upper limb spasticity. In yet another embodiment, the sensorimotor impairment treated in accordance with the invention is an impairment in hand function, an impairment in fine hand coordination, or an impairment in grip strength. In another embodiment, the sensorimotor impairment treated in accordance with the invention is an impairment in hand strength. In another embodiment, the sensorimotor impairment treated in accordance with the invention is an impairment in manual dexterity. In a particular embodiment, the sensorimotor impairment treated in accordance with the invention is an impairment in oral motor functioning. In a particular embodiment, the sensorimotor impairment treated in accordance with the invention is a speech impairment (e.g., dysarthria, apraxia, or dysphonia). In a particular embodiment, the sensorimotor impairment treated in accordance with the invention is an impairment in chewing and/or swallowing (e.g., dysphagia). In a particular embodiment, the sensorimotor impairment treated in accordance with the invention is facial paralysis. In a particular embodiment, the sensorimotor impairment treated in accordance with the invention is limb paralysis. In a particular embodiment, the sensorimotor impairment treated in accordance with the invention is hand paralysis. In one embodiment, the sensorimotor impairment treated in accordance with the invention is an impairment in balance. In one embodiment, the sensorimotor impairment treated in accordance with the invention is an impairment in sensation. In some embodiments, the sensorimotor impairment treated in accordance with the invention is a visual impairment, such as a sensory and/or ocular motor impairment of visual function.

In specific embodiments, the treatment in accordance with the invention is effective to treat (e.g., improve, ameliorate, reduce the severity of, or reduce the duration of) the symptoms of one or more stroke-related sensorimotor impairments. In some embodiments, the treatment in accordance with the invention is effective to treat (e.g., improve, ameliorate, reduce the severity of, or reduce the duration of) the symptoms of one or more stroke-related motor impairments. In some embodiments, the treatment in accordance with the invention is effective to treat (e.g., improve, ameliorate, reduce the severity of, or reduce the duration of) the symptoms of one or more stroke-related sensory impairments. In some embodiments, the treatment in accordance with the invention restores one or more motor, sensory or sensorimotor functions impaired due to stroke. Also described are methods for assessing the level of said motor, sensory or sensorimotor impairment after (or before and after) repeated administration of an aminopyridine. Such method can be any method for evaluating motor, sensory or sensorimotor function described herein or known in the art.

In some of the embodiments, the therapeutically effective amount of an aminopyridine or a pharmaceutically acceptable salt thereof used in the methods described herein is such that a Cₘᵢₙₛₛ or average Cₘᵢₙₛₛ of at least about 11, 12, 13, 14, 15, 16, 17, 18, 19 or 20 ng/ml is obtained in a human. In one embodiment, the therapeutically effective amount of an aminopyridine or a pharmaceutically acceptable salt thereof is such that a Cₘᵢₙₛₛ or average Cₘᵢₙₛₛ in a range of about 12 ng/ml to 20 ng/ml is obtained in a human. In some of these embodiments, the aminopyridine is 4-aminopyridine.

In specific disclosures, any of the methods, dosages, and dosage regimens described in this application can be used to treat a patient with stable motor deficits following a stroke.

### 3.1 Terminology

In order to provide a clear and consistent understanding of the specification and claims, the following definitions are provided:

As used herein, the term "about" comprises the specified value plus or minus 1, 2, 3, 4, 5, 6, 7, 8, 9, 10, 11, 12, 13, 14, or 15 % of the specified value. In one embodiment "about" signifies 98-102% of the specified value. In one embodiment "about" signifies 95-105% of the specified value. In particular, however, a value "about" a particular ng/ml includes plus or minus 0.6, 0.5. 0.4, 0.3, 0.2 or 0.1 ng/ml. The meaning of the term "about" will be clear depending on the context in which it appears.

As used herein, if no fluid is mentioned or the context does not indicate otherwise, Cₘᵢₙₛₛ, Cₘₐₓₛₛ, Cₐᵥₛₛ values generally relate to blood plasma.

The term "improvement" with respect to an impairment designates an alteration in a parameter in a therapeutic direction. As used herein, "improvement" also comprises stabilization of a parameter that would otherwise be deteriorating or moving in a non-therapeutic direction.

By "pharmaceutically acceptable", it is meant the carrier, diluent or excipient must be compatible with the other ingredients of the formulation and not prohibited for human or veterinary administration (as the case may be) by a regulatory agency such as the Food and Drug Administration or European Medicines Agency.

The term "pharmaceutically acceptable salt(s)," with reference to an aminopyridine, as used herein, refers to a salt prepared from a pharmaceutically acceptable non-toxic acid or base, including an inorganic acid or base, or an organic acid or base. In one embodiment, the pharmaceutically acceptable salt is prepared from a pharmaceutically acceptable non-toxic acid which can be an inorganic or organic acid. In one embodiment, non-toxic acids include, but are not limited to, inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. In one embodiment, the non-toxic acid is hydrochloric acid. Suitable pharmaceutically acceptable salts will be apparent to those skilled in the art and include those described in S.M. Barge et al., "Pharmaceutical Salts," 1977, J. Pharm. Sci. 66:1-19.

As used herein, the term "steady state" indicates a system that has one or more properties that are unchanging over time or "steady state" indicates a system that has one or more properties that are changing within a limited range over time. Typically, steady state is a more general situation than dynamic equilibrium. If a system is in steady state, then the recently observed behavior of the system will generally continue into the future. In many systems, steady state is not achieved until some time has elapsed after the system is started or initiated. This initial situation is often identified as a transient state, titration period, start-up or warm-up period.

As used herein "stroke" can also be referred to as a "brain attack." Stroke occurs when blood flow to a region of the brain is obstructed, so the supply of oxygen and nutrients to brain cells are cut off, causing parts of cells to die. There are two main types of stroke: ischemic and hemorrhagic. Ischemic stroke is resulted from a blockage of the blood flow to the brain (ischemia) and almost always caused by a blood clot blocking a blood vessel, while hemorrhagic stroke results from bleeding (hemorrhage) of a ruptured blood vessel.

Other terms and/or abbreviations are provided below:

| **Abbreviation or Specialist Term** | **Explanation** |
|---|---|
| b.i.d. (bid) | Twice daily |
| Cm | Centimeter |
| Cₘₐₓ | Maximum measured plasma concentration |
| Cₘₐₓₛₛ | Maximum measured plasma concentration at steady state |
| Cₘᵢₙ | Minimum measured plasma concentration |
| Cₘᵢₙₛₛ | Minimum measured plasma concentration at steady state |
| CNS | Central nervous system |
| Dalfampridine | Fampridine, 4-aminopyridine |
| DAP | di-aminopyridine |
| Fampridine | Dalfampridine, 4-aminopyridine |
| g, kg, mg, µg, ng | Gram, kilogram, milligram, microgram, nanogram |
| GLP | Good Laboratory Practice |
| h, hr | Hour |
| HPLC | High performance liquid chromatography |
| IR | Immediate-release |
| IV, i.v., or iv | Intravenous |
| K⁺ | Ionic Potassium |
| L, mL | Liter, milliliter |
| LEMMT | Lower Extremity Manual Muscle Test |
| LCMS, LC/MS/MS | Liquid chromatography/ mass spectrometry |
| MCAO | Middle Cerebral Artery Occlusion |
| Min | Minute |
| mM, µM | Millimolar, micromolar |
| MS | Multiple sclerosis |
| MSWS-12 | 12-item Multiple Sclerosis Walking Scale |
| NF | National Formulary |
| p.o. | Oral |
| q.d. (qd) | Once a day |
| SR | Sustained-release |
| SS | Steady state |
| T25FW | Timed 25 Foot Walk |
| t.i.d. (tid) | Three times daily |
| Tₘₐₓ | Time of the maximum measured plasma concentration post-dose |
| USP | United States Pharmacopeia |
| WS | Walking speed |
| 3AP, or 3-AP | 3 -aminopyridine |
| 4AP, or 4-AP | 4-aminopyridine |
| 3,4 DAP, or 3,4-DAP | 3,4, di-aminopyridine |

### 4. BRIEF DESCRIPTION OF DRAWINGS

**Figure 1** shows information regarding 4-aminopyridine.

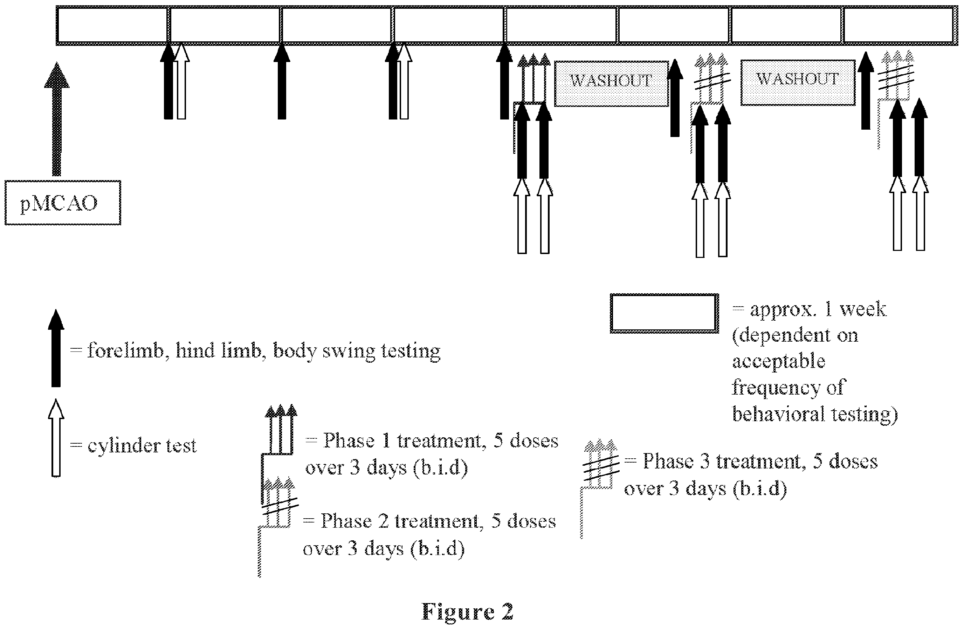

**Figure 2** is a schematic showing the timetable of dosing and behavior testing.

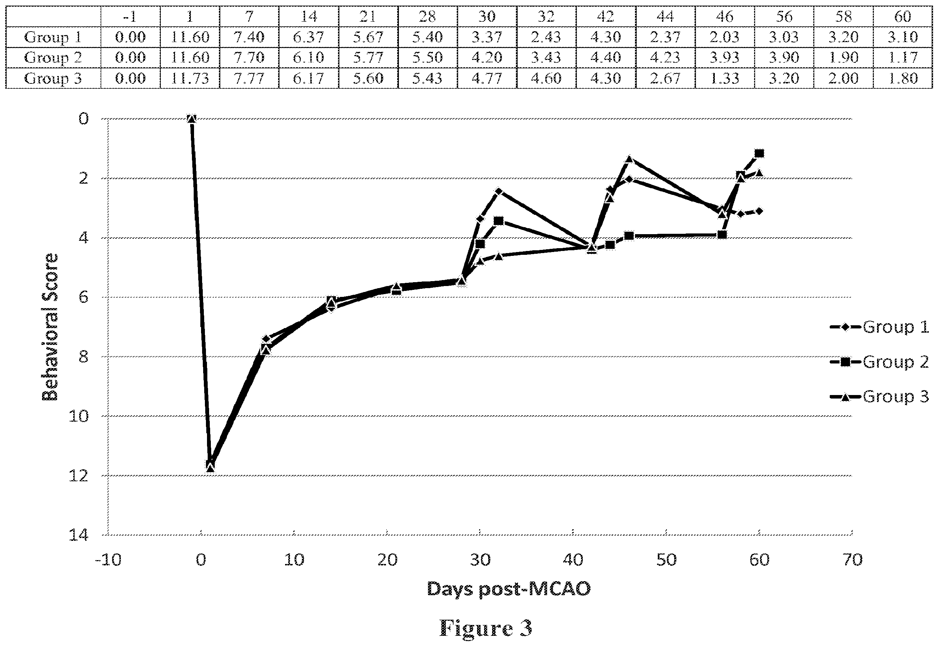

**Figure 3** shows the results of the forelimb placing test: X axis represents the number of days after the stroke event (i.e., days post-MCAO). The Y axis represents the behavioral score (0 to 12, with 0 being normal function and 12 being maximally impaired). The graph shows an average behavioral score of the animals in each test group (i.e., Groups 1-3) at D-1, D1, D7, D14, D21, D28, D30, D32, D42, D44, D46, D56, D58, D60 as described in the Examples ("D"=day).

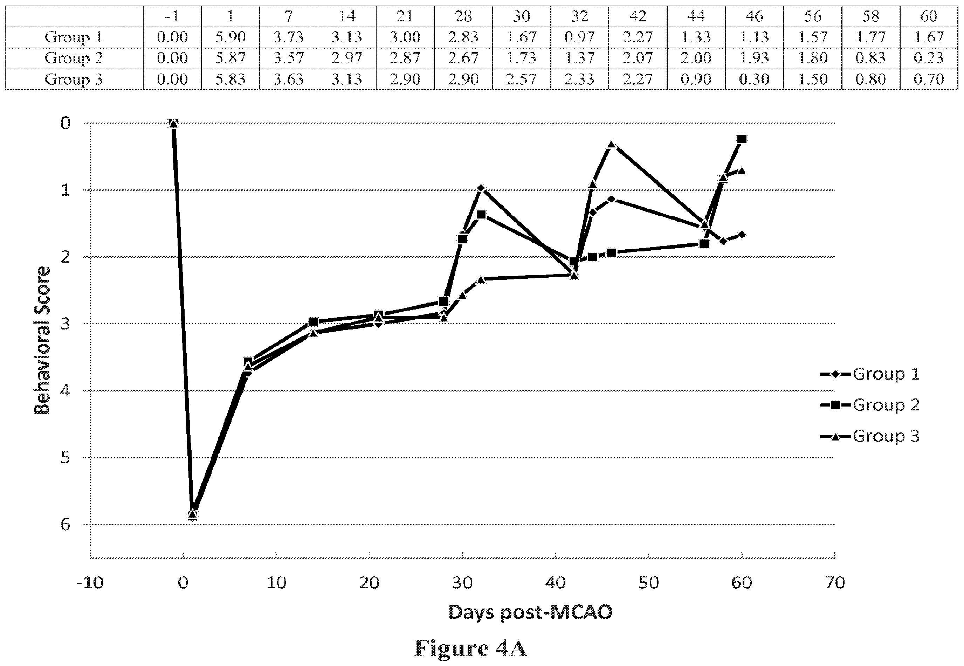

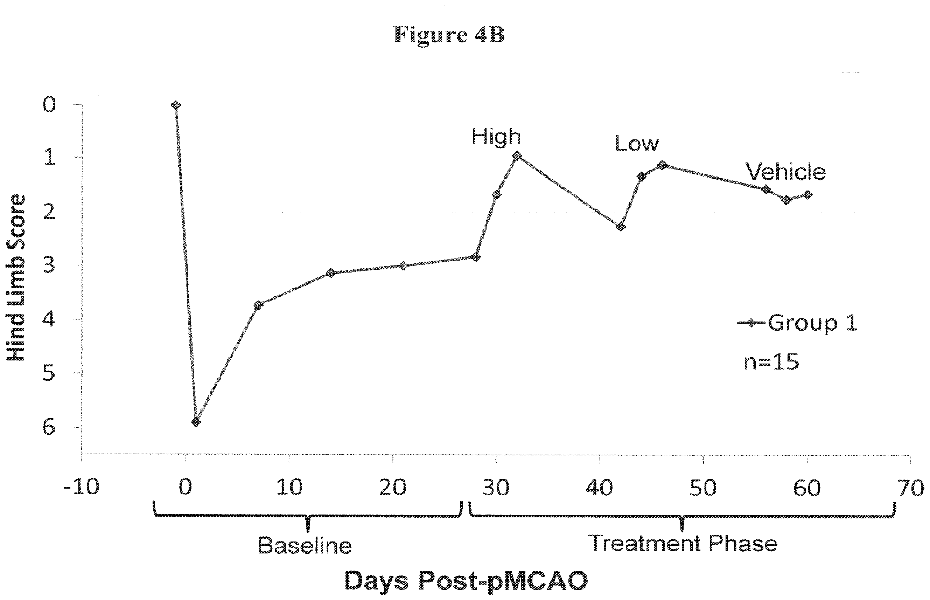

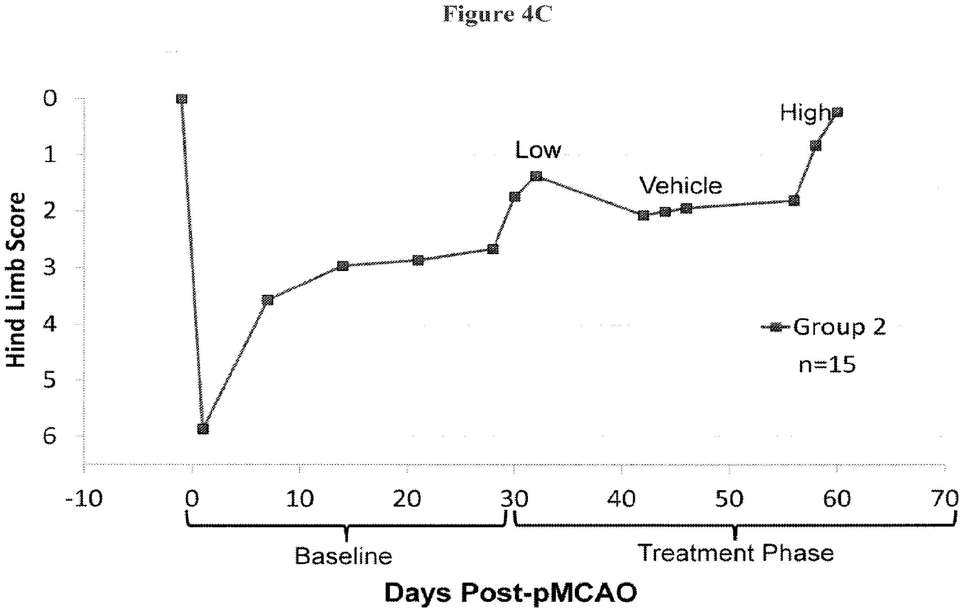

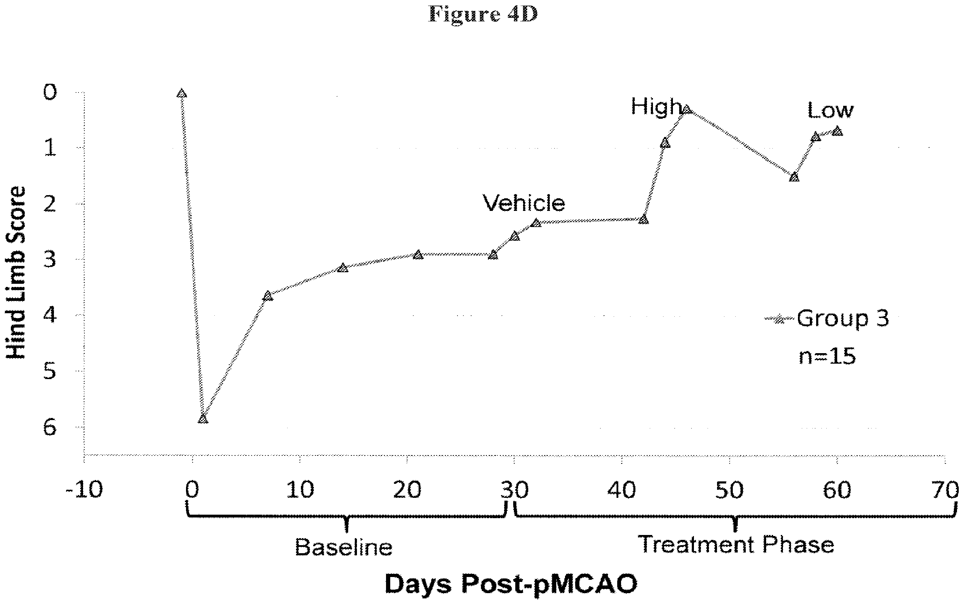

**Figures 4A-D** show the results of the hindlimb placing test: X axis represents the number of days after the stroke event (i.e., days post-MCAO). The Y axis represents the behavioral score (0 to 6, with 0 being normal function and 6 being maximally impaired). Figure 4A shows an average behavioral score of the animals in each test group (i.e., Groups 1-3) at D-1, D1, D7, D14, D21, D28, D30, D32, D42, D44, D46, D56, D58, D60 as described in the Examples. Figure 4B shows an average behavioral score of the animals in Group 1 at D-1, D1, D7, D14, D21, D28, D30, D32, D42, D44, D46, D56, D58, D60 as described in the Examples. Figure 4C shows an average behavioral score of the animals in Group 2 at D-1, D1, D7, D14, D21, D28, D30, D32, D42, D44, D46, D56, D58, D60 as described in the Examples. Figure 4D shows an average behavioral score of the animals in Group 3 at D-1, D1, D7, D14, D21, D28, D30, D32, D42, D44, D46, D56, D58, D60 as described in the Examples.

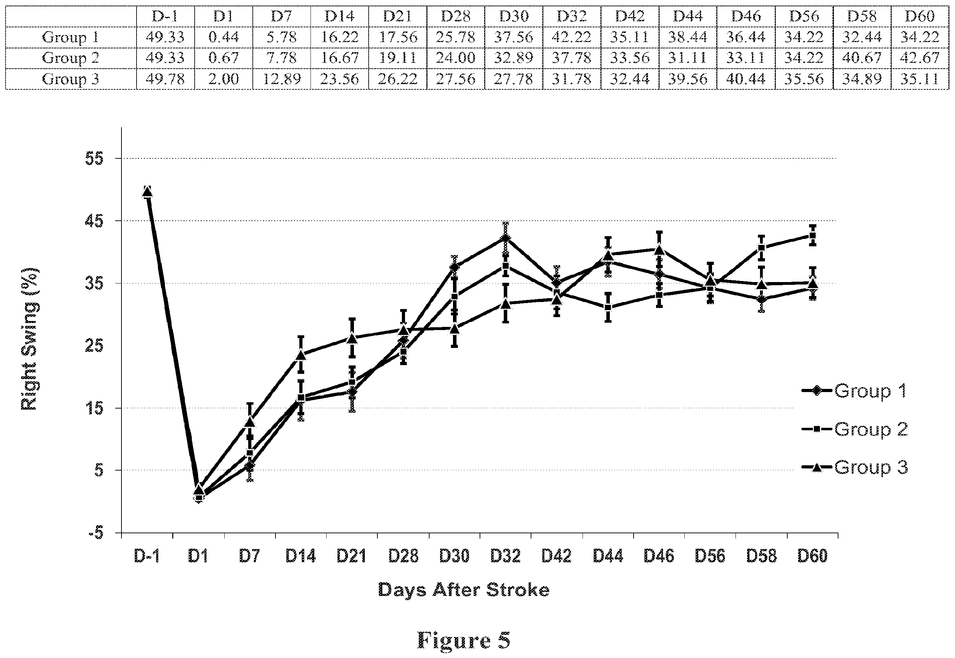

**Figure 5** shows the results of the body swing test: X axis represents the number of days after the stroke event (i.e., days post-MCAO). The Y axis represents the behavioral score. The graph shows an average behavioral score of the animals in each test group (i.e., Groups 1-3) at D-1, D1, D7, D14, D21, D28, D30, D32, D42, D44, D46, D56, D58, D60 as described in the Examples.

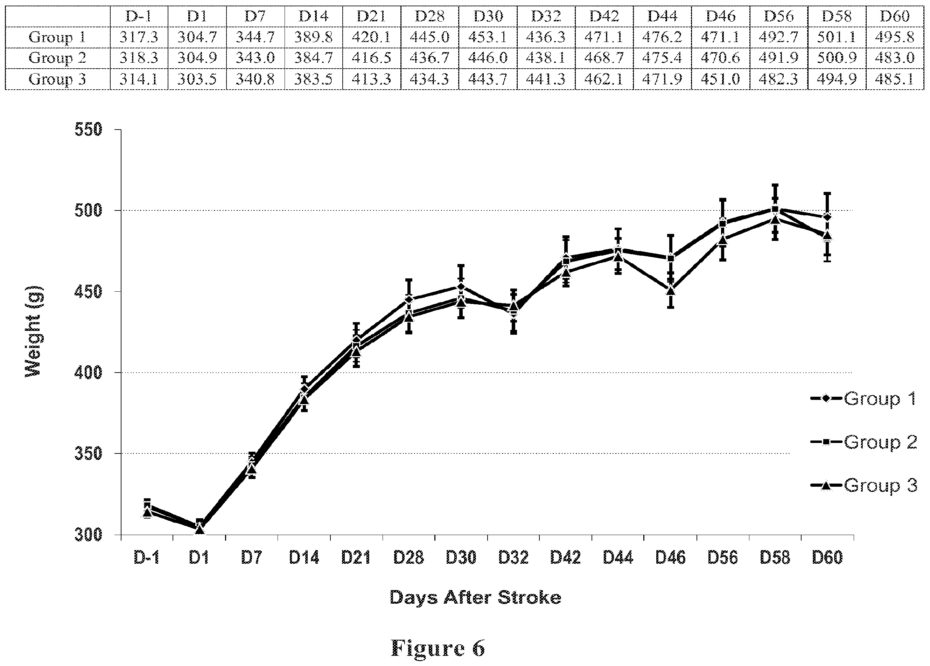

**Figure 6** shows the average weight (g) of the animals in each test group (i.e., Groups 1-3) at days (i.e., D-1, D1, D7, D14, D21, D28, D30, D32, D42, D44, D46, D56, D58, D60) after the stroke event (i.e., MCAO).

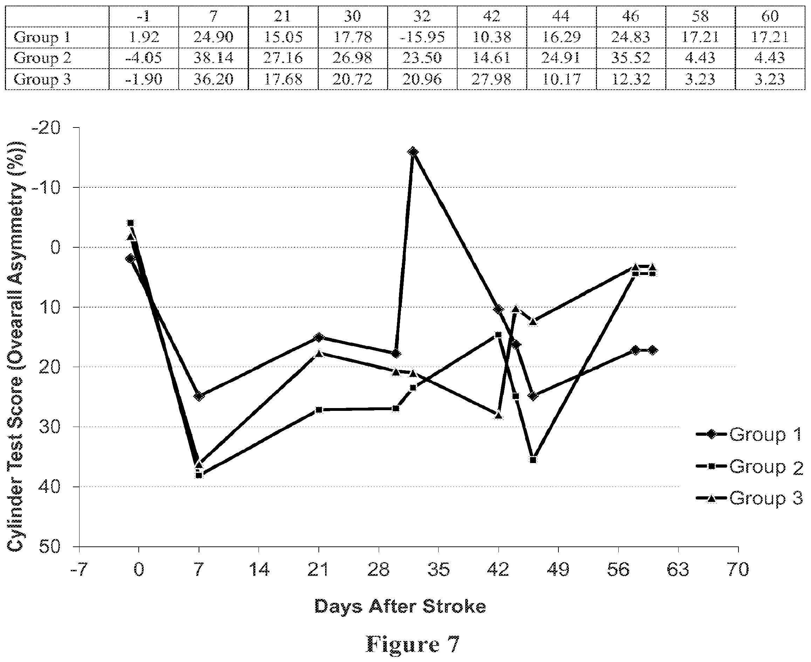

**Figure 7** shows the results of the cylinder test: X axis represents the number of days after the stroke event (i.e., days post-MCAO). The Y axis represents the behavioral score. The graph shows an average behavioral score of the animals in each test group (i.e., Groups 1-3) at Day -1 (pre-operation), Day 7, Day 21, Day 30, Day 32, Day 44, Day 46, Day 58, Day 60 as described in the Examples.

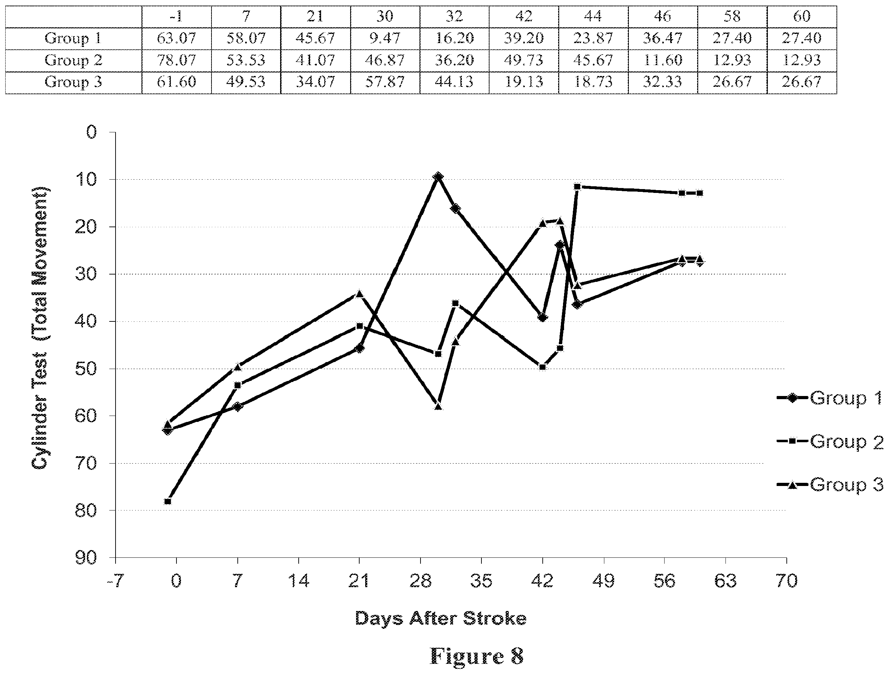

**Figure 8** shows the total movement score of animals subjected to the cylinder test: X axis represents the number of days after the stroke event (i.e., days post-MCAO). The Y axis represents the behavioral score. The graph shows an average behavioral score of the animals in each test group (i.e., Groups 1-3) at Day -1 (pre-operation), Day 7, Day 21, Day 30, Day 32, Day 44, Day 46, Day 58, Day 60.

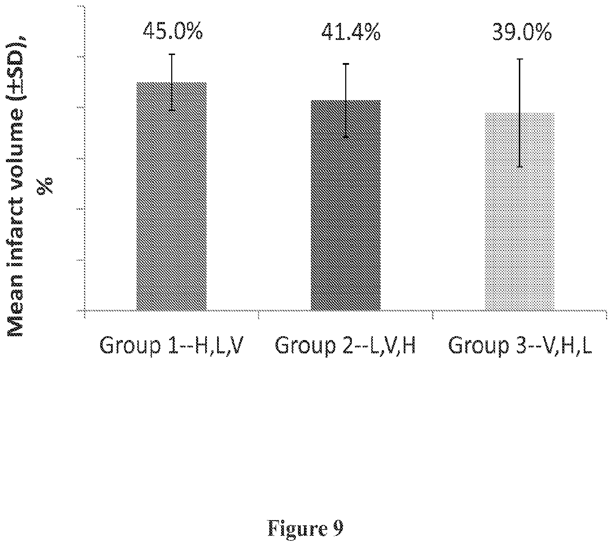

**Figure 9** shows the mean infarct volume (%) of animals in Groups 1, 2 and 3 after MCAO.

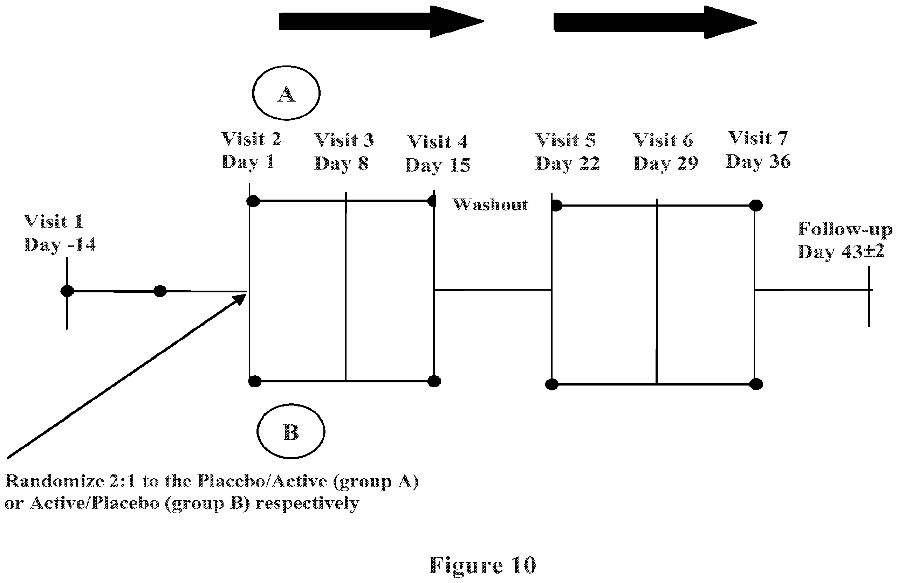

**Figure 10** shows the study design of the clinical protocol described in Example 16.

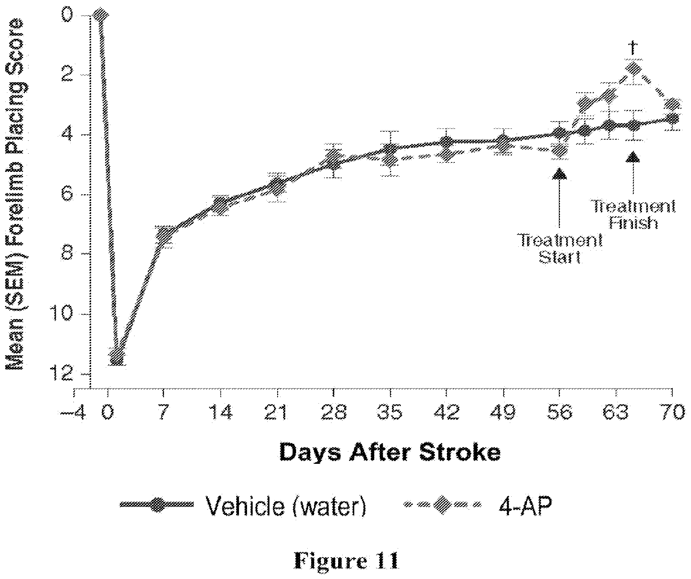

**Figure 11** shows the results of the forelimb placing test: The X axis represents the number of days after the stroke event (i.e., days post-MCAO). The Y axis represents the behavioral score (0 to 12, with 0 being normal function and 12 being maximally impaired). The graph shows an average behavioral score of the animals in each test group (i.e., vehicle and 4-AP) as described in Example 17 ("D"=day). Data is expressed as means ± SEM. *=p<0.05; †=p<0.001; ‡=p<0.0001.

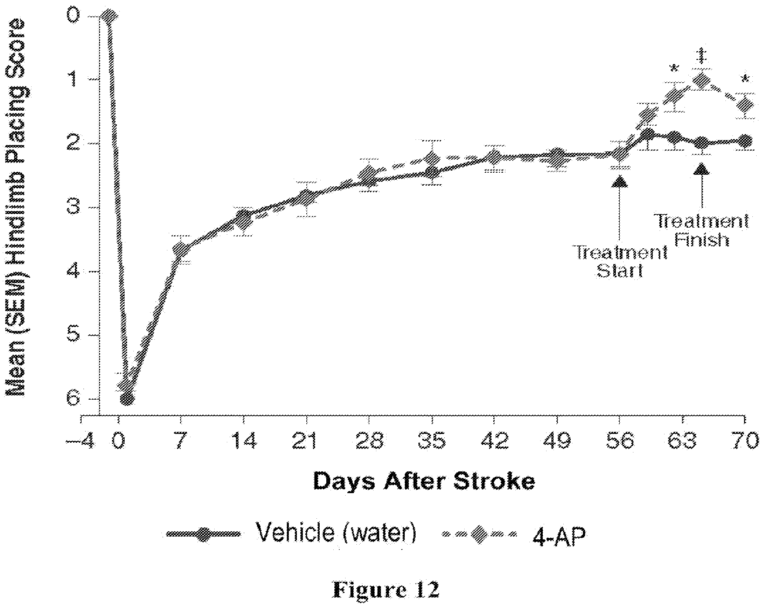

**Figure 12** shows the results of the hindlimb placing test: The X axis represents the number of days after the stroke event (i.e., days post-MCAO). The Y axis represents the behavioral score (0 to 6, with 0 being normal function and 6 being maximally impaired). The graph shows an average behavioral score of the animals in each test group (i.e., vehicle and 4-AP) as described in Example 17 ("D"=day). Data is expressed as means ± SEM. *=p<0.05; †=p<0.001; ‡=p<0.0001.

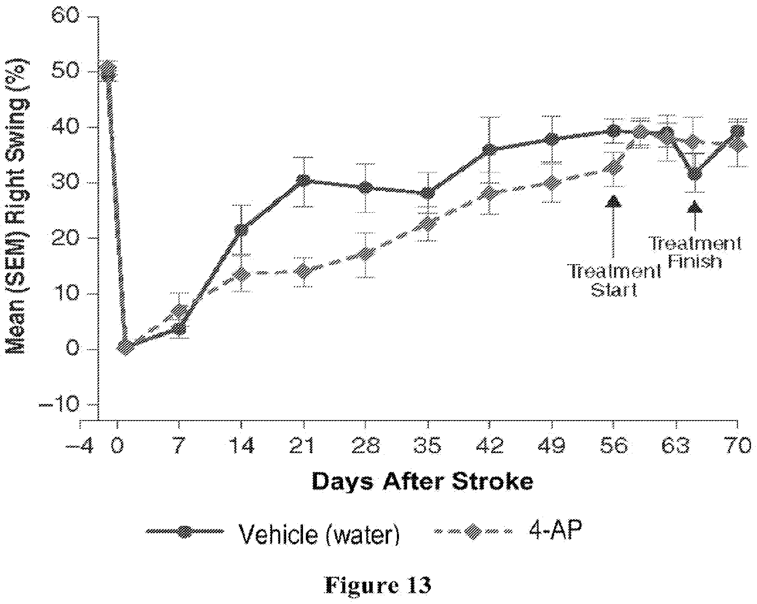

**Figure 13** shows the results of the body swing test: The X axis represents the number of days after the stroke event (i.e., days post-MCAO). The Y axis represents the behavioral score. The graph shows an average behavioral score of the animals in each test group (i.e., vehicle and 4-AP) as described in Example 17 ("D"=day). Data is expressed as means ± SEM. *=p<0.05; †=p<0.001; ‡=p<0.0001.

### 5. DETAILED DESCRIPTION

As sequelae of stroke, individuals suffer a neural injury, and as a result, are often left with some degree of motor, sensory or sensorimotor impairment. Experimental therapeutics have focused on protecting neurons from death during and shortly after ischemia. There is no FDA approved drug other than time-constrained tPA administration that restores function in people following stroke, TIA or multi-infarct syndromes.

The invention provides for treatment of patients who have had strokes, and in some embodiments, for treatment of patients who have suffered a neural injury due to stroke. In particular, the invention provides for treatment of patients left with some degree of motor, sensory or sensorimotor impairment following a stroke. This impairment can range from extremely mild to severe and incapacitating. Such impairment can be due to loss of neurons and myelin from an ischemic event or from the inflammation and immune responses after the ischemic episode. Such impairment can be due to loss or damage to the neurons or myelin in the regions of the brain (e.g., cortical, subcortical, or noncortical) regulating sensorimotor functions as a result of a stroke. For example, such impairment can be due to loss or damage to the neurons or myelin in motor cortex, sensory cortex, or somatosensory cortex, or loss or damage to the neurons or myelin in the sensorimotor cortex or areas of the cortex responsible for sensorimotor functions. In some embodiments, a patient treated in accordance with the methods described herein has had an ischemic stroke. In one embodiment, a patient treated in accordance with the methods described herein has had a middle cerebral artery stroke (such as due to middle cerebral artery occlusion). In other embodiments, a patient treated in accordance with the methods described herein has had a hemorrhagic stroke. In some embodiments, a patient treated in accordance with the methods described herein has a stable or chronic sensorimotor deficit due to a stroke (such as hemorrhagic stroke or ischemic stroke, e.g., middle cerebral artery stroke). In one embodiment, a patient treated in accordance with the methods described herein does not have multiple sclerosis.

Disclosed herein is the use of an aminopyridine (e.g., 4-AP or 3, 4-DAP) or a pharmaceutically acceptable salt thereof to treat stroke-related neuronal loss or damage, particularly in the region of the brain (e.g., cortical, subcortical or noncortical) regulating a sensorimotor function. In particular, it is disclosed herein that 4-AP and other aminopyridines, or pharmaceutically acceptable salts thereof, are useful in restoring the loss of sensorimotor functions following a stroke event. As set forth herein, in preferred embodiments, an aminopyridine (e.g., 4-AP) or a pharmaceutically acceptable salt thereof is administered to individuals who have demonstrated loss of sensorimotor function associated with or following a stroke event. In certain embodiments, described herein is the use of an aminopyridine (e.g., 4-AP or 3, 4-DAP) or a pharmaceutically acceptable salt thereof to treat a stroke-related impairment of a neurological function. In some of these embodiments, treatment of a patient with an effective amount of an aminopyridine recovers or improves a neurological function impaired due to stroke.

In specific embodiments, the impairment that is being treated in accordance with the methods described herein does not affect memory or cognition. In other specific embodiments, the composition comprising an aminopyridine administered to a patient in accordance with the invention does not contain choline, a source of choline, a precursor of acetylcholine, or a precursor of choline.

The patients or subjects that are treated by the methods described herein include, but are not limited to, humans and non-human vertebrates such as wild, domestic and farm animals. In certain embodiments, the patient treated in accordance with the invention is a mammal, e.g., a human, a cow, a dog, a cat, a goat, a sheep, a horse, or a pig. In a preferred embodiment, the patient is a human.

As set forth herein, the present inventors demonstrate that an aminopyridine, and specifically, 4-aminopyridine is effective in restoring neurological function after middle cerebral artery occlusion in rats that is a model for ischemic stroke in humans. The studies described in Example 2 and Example 17 used a rat permanent middle cerebral artery occlusion (MCAO) model of stroke to evaluate the effects of 4-aminopyridine on sensorimotor function at a time when endogenous recovery has stabilized. As described herein, the present inventors have made the surprising discovery that 4-aminopyridine is effective for treating sensorimotor impairments following an ischemic event. The data obtained by the inventors and described herein show efficacy even when dosing is initiated during a chronic phase following an ischemic event, e.g., 4 weeks or 8 weeks after the ischemic event. Accordingly, in certain embodiments, described herein are methods of treating a stroke-related sensorimotor impairment in a patient using an aminopyridine, e.g., 4-aminopyridine, or a pharmaceutically acceptable salt thereof. In particular embodiments, the treatment is during the early chronic phase and/or stable chronic phase post-stroke. In some embodiments, described herein is treatment of patients in accordance with the methods disclosed herein at, or after, 1, 2, 3, 4, 5, 6, 7, 8 weeks; 1,2 3, 4, 5, 6 months; or 1, 2, 3, 4, 5, 10, 15, 20 years, or any time, post-stroke. In other embodiments, described herein is treatment of patients in accordance with the methods disclosed herein within, or after, 1, 2, 4, 6, 8, 10, 12, 14, 16, 18, 20, or 22 hours; or 1,2, 3, 4, 5, 6, 7, 8, 9, or 10 days post-stroke.

### 5.1 Aminopyridines and Compositions Comprising Aminopyridines

The structure of an aminopyridine is well known in the art. As shown in U.S. Patent No. 5,952,357, a mono- or diaminopyridine has the following structure: 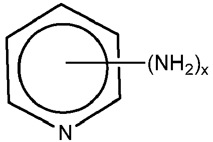 , wherein x is 1 or 2.

Aminopyridines having the above structural formula wherein x is 1 are, e.g., 2-aminopyridine, 3- aminopyridine and 4- aminopyridine. Aminopyridine compounds having the above structural formula wherein x is 2 are, e.g., 2,3-diaminopyridine; 2,5-diaminopyridine; 2,6- diaminopyridine; 3,4- diaminopyridine; 4,5- diaminopyridine and 4,6-diaminopyridine.

In one embodiment, the aminopyridine is a mono- or di-aminopyridine. In one embodiment, the mono- aminopyridine is 3-aminopyridine or 4-aminopyridine. In one embodiment the di- aminopyridine is 3,4-diaminopyridine.

As will be appreciated, a pharmaceutically acceptable salt of an aminopyridine may be used instead of or in addition to an aminopyridine in any or all of the methods of treating discussed herein. Thus, in specific embodiments, a pharmaceutically acceptable salt of an aminopyridine (i.e., any pharmaceutically acceptable salt of any of the aminopyridine compounds listed above) is used in the methods of treating a stroke-related impairment, e.g., a sensorimotor impairment, provided herein. These salts can be prepared, for example, in situ during the final isolation and purification of the compounds or by separately reacting the purified compound in its free base form with a suitable organic or inorganic acid and isolating the salt thus formed. In some embodiments, a salt of a mono- or di-aminopyridine is used in the methods described herein. In another embodiment, a salt of 3-aminopyridine or 4-aminopyridine is used. In yet another embodiment, a salt of 3,4-diaminopyridine is used. In some embodiments, the pharmaceutically acceptable salt of an aminopyridine is prepared using acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, or p-toluenesulfonic acid. In one embodiment, one equivalent of an aminopyridine, as used herein, may form an acid salt with less than one or with one or more than one equivalent of an acid. In one embodiment an aminopyridine, as used herein, may form a dihydrochloride salt. In one embodiment an aminopyridine, as used herein, may form a phosphate salt. For further description of pharmaceutically acceptable salts that can be used in the methods described herein see, for example, S.M. Barge et al., "Pharmaceutical Salts," 1977, J. Pharm. Sci. 66:1-19.

In preferred embodiments, an aminopyridine itself, and not a pharmaceutically acceptable salt thereof, is used in any of the methods of treating stroke-related impairments described herein.

Preferred aminopyridines or pharmaceutically acceptable salts thereof for use according to the invention are compounds that specifically inhibit potassium channels. Such compounds preferably exhibit a profile or pattern of selective inhibition of neuronal potassium channels, relative to other tissues, analogous to the inhibition profile of 4-aminopyridine or 3,4-diaminopyridine, or exhibit a profile of selective inhibition of neuronal potassium channels, relative to other tissues, analogous to the inhibition profile common to that of 3,4-diaminopyridine and 4-aminopyridine. Preferred aminopyridines include, without limitation, 4-aminopyridine, and 3, 4-diaminopyridine.

Aminopyridines or pharmaceutically acceptable salts thereof for use according to the invention can be in sustained release or immediate release compositions. In certain embodiments, aminopyridines or pharmaceutically acceptable salts thereof for use according to the invention are formulated for oral, subcutaneous, intramuscular or intravenous administration.

In a specific embodiment, the sustained release composition of an aminopyridine or a pharmaceutically acceptable salt thereof results in the release of the aminopyridine or a pharmaceutically acceptable salt thereof from the dosage formulation at a sustained rate such that a therapeutically beneficial blood level is maintained over a period of at least about 6, 7, 8, 9, 10, 11, 12, 13, 14, 15, 16, 17, 18, 19, 20, 21, 22, 23, 24, 25, 26, 27, 28, 29, 30, hours, or more than 18 hours, or more than 24 hours, or more than 30 hours. Preferably, the amount of the aminopyridine or a pharmaceutically acceptable salt thereof in the oral dosage formulations according to embodiments of the present invention establishes a therapeutically useful plasma or CNS concentration through t.i.d., b.i.d., or q.d. administration of the pharmaceutical composition. The terms "sustained release" and "extended release" are generally synonymous unless the context clearly indicates otherwise.

In certain embodiments, a therapeutically effective amount of an aminopyridine or a pharmaceutically acceptable salt thereof is between 4 mg and 17.5 mg (e.g., 4, 5, 6, 7, 7.5, 8, 9, 10, 11, 12, 12.5, 13, 14, 15, 16, 17 or 17.5 mg), or in the range from 4 to 40 mg, and in a specific embodiment, it is administered once daily or twice daily, preferably in a sustained release composition. In specific embodiments, aminopyridine or a pharmaceutically acceptable salt thereof is administered in a sustained release composition. In other specific embodiments, aminopyridine or a pharmaceutically acceptable salt thereof is administered in an immediate release composition. In certain embodiments a therapeutically effective amount of 4-aminopyridine, or a pharmaceutically acceptable salt thereof, is between 4 mg and 17.5 mg (e.g., 4, 5, 6, 7, 7.5, 8, 9, 10, 11, 12, 12.5, 13, 14, 15, 16, 17 or 17.5 mg), or in the range from 4 to 40 mg, and, in a specific embodiment, it is administered once daily or twice daily, preferably in a sustained release composition. In one embodiment, twice daily administration is administration of an aminopyridine or a pharmaceutically acceptable salt thereof every 12 hours.

In certain embodiments, an aminopyridine or a pharmaceutically acceptable salt thereof is administered in an amount in the range between 4 mg and 17.5 mg (e.g., 4, 5, 6, 7, 7.5, 8, 9, 10, 11, 12, 12.5, 13, 14, 15, 16, 17 or 17.5 mg), or from 4 mg to 17.5 mg, or from 4 mg to 40 mg, once daily or twice daily, preferably in a sustained release composition. In specific embodiments, aminopyridine or a pharmaceutically acceptable salt thereof is administered in a sustained release composition. In other specific embodiments, aminopyridine or a pharmaceutically acceptable salt thereof is administered in an immediate release composition. In certain embodiments an amount of 4-aminopyridine, or a pharmaceutically acceptable salt thereof, is administered in the range between 4 mg and 17.5 mg (e.g., 4, 5, 6, 7, 7.5, 8, 9, 10, 11, 12, 12.5, 13, 14, 15, 16, 17 or 17.5 mg), or from 4 mg to 17.5 mg, or from 4 mg to 40 mg, once daily or twice daily, preferably in a sustained release composition. In one embodiment, twice daily administration is administration of an aminopyridine or a pharmaceutically acceptable salt thereof every 12 hours.

In a specific embodiment, an aminopyridine (e.g., 4-aminopyridine) is administered in an amount in the range of 4 to 17.5 mg (e.g., 4, 5, 6, 7, 7.5, 8, 9, 10, 11, 12, 12.5, 13, 14, 15, 16, 17 or 17.5 mg) twice daily in a sustained release composition, or is administered in an amount in the range of 8 to 40 mg (e.g., 8, 9, 10, 11, 12, 13, 14, 15, 16, 17, 18, 19, 20, 21, 22, 23, 24, 25, 26, 27, 28 ,29, 30, 31, 32, 33, 34, 35, 36, 37, 38, 39, or 40 mg) once daily in a sustained release composition.

Also described is a method wherein said therapeutically effective amount of an aminopyridine (such as 3,4-diaminopyridine, 4-aminopyridine and the like) or a pharmaceutically acceptable salt thereof is 10 milligrams in a sustained release composition twice daily.

In another disclosure, a method is provided wherein said therapeutically effective amount of an aminopyridine (e.g., 4-aminopyridine) or a pharmaceutically acceptable salt thereof is 5 milligrams in a sustained release composition twice daily. In another disclosure, a method is provided wherein said therapeutically effective amount of an aminopyridine (e.g., 4-aminopyridine) or a pharmaceutically acceptable salt thereof is 7.5 milligrams in a sustained release composition twice daily. In another disclosure, a method is provided wherein said therapeutically effective amount of an aminopyridine (e.g., 4-aminopyridine) or a pharmaceutically acceptable salt thereof is 10 milligrams in a sustained release composition twice daily. In another disclosure, a method is provided wherein said therapeutically effective amount of an aminopyridine (e.g., 4-aminopyridine) or a pharmaceutically acceptable salt thereof is 12.5 milligrams in a sustained release composition twice daily. In another disclosure, a method is provided wherein said therapeutically effective amount of an aminopyridine (e.g., 4-aminopyridine) or a pharmaceutically acceptable salt thereof is 15 milligrams in a sustained release composition twice daily. In another disclosure, a method is provided wherein said therapeutically effective amount of an aminopyridine (e.g., 4-aminopyridine) or a pharmaceutically acceptable salt thereof is 17.5 milligrams in a sustained release composition twice daily.

In some disclosures, a method is provided wherein the therapeutically effective amount of an aminopyridine (e.g., 4-aminopyridine) or a pharmaceutically acceptable salt thereof is 20 milligrams in a sustained release composition once-daily. In another disclosure, a method is provided wherein said therapeutically effective amount of an aminopyridine (e.g., 4-aminopyridine) or a pharmaceutically acceptable salt thereof is 8, 10, 11, 12, 12.5, 13, 14, 15, 16, 17, 17.5, 18, 19, 20, 20.5, 21, 21.5, 22, 22.5, 23, 23.5, 24, 24.5, 25, 25.5, 26, 26.5, 27.5, 30 or 35 milligrams in a sustained release composition once daily.

Also described herein is a method comprising administration of a therapeutically effective amount of 4-aminopyridine, or a pharmaceutically acceptable salt thereof, in a total daily amount of 8, 10, 11, 12, 12.5, 13, 14, 15, 16, 17, 17.5, 18, 19, 20, 20.5, 21, 21.5, 22, 22.5, 23, 23.5, 24, 24.5, 25, 25.5, 26, 26.5, 27.5, 28, 29, 30, 31, 32, 33, 34, 35, 36, 37, 38, 39, or 40 milligrams in a sustained release composition. An exemplary embodiment comprises twice daily administration where 15 milligrams in a sustained release composition is administered in the morning; and 10 milligrams in a sustained release composition is administered in the evening. An exemplary embodiment comprises twice daily administration where 12.5 milligrams in a sustained release composition is administered in the morning; and 7.5 milligrams in a sustained release composition is administered in the evening. Another exemplary embodiment comprises administration of a total daily amount in a once-daily composition.

Also described is a method comprising administration of a therapeutically effective amount of 4-aminopyridine, or a pharmaceutically acceptable salt thereof, in a total daily amount of 8, 10, 11, 12, 12.5, 13, 14, 15, 16, 17, 17.5, 18, 19, 20, 20.5, 21, 21.5, 22, 22.5, 23, 23.5, 24, 24.5, 25, 25.5, 26, 26.5, 27.5, 28, 29, 30, 31, 32, 33, 34, 35, 36, 37, 38, 39, or 40 milligrams in an immediate release composition. In some embodiments, an immediate release composition comprising an aminopyridine or a pharmaceutically acceptable salt thereof is administered three times daily or more than three times daily (e.g., 4, 5, or 6 times daily).

In certain embodiments, an aminopyridine (e.g., 4-aminopyridine) or a pharmaceutically acceptable salt thereof is formulated as a sustained-release (SR) or extended release (ER) matrix tablet in various strengths, for example, from 4 to 40 mg, where 5-, 7.5-, 10-, 12.5-, 15-, and 17.5 are presently preferred. One embodiment of 4-aminopyridine-SR is 10 mg which is preferred for b.i.d. dosing, other dosing regimens are within the scope of the invention; accordingly other amounts of active ingredient in sustained-release formulations are also encompassed within the scope of the invention.

In yet other embodiments, the sustained release formulation that is used in the methods described herein is 4-aminopyridine-SR, or AMPYRA® (Acorda Therapeutics, Hawthorne, NY), or a sustained release composition for 4-aminopyridine as set forth in US Patent 5,370,879, US Patent 5,540,938; US Patent 8,007,826; or, US Patent Publication US2005-0228030.

In certain embodiments, an aminopyridine or a pharmaceutically acceptable salt thereof may be present in the pharmaceutical compositions, such as a tablet, a chewable tablet, a pill, a capsule, a microcapsule, a solution, a suspension, a parenteral solution, a lozenge, a powder, a granule, a troche, a syrup, a suppository, an injection, or a blister pack. Compositions can be formulated to contain a daily dose, a half-daily dose, or a convenient fraction of a daily dose, in a dosage unit, which may be a single tablet or capsule or convenient volume of a liquid. In one embodiment, the solutions are prepared from water-soluble salts, such as the hydrochloride salt. In general, all of the compositions are prepared according to known methods in pharmaceutical chemistry. Capsules can be prepared by mixing an aminopyridine or a pharmaceutically acceptable salt thereof with a suitable carrier or diluent and filling the proper amount of the mixture in capsules. The usual carriers and diluents include, but are not limited to, inert powdered substances such as starch of many different kinds, powdered cellulose, especially crystalline and microcrystalline cellulose, sugars such as fructose, mannitol and sucrose, grain flours and similar edible powders.

Suitable formulations can be prepared by methods commonly employed using conventional, organic or inorganic additives, such as one or more of: an excipient (e.g., sucrose, starch, mannitol, sorbitol, lactose, glucose, cellulose, talc, calcium phosphate or calcium carbonate), a binder (e.g., cellulose, methylcellulose, hydroxymethylcellulose, polypropylpyrrolidone, polyvinylpyrrolidone, gelatin, gum arabic, polyethyleneglycol, sucrose or starch), a disintegrator (e.g., starch, carboxymethylcellulose, hydroxypropylstarch, low substituted hydroxypropylcellulose, sodium bicarbonate, calcium phosphate or calcium citrate), a lubricant (e.g., magnesium stearate, light anhydrous silicic acid, talc or sodium lauryl sulfate), a flavoring agent (e.g., citric acid, menthol, glycine or orange powder), a preservative (e.g., sodium benzoate, sodium bisulfite, methylparaben or propylparaben), a stabilizer (e.g., citric acid, sodium citrate or acetic acid), a suspending agent (e.g., methylcellulose, polyvinyl pyrroliclone or aluminum stearate), a dispersing agent (e.g., hydroxypropylmethylcellulose), a diluent (e.g., water), and base wax (e.g., cocoa butter, white petrolatum or polyethylene glycol). In some embodiments, suitable formulations of an aminopyridine or a pharmaceutically acceptable salt thereof can be prepared using one, two, three or more, or all, of the following additives: colloidal silicon dioxide, hydroxypropyl methylcellulose, magnesium stearate, microcrystalline cellulose, polyethylene glycol, and titanium dioxide.

In one embodiment, an aminopyridine or a pharmaceutically acceptable salt thereof used in the methods described herein is formulated as a tablet. Tablets can be prepared by direct compression, by wet granulation, or by dry granulation. In certain embodiments, their formulations incorporate diluents, binders, lubricants and disintegrators as well as the compound. Typical diluents include, for example, various types of starch, lactose, mannitol, kaolin, calcium phosphate or sulfate, inorganic salts such as sodium chloride and powdered sugar. Powdered cellulose derivatives are also useful. In one embodiment, the pharmaceutical composition is lactose-free. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders. In certain embodiments, the following excipients can be included in the tablet: hydroxypropyl methylcellulose, USP; microcrystalline cellulose, USP; colloidal silicon dioxide, NF; magnesium stearate, USP; and/or Opadry White.

Pharmaceutical compositions used in the methods describe herein can be as described, for example, in U.S. Patent Application Publication No. 2005/0276851, published December 15, 2005 and U.S. Patent Application Publication No. 2005/0228030, published October 13, 2005. The aminopyridines according to the instant invention can exist in unsolvated as well as solvated forms with pharmaceutically acceptable solvents such as water, ethanol, and the like. In general, the solvated forms are considered equivalent to the unsolvated forms for the purposes of the present invention.

Also described is a method wherein said therapeutically effective amount of an aminopyridine, e.g., 4-aminopyridine, or a pharmaceutically acceptable salt thereof, achieves a Cₘᵢₙₛₛ of at least or more than: 5, 6, 7, 8, 9, 10, 11, 12, 13, 14, 15, 16, 17, 18, 19 or 20 ng/ml. In another disclosure, a method is described wherein said therapeutically effective amount of an aminopyridine (e.g., 4-aminopyridine) or a pharmaceutically acceptable salt thereof achieves an average Cₘᵢₙₛₛ of at least or more than: 5, 6, 7, 8, 9, 10, 11, 12, 13, 14, 15, 16, 17, 18, 19 or 20 ng/ml. In some disclosures, a method is described wherein said therapeutically effective amount of an aminopyridine (e.g., 4-aminopyridine) or a pharmaceutically acceptable salt thereof achieves an average Cₘᵢₙₛₛ of about 20 ng/ml, which comprises an average lower limit value of from 11, 12, 13, 14, 15, 16, 17, 18, 19, or 20 ng/ml, and an average upper limit value of 20, 21, 22, 23, 24, 25, 26, or 27 ng/ml. In one embodiment, an amount of drug is given to an individual patient (e.g., a dose amount) wherein that dose amount corresponds to an amount that when administered to a normative or reference population obtains an average Cₘᵢₙₛₛ of at least or more than: 10, 11, 12, 13, 14, 15, 16, 17, 18, 19 or 20 ng/ml. Fluid or tissue levels (e.g., Cₘᵢₙₛₛ, Cₘₐₓₛₛ, Cₐᵥₛₛ) in reference population can be referred to as normative values. In another disclosure, a method is described wherein said therapeutically effective amount of an aminopyridine or a pharmaceutically acceptable salt thereof achieves a Cₘᵢₙₛₛ in a range of about 5 to 25 ng/ml, 10 to 18 ng/ml, 13 to 15 ng/ml, or 15 to 30 ng/ml. In another disclosure, a method is described wherein said therapeutically effective amount of an aminopyridine or a pharmaceutically acceptable salt thereof achieves a Cₘᵢₙₛₛ of about 20 ng/ml. In another disclosure, a method is described wherein said therapeutically effective amount of an aminopyridine or a pharmaceutically acceptable salt thereof achieves a Cₘᵢₙₛₛ of about 20 ng/ml; in certain embodiments, a Cₘᵢₙₛₛ of about 20 ng/ml comprises a lower limit value of from 11, 12, 13, 14, 15, 16, 17, 18, 19, or 20 ng/ml, and an upper limit value of 20, 21, 22, 23, 24, 25, 26, or 27 ng/ml.

Also described is a method of treating a stroke-related motor, sensory, or sensorimotor impairment in a patient comprising: administering a therapeutically effective amount of an aminopyridine (e.g., 4-aminopyridine) or a pharmaceutically acceptable salt thereof to said patient such that a Cₘᵢₙₛₛ in a range of 5 to 25 ng/ml, 10 to 20 ng/ml, 15 to 30 ng/ml, or 12 to 20 ng/ml is obtained. In another disclosure, a method for treating a stroke-related motor, sensory or sensorimotor impairment in a patient comprises administering a therapeutically effective amount of an aminopyridine (e.g., 4-aminopyridine) or a pharmaceutically acceptable salt thereof to said patient such that a Cₘᵢₙₛₛ in a range of at least 12 ng/ml to 15 ng/ml is obtained. In another disclosure, a method for treating a stroke-related motor, sensory or sensorimotor impairment in a patient comprises: administering a therapeutically effective amount of an aminopyridine (e.g., 4-aminopyridine) or a pharmaceutically acceptable salt thereof to said patient such that a Cₘᵢₙₛₛ in a range of at least 13 ng/ml to 15 ng/ml is obtained. In one embodiment, an amount of drug is given to an individual patient (e.g., a dose amount) wherein that dose amount corresponds to a dose that when administered to a normative or reference population obtains an average Cₘᵢₙₛₛ of at least or more than: 5, 6, 7, 8, 9, 10, 11, 12, 13, 14, 15, 16, 17, 18, 19 or 20 ng/ml; the plasma levels (e.g., Cₘᵢₙₛₛ, Cₘₐₓₛₛ, Cₐᵥₛₛ) in reference population can be referred to as a normative values. Also described is a method comprising administering a therapeutically effective amount of an aminopyridine (e.g., 4-aminopyridine) or a pharmaceutically acceptable salt thereof to a patient such that a Cₘᵢₙₛₛ of at least 11 or 12 ng/ml is obtained.

Also described is a method wherein said therapeutically effective amount of an aminopyridine or a pharmaceutically acceptable salt thereof achieves a Tₘₐₓ of about 2 hours to about 6 hours in a patient. In some of these embodiments, the aminopyridine or a pharmaceutically acceptable salt thereof is administered in a sustained-release composition (e.g., once daily, twice daily or thrice daily). In one of these embodiment, the aminopyridine is 4-aminopyridine. The therapeutically effective amount of 4-aminopyridine can be any amount disclosed herein. In one embodiment, the patient is a human. In some embodiments, a therapeutically effective amount of 4-aminopyridine administered once daily, twice daily or three times daily in a sustained-release composition achieves a Tₘₐₓ of about 2 hours to about 6 hours in a human.

Also described is a method wherein said therapeutically effective amount of an aminopyridine, e.g., 4-aminopyridine, or a pharmaceutically acceptable salt thereof achieves a Cₘₐₓₛₛ of the following, or less than the following values: 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, or 20 ng/ml. In another disclosure, a method is provided wherein said therapeutically effective amount of an aminopyridine (e.g., 4-aminopyridine) or a pharmaceutically acceptable salt thereof achieves an average Cₘₐₓₛₛ of the following, or less than the following values: 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, or 20 ng/ml. In one embodiment, an amount of drug is given to an individual patient (e.g., a dose amount) wherein that dose amount corresponds to an amount that when administered to a normative or reference population obtains an average Cₘₐₓₛₛ of the following, or less than the following values: 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, or 20 ng/ml. Fluid or tissue levels (e.g., Cₘᵢₙₛₛ, Cₘₐₓₛₛ, Cₐᵥₛₛ) in reference population can be referred to as normative values. In anotherdisclosure, a method is provided wherein said therapeutically effective amount of an aminopyridine (e.g., 4-aminopyridine) or a pharmaceutically acceptable salt thereof achieves a Cₘₐₓₛₛ in a range of about 15 to 30 ng/ml, 25 to 35 ng/ml, 25 to 40 ng/ml, or 35 to 55 ng/ml. In another disclosure, a method is provided wherein said therapeutically effective amount of an aminopyridine or a pharmaceutically acceptable salt thereof achieves a Cₘₐₓₛₛ of about 30 ng/ml. In another disclosure, a method is provided wherein said therapeutically effective amount of an aminopyridine (e.g., 4-aminopyridine) or a pharmaceutically acceptable salt thereof achieves a Cₘₐₓₛₛ in a range that comprises a lower limit value of from 15, 16, 17, 18, 19, 20, 21, 22, 23, 24, 25, 26, 27, 28, 29, or 30 ng/ml, and an upper limit value of 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 ng/ml.

In another disclosure, a method is described wherein said therapeutically effective amount of an aminopyridine (e.g., 4-aminopyridine) or a pharmaceutically acceptable salt thereof achieves an average Cₘₐₓₛₛ of, or less than: 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, or 20 ng/ml. In one embodiment, an amount of drug is given to an individual patient (e.g., a dose amount) wherein that dose amount corresponds to a dose that when administered to a normative or reference population obtains an average Cₘₐₓₛₛ of, or less than: 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, or 20 ng/ml; the plasma levels (e.g., Cₘᵢₙₛₛ, Cₘₐₓₛₛ, Cₐᵥₛₛ) in reference population can be referred to as a normative values.

In another embodiment, a unit dose of a composition as substantially described herein is used.

The actual dosage amount of an aminopyridine, a pharmaceutically acceptable salt thereof, or a composition comprising an aminopyridine administered to a subject may be determined by physical and physiological factors such as age, sex, body weight, severity of condition, the type of disease being treated, previous or concurrent therapeutic interventions, idiopathy of the subject and on the route of administration. These factors are readily determined by a skilled artisan. The practitioner responsible for administration will typically determine the concentration of active ingredient(s) in a composition and appropriate dose(s) for the individual subject. The dosage may be adjusted by the individual practitioner in the event of any complication or alteration in patient status.

It is especially advantageous to formulate parenteral compositions in dosage unit form for ease of administration and uniformity of dosage. Dosage unit form as used herein refers to physically discrete units suited as unitary dosages for the subjects to be treated; each unit containing a predetermined quantity of therapeutic compound calculated to produce the desired therapeutic effect in association with the required pharmaceutical carrier. The specification for the dosage unit forms of the invention are dictated by and directly dependent on (a) the unique characteristics of the therapeutic compound and the particular therapeutic effect to be achieved, and (b) the limitations inherent in the art of compounding such a therapeutic compound for the treatment of a selected condition in a patient. Unit dosage forms can be tablets or blister packs. In certain administration protocols a patient may utilize more than a single unit dose at a time, e.g., consume two tablets contained in separate blisters of a blister pack.

Optical Isomers - Diastereomers - Geometric Isomers - Tautomers: Compounds described herein may contain an asymmetric center and may thus exist as enantiomers. Where the compounds according to the invention possess two or more asymmetric centers, they may additionally exist as diastereomers. The present invention includes all such possible stereoisomers as substantially pure resolved enantiomers, racemic mixtures thereof, as well as mixtures of diastereomers. The formulas are shown without a definitive stereochemistry at certain positions. The present invention includes all stereoisomers of such formulas and pharmaceutically acceptable salts thereof. Diastereoisomeric pairs of enantiomers may be separated by, for example, fractional crystallization from a suitable solvent, and the pair of enantiomers thus obtained may be separated into individual stereoisomers by conventional means, for example by the use of an optically active acid or base as a resolving agent or on a chiral HPLC column. Further, any enantiomer or diastereomer of a compound of the general formula may be obtained by stereospecific synthesis using optically pure starting materials or reagents of known configuration.

Aminopyridines of the invention or pharmaceutically acceptable salts thereof are administered at a therapeutically effective dosage sufficient to treat an impairment associated with a stroke in a patient. In certain embodiments, the treatment reduces the amount of symptoms of the impairment in the patient by at least about 10%, more preferably 20%, more preferably by at least about 40%, even more preferably by at least about 60%, and still more preferably by at least about 80% relative to untreated subjects. Such percent change quantification is preferably applied to assays of sensorimotor function that provide measurements of results in continuous linear scales, such as T25FW, etc. Other tests of sensorimotor function will not be expressed as percent change but would be predicted to result in a significant change with the appropriate statistical comparison. Such tests include semiquantative measures that assign values to the ability to perform certain skills. In some embodiments, treatment in accordance with the invention results in a statistically significant improvement in a stroke-related sensorimotor impairment (e.g., as measured by the patient's ability to perform certain task or skill) in comparison to a control. Such control can be the patient's ability to perform the assessed task or skill prior to the commencement of treatment.

### 5.2 Sensorimotor Impairments and Outcomes of Aminopyridine Administration in accordance with the invention

Also described herein are methods for treating a stroke-induced neural injury in a mammal, and in particular, a method for treating a stroke-related sensorimotor impairment. In some embodiments, the patient treated in accordance with the methods described herein has one or more sensorimotor impairments (e.g., has been diagnosed with, or exhibits one or more symptoms of a sensorimotor impairment). In certain embodiments, the patient treated in accordance with the methods described herein has an impairment due to neuronal damage (e.g., neuronal loss or demyelination) in the area of the cortex or other region of the brain responsible for or involved with sensorimotor functions. Also described herein are methods of using 4-aminopyridine for treating an impairment of sensorimotor function resulting from a stroke. Such treating can be by administering any of the doses and dosage regimens described in this application.

Sensorimotor impairments, or an impairment of sensorimotor function, treated in accordance with the invention include, without limitation: ataxia, global body control impairments, coordination or balance impairments, impairment in body sense, impairment in propioception, impairment in gait, impairment in reflexes, impairment in dexterity, endurance impairment, impairment in hand function, fine hand coordination loss or impairment, hyperreflexia, impairment in hand strength, impairment in manual dexterity, impairment in grip strength, muscle weakness, muscle tone impairment, range of motion impairments, spasticity, strength impairment/weakness, tremor, impairment in limb function, upper extremity function impairment, lower extremity function impairment, impairment in lower extremity muscle strength, walking impairments (e.g., decreased walking speed), speech impairments (e.g., dysarthria), impairment in jaw function, impairment in chewing, or impairment in jaw articulation. In one embodiment, the sensorimotor impairment treated in accordance with the methods described herein is an impairment in proprioception. In one embodiment, the sensorimotor impairment treated in accordance with the methods described herein is an impairment in oral motor functioning. In particular embodiments, the sensorimotor impairment treated in accordance with the methods described herein is a speech impairment (e.g., dysarthria, apraxia, or dysphonia), or an impairment in chewing and/or swallowing (e.g., dysphagia). In one embodiment, the sensorimotor impairment treated in accordance with the methods described herein is a visual impairment, such as a sensory and/or ocular motor impairment of visual function. In other particular embodiments, the sensorimotor impairment treated in accordance with the methods described herein is an impairment in walking speed, impairment in manual dexterity, impairment in hand strength, or upper limb spasticity. In some embodiments, the sensorimotor impairment treated in accordance with the methods described herein is an impairment in motor and/or sensory function as measured using Fugl-Meyer Assessment. In particular embodiments, the sensorimotor impairment treated in accordance with the methods described herein is an impairment in motor functioning, impairment in balance, impairment in sensation, or an impairment in joint functioning. In specific embodiments, the sensorimotor impairment treated in accordance with the methods described herein is facial paralysis, limb paralysis or hand paralysis.

In certain embodiments, the sensorimotor impairments treated in accordance with the invention include, but are not limited to impairment in walking, impairment in limb function, impairment in lower extremity function, impairment in lower extremity muscle strength, impairment in muscle tone, spasticity, impairment in upper extremity function, impairment in hand function, impairment in fine hand coordination, impairment in grip strength, impairment in balance or coordination, impairment in global body control, impairment in jaw function, impairment in chewing, or impairment in jaw articulation.

In one embodiment, the sensorimotor impairment treated in accordance with the invention is an impairment in lower extremity function and/or lower extremity muscle strength. In one embodiment, the sensorimotor impairment treated in accordance with the invention is an impairment in lower extremity motor function. In one embodiment, the sensorimotor impairment treated in accordance with the invention is an impairment in walking (such as decreased walking speed). In one embodiment, the sensorimotor impairment treated in accordance with the invention is an impairment in upper extremity function (e.g., upper extremity motor function). In one embodiment, the sensorimotor impairment treated in accordance with the invention is limb paralysis. In one embodiment, the sensorimotor impairment treated in accordance with the invention is an impairment in muscle tone or spasticity (e.g, upper limb spasticity). In one embodiment, the sensorimotor impairment treated in accordance with the invention is an impairment in balance or coordination. In one embodiment, the sensorimotor impairment treated in accordance with the invention is an impairment in sensation. In one embodiment, the sensorimotor impairment treated in accordance with the invention is an impairment in oral motor functioning. In a particular embodiment, an impairment in oral motor functioning is an impairment in strength of the muscles of lips and/or tongue (such as in dysarthria). In another particular embodiment, an impairment in oral motor functioning is an impairment in coordination of the muscles of lips and/or tongue (such as in apraxia). In yet another particular embodiment, an impairment in oral motor functioning is an impairment in strength of the muscles involved in breathing. In one embodiment, the sensorimotor impairment treated in accordance with the invention is a speech impairment (e.g., dysarthria, apraxia, dysphonia). In some embodiments, the sensorimotor impairment treated in accordance with the invention is a sensorimotor disturbance of the face, tongue and/or glossopharyngeal muscles. In one embodiment, the sensorimotor impairment treated in accordance with the invention is an impairment in chewing and/or swallowing (e.g., dysphagia). In one embodiment, the sensorimotor impairment treated in accordance with the invention is an impairment in jaw function or jaw articulation. In one embodiment, the sensorimotor impairment treated in accordance with the invention is facial paralysis. In one embodiment, the sensorimotor impairment treated in accordance with the invention is an impairment in hand function, an impairment in hand coordination (e.g., an impairment in fine hand coordination), an impairment in grip strength, an impairment in manual dexterity, or hand paralysis. In some embodiments, the sensorimotor impairment treated in accordance with the invention is a visual impairment or disturbance. The visual impairment treated in accordance with the methods described herein can be a sensory and/or an ocular motor impairment of visual function. In one embodiment, the visual impairment treated in accordance with the methods described herein is a sensory impairment of visual function. In one embodiment, the visual impairment treated in accordance with the methods described herein is an ocular motor impairment of visual function.

In one embodiment, aminopyridine administration restores one or more sensorimotor functions. This is manifest or measured as an improvement, e.g., in walking ability, balance, the ability to stand, hand strength, dexterity, reflexes, answers to art-accepted measures of quality of life, or improvement in any other sensorimotor function described herein or known in the art.

In certain embodiments, treating a patient by administering an amount of an aminopyridine or a pharmaceutically acceptable salt thereof is effective to ameliorate or prevent a stroke-related sensorimotor impairment. In one embodiment, treating a patient by administering an amount of an aminopyridine or a pharmaceutically acceptable salt thereof is effective to prevent the onset of the symptoms of a sensorimotor impairment. In other embodiments, treating a patient by administering an amount of an aminopyridine or a pharmaceutically acceptable salt thereof is effective to alleviate the symptoms (e.g., decrease the severity) of a stroke-related sensorimotor impairment. In yet other embodiments, treating a patient by administering an amount of an aminopyridine or a pharmaceutically acceptable salt thereof is effective to decrease the duration of a stroke-related sensorimotor impairment. In a specific embodiment, treating a patient by administering an amount of an aminopyridine or a pharmaceutically acceptable salt thereof is effective to eliminate a stroke-related sensorimotor impairment, and/or effective to regain the sensorimotor function impaired by the stroke. In certain embodiments, the administering of an aminopyridine or a pharmaceutically acceptable salt thereof is effective to restore a sensorimotor function impaired by a stroke. In some of these embodiments, the stroke is an ischemic stroke. In one embodiment, the stroke is a middle cerebral artery stroke (such as due to middle cerebral artery occlusion). In other embodiments, the stroke is a hemorrhagic stroke.

In another disclosure, a method for maintaining improvement of a sensorimotor function in a patient is provided, wherein such function is impaired as a result of a stroke, said method comprising: administering a therapeutically effective amount of an aminopyridine (such as 3,4-diaminopyridine, 4-aminopyridine and the like) or a pharmaceutically acceptable salt thereof to said patient after previously achieving an improvement of such impaired sensorimotor function in said patient during administration of 4-aminopyridine.

In one disclosure, a method for maintaining improvement(s) in a sensorimotor function in a patient with a stroke-related impairment of such function comprises administering a therapeutically effective amount of an aminopyridine or a pharmaceutically acceptable salt thereof to said patient over an extended period of time. In another disclosure, a method for achieving sustained improvement in a patient with a stroke-related sensorimotor impairment comprises continuing administration of a therapeutically effective amount of an aminopyridine (such as 3,4-diaminopyridine, 4-aminopyridine and the like) or a pharmaceutically acceptable salt thereof to said patient over an extended period of time.

In specific embodiments, the improvement(s) among patients experiencing stroke-related sensorimotor impairment occur over periods of at least or more than: 1, 2, 3, 4, 5, 6, 7, 8, 9, 10, 11, 12, 13, 14, 15,16, 17, 18, 19, 20, 21 days; 1, 2, 3, 4, 5, 6, 7, 8, 9, 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 6, or greater than 5 years of treatment.

The sensorimotor functions, including impairment of sensorimotor functions and improvement in sensorimotor functions, can be assessed using any method known in the art. For example, assessment tests can include, without limitation the timed 25 foot walk (T25FW), 2 minute walk, 6 minute walk (6MW), Box & Block test, Six Spot Step Test, the Manual Muscle test for lower extremity function, LEMMT, the Ashworth score, the Modified Ashworth Scale, grip strength test, 9-hole peg test, fine finger movement, rapid alternating fingers for upper extremity function, functional system scoring for sensory function, and finger-to-nose and heel-to-shin for ataxia. In particular, T25W can be used to measure walking, LEMMT can be used to measure lower extremity muscle strength, the Modified Ashworth Scale can be used to measure spasticity. Art-accepted upper extremity function assessments include, without limitation, performance scale-self-report measures, hand-held dynamometry, and Upper Extremity Index (UEI). Other assessment tests that can be used to measure sensorimotor functions include but are not limited to: Berg Balance Scale (BBS), Kela Coordination Test, Postural Stability Test, Timed 10-meter Gait Test, Shoulder Tug Test, Grip Strength, Maximal isometric force of the knee extensors, muscle endurance tests, passive straight leg raise, TEMPA (upper extremity performance test for the elderly), the Jebsen-Taylor Hand Function Test, The Disabilities of the Arm, Shoulder and Hand (DASH) Questionnaire, and Manual Ability Measure-36 (MAM-36). Another assessment test that can be used to measure sensorimotor functions is Fugl-Meyer Assessment. In some embodiments, Fugl-Meyer Assessment can be used to measure motor functioning (e.g., lower extremity motor function and/or upper extremity motor function), balance, sensation and/or joint functioning. In specific embodiments, Fugl-Meyer Assessment is used to measure lower extremity motor function, upper extremity motor function, and/or sensation. Such assessments can be performed before and after administration of an aminopyridine or a pharmaceutically acceptable salt thereof to a patient in accordance with the methods disclosed herein. For example, a sensorimotor function of a patient suffering from a stroke-related impairment of such function can be assessed before administering an aminopyridine and/or after administering an aminopyridine, e.g., at or after 1, 2, 3, 4, 5, 6, 7, 8, 9, 10 days; 1, 2, 3, 4, 5, 6, 7, 8 weeks; 1, 2, 3, 4, 5, 6, 7, 8, 9, 10 months; or 1, 2, 3, 4, 5 years since the commencement of treatment in accordance with the methods described herein.

In a specific embodiment, a therapeutic outcome in a stroke-related sensorimotor impairment is assayed for and detected at any one, two, three, four, five or more, or each, of the following time points, and/or at a time point later than any one of the following time points: 1, 2, 3, 4, 5, 6, 7, 8, 9, 10, 11, 12, 13, 14, 15, 16, 17, 18, 19, 20, 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, and 66 months; .5, 1, 1.5, 2, 2.5, 3, 3.5, 4, 4.5, 5, 5.5, 6 and 6.5 years post-commencement of treatment with an aminopyridine or a pharmaceutically acceptable salt thereof.

### 5.3 Modes of Administration of Aminopyridines

Also described is a method comprising administering an aminopyridine or a pharmaceutically acceptable salt thereof once daily, twice daily or thrice daily. In certain embodiments, an aminopyridine or a pharmaceutically acceptable salt thereof is administered orally. In other embodiments, an aminopyridine or a pharmaceutically acceptable salt thereof is administered intravenously. In yet other embodiments, an aminopyridine or a pharmaceutically acceptable salt thereof is administered, e.g., intramuscularly or subcutaneously.

Also described is a method comprising administering an aminopyridine or a pharmaceutically acceptable salt thereof during the acute phase post-stroke. The acute phase following stroke is characterized by ongoing damage to the brain tissue (e.g., expansion of an ischemic lesion) post-stroke. For example, during the acute phase, the ongoing damage to the brain tissue can occur in the penumbral area surrounding the core area where the initial injury due to stroke had occurred. Such damage may include cell death, e.g., due to oxygen deprivation. Typically, the acute phase lasts from the time of onset of stroke to approximately six hours post-stroke. In some embodiments, treatment in accordance with the invention comprises administering an aminopyridine or a pharmaceutically acceptable salt thereof to a patient during the period post-stroke in which damage to the brain tissue is ongoing. In one embodiment, such treatment is during the period post-stroke when an ischemic lesion is still expanding. For example, a patient can be treated in accordance with the invention during the acute phase within 1, 2, 3, 4, 5 or 6 hours post-stroke.

Also described is a method comprising administering an aminopyridine or a pharmaceutically acceptable salt thereof during the early chronic phase post-stroke. Following the acute phase post-stroke, there is a period of spontaneous recovery of neurological function-the early chronic phase-which can last for several weeks in rodent species (e.g., up to 4, 5, or 6 weeks), and for several months in humans (e.g., up to 4, 5, 6, 7, 8, 10, 11 or 12 months). The early chronic phase is characterized by ongoing, persistent endogenous recovery of a neurological function impaired by a stroke, and in particular, sensorimotor function. In some embodiments, treatment in accordance with the invention comprises administering an aminopyridine or a pharmaceutically acceptable salt thereof to a patient during the period post-stroke during which spontaneous or endogenous recovery of neurological function, e.g., sensorimotor function, is observed. For example, a human patient can be treated in accordance with the invention during the early chronic phase at, or after, 6, 8, 10, 12, 14, 16, 18, 20, 22, 24 hours; 1, 2, 3, 4, 5, 6, 7, 8, 9 10 days; 1, 2, 3, 4, 5, 6 weeks, or 1, 2, 3, or 4 months post stroke, and before 4, 5, 6, 7, 8, 9, 10, 11, 12 months, or 1 year post-stroke.

Described herein is a method comprising administering an aminopyridine or a pharmaceutically acceptable salt thereof during the stable chronic phase post-stroke. The stable chronic phase post-stroke is characterized by little or no measurable spontaneous or endogenous improvement of neurological function impaired by a stroke, particularly sensorimotor function. Typically, in rodent species the stable chronic phase is reached after 4 to 6 weeks post-stroke; and in human species it is reached after 4 to 8 months (and, sometimes, after 1 year) post-stroke. The stable chronic phase often manifests as stable life-long disability, and particularly stable life-long sensorimotor impairment, that does not measurably improve in the absence of treatment. In certain embodiments, an aminopyridine or a pharmaceutically acceptable salt thereof is effective to improve a stroke-related sensorimotor impairment in a patient when administered during the stable chronic phase post-stroke. In some embodiments, treatment in accordance with the invention comprises administering an aminopyridine or a pharmaceutically acceptable salt thereof to a patient during the period post-stroke during which little or no measurable spontaneous or endogenous improvement of neurological function, e.g., sensorimotor function, is observed. For example, a human patient can be treated in accordance with the invention during the stable chronic phase at, or after, 4, 5, 6, 7, 8, 9, 10, 11, 12 months; 1, 2, 3, 4, 5, 7, 10, 12, 15, 20 years, or any time, post-stroke.

In one embodiment of the invention, treatment is initiated after the acute phase post-stroke. In one embodiment of the invention, treatment is initiated during and yet continues after the acute phase post-stroke. In one embodiment, treatment is initiated after the early chronic phase post-stroke. In another embodiment, treatment is initiated during and yet continues after the early chronic phase post-stroke. In yet another embodiment, treatment is initiated during the stable chronic phase post-stroke.

Therapeutic benefits of an aminopyridine (e.g., 4-aminopyridine) or a pharmaceutically acceptable salt thereof can be achieved by administering a therapeutically effective amount to a mammal. In certain embodiments, the treatment is performed before, at or after hour 1, hour 2, hour 6, hour 8, hour 12, hour 24, hour 30, hour 36, hour 42, day 2, day 3, day 4, day 5, week 1, week 2, week 3, week 4 or later following stroke. In certain embodiments, the treatment is performed at or after hour 3, hour 6, hour 8, hour 12, hour 24, hour 30, hour 36, hour 42, day 2, day 3, day 4, day 5, week 1, week 2, week 3, week 4, week 8 or later following stroke. In one embodiment, the treatment is performed at or after hour 6 following stroke. In one embodiment, the treatment is performed at or after hour 24 following stroke. In one embodiment, the treatment is performed at or after 7 days (1 week) following stroke. In one embodiment, the treatment is performed at or after 14 days (2 weeks) following stroke. In one embodiment, the treatment is performed at or after 1 month following stroke. In one embodiment, the treatment is performed at or after 4 months following stroke. In one embodiment, the treatment is performed at or after 6 months following stroke. In one embodiment, the treatment is performed at or after 8 months following stroke. In one embodiment, the treatment is performed at or after 12 months following stroke. Also specifically described is a method comprising administering an aminopyridine (e.g., 4-aminopyridine) or a pharmaceutically acceptable salt thereof to a mammal, where the administering is performed at least two, three, four, seven or ten days after the ischemic event, and in a therapeutically effective amount sufficient to promote improvement in a sensorimotor function during the early chronic phase and/or during the stable chronic phase following an ischemic event in a mammal. In certain embodiments, the treatment in accordance with the invention is performed any time post-stroke. Also specifically described is a method comprising administering an aminopyridine (e.g., 4-aminopyridine) or a pharmaceutically acceptable salt thereof to a mammal, where the administering is performed any time post-stroke, in an amount sufficient to promote improvement in a sensorimotor function.

In certain embodiments, the present invention comprises administration of an aminopyridine (e.g., 4-aminopyridine) or a pharmaceutically acceptable salt thereof to a mammal starting at day 1, 2 or 3, and up to and including days 4, 5, 6, 7, 8, 9, 10, 11, 12, 13, 14 days post-stroke; a week or more than one week post-stroke, two weeks or more than two weeks post-stroke; three weeks or more than three weeks post-stroke; four weeks or more than four weeks post-stroke; one month or more than one month post-stroke; two months or more than two months post-stroke; three months or more than three months post-stroke; four months or more than four months post-stroke; five months or more than five months post-stroke; six months or more than six months post-stroke. In certain embodiments, an aminopyridine (e.g., 4-aminopyridine) or a pharmaceutically acceptable salt thereof is administered to patients at or after 1, 2, 3, 4, 5, 6, 7 or 8 weeks post-stroke.

Also described is a method for promoting improvement in a neurological function, e.g., a sensorimotor function, during a period outside the acute phase following an ischemic event in a mammal is presented. In a specific embodiment, treatment in accordance with the invention can begin within the acute phase but includes at least one, two three, four, five, six or more than six treatments beyond the acute phase.

In certain embodiments, the administering step begins within: 1, 2, 3, 4, 5, 6, 7, 8, 9, 10, 11, 12, 13, 14, 15, 16, 17, 18, 19, 20, 21, 22, 23 or 24 hours; 1, 2, 3, 4, 5, 6, 7, 8, 9, 10, 11, 12, 13, 14, 15, 16, 17, 18, 19, 20, or 21 days; 1, 2, 3, 4, 5, 6, 7, 8, 9, 10, 11, 12, 13, 14, 15, 16, 17, 18, 19, 20, 21, 22, 23 or 24 weeks; 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 42, 48, 54, 60, or 66 months; 0.5, 1, 1.5, 2, 2.5, 3, 3.5, 4, 4.5, 5, 5.5, 6, 7, 8, 9, 10, 12, 15, 20, 25, or 30 years, or later, following a stroke. In other embodiments, the administering step begins after: 1, 2, 3, 4, 5, 6, 7, 8, 9, 10, 11, 12, 13, 14, 15, 16, 17, 18, 19, 20, 21, 22, 23 or 24 hours; 1, 2, 3, 4, 5, 6, 7, 8, 9, 10, 11, 12, 13, 14, 15, 16, 17, 18, 19, 20, or 21 days; 1, 2, 3, 4, 5, 6, 7, 8, 9, 10, 11, 12, 13, 14, 15, 16, 17, 18, 19, 20, 21, 22, 23 or 24 weeks; 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 42, 48, 54, 60, or 66 months; 0.5, 1, 1.5, 2, 2.5, 3, 3.5, 4, 4.5, 5, 5.5, 6, 7, 8, 9, 10, 12, 15, 20, 25, or 30 years, or later, following a stroke.

Also described is a method of treating a stroke-related sensorimotor impairment in a patient: comprises administering a therapeutically effective amount of an aminopyridine (such as 3,4-diaminopyridine, 4-aminopyridine and the like) or a pharmaceutically acceptable salt thereof to said patient for a period of time. In certain embodiments, the administering step *begins within:* 1, 2, 3, 4, 5, 6, 7, 8, 9, 10, 11, 12, 13, 14, 15, 16, 17, 18, 19, 20, 21, 22, 23 or 24 hours; 1, 2, 3, 4, 5, 6, 7, 8, 9, 10, 11, 12, 13, 14, 15, 16, 17, 18, 19, 20, or 21 days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 42, 48, 54, 60, or 66 months; 0.5, 1, 1.5, 2, 2.5, 3, 3.5, 4, 4.5, 5, 5.5, 6, 7, 8, 9, 10, 12, 15, 20, 25, or 30 years, or later, following a stroke event. In a further embodiment of the forgoing, the administering step *continues for* a period of at least, or more than: 1, 2, 3, 4, 5, 6, 7, 8, 9, 10, 11, 12, 13, 14, 15, 16, 17, 18, 19, 20, 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, and 66 months; 0.5, 1, 1.5, 2, 2.5, 3, 3.5, 4, 4.5, 5, 5.5, 6, 7, 8, 9, 10, 12, 15, 20, 25, 30, or 35 years.

In some embodiments, the treatment regimen (a particular dose and frequency of administration, which can be selected from any described herein) is stable over a period of time, e.g., for at least 4 days, at least 1 week, at least 2 weeks, at least 3 weeks, at least 1 month, at least 2 months, at least 3 months, at least 4 months, or at least 6 months.

Also described herein is a method of effectively treating a stroke-related sensorimotor impairment, in a patient over a short-term, initial, or non-chronic phase comprising administering a therapeutically effective amount of an aminopyridine (such as 3,4-diaminopyridine, 4-aminopyridine and the like) or a pharmaceutically acceptable salt thereof to said patient. In certain embodiments provided herein the patient is treated with an aminopyridine or a pharmaceutically acceptable salt thereof for a period of 1, 2, 3, 4, 5, 6, 7, 8, 9, 10, 11, 12, 13, 14, 15 day(s); 1, 2, 3, 4, 5, 6, 7, 8, 9, 10, 11, 12, 13, 14, 15 weeks; 1, 2, 3, or 4 months. It is understood that one can continue beyond such period and still be within the scope of the invention.

Also described is a method of effectively treating a stroke-related sensorimotor impairment in a patient over an early chronic and/or a stable chronic phase comprising administering a therapeutically effective amount of an aminopyridine (such as 3,4-diaminopyridine, 4-aminopyridine and the like) or a pharmaceutically acceptable salt thereof to said patient for an extended period of time. Also described herein is a method of durably treating a stroke-related sensorimotor impairment, comprising: administering a therapeutically effective amount of an aminopyridine (such as 3,4-diaminopyridine, 4-aminopyridine and the like) or a pharmaceutically acceptable salt thereof to said patient for an extended period of time. In some embodiments, the extended period is at least or is more than: 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 6, 7, 8, 9, 10 or greater than 10 years.

In a certain embodiment, a therapeutically effective amount of an aminopyridine, e.g., 4-aminopyridine, or a pharmaceutically acceptable salt thereof is administered intravenously during the acute phase following a stroke. In some embodiments, a therapeutically effective amount of an aminopyridine, e.g., 4-aminopyridine, or a pharmaceutically acceptable salt thereof is administered intravenously within 1, 2, 3, 4, 5, 6, 7 days, or 1, 2, 3, 4, 8 weeks post-stroke. Intravenous administration can occur once daily, twice daily, three times daily, once in two days, once every three days, or once a week. In one embodiment, the patient is treated with a single intravenous administration of an aminopyridine or a pharmaceutically acceptable salt thereof.

In another embodiment, a therapeutically effective amount of an aminopyridine, e.g., 4-aminopyridine, or a pharmaceutically acceptable salt thereof is administered orally during the acute phase, the early chronic phase and/or the stable chronic phase following stroke. In a specific embodiment, a therapeutically effective amount of an aminopyridine, e.g., 4-aminopyridine, or a pharmaceutically acceptable salt thereof is administered orally only during the early chronic phase and/or the stable chronic phase post-stroke. Oral administration can occur once daily, twice daily, three times daily, or more than three times daily, in either an immediate or in a sustained release composition.

Administering of an aminopyridine compound may be accomplished by various techniques as described herein. The administering of an aminopyridine or a pharmaceutically acceptable salt thereof according to the invention can be carried out, e.g., by administering the compound into or onto the target tissue; providing the compound systemically to a patient by, e.g., intravenous injection (e.g., parenteral) or oral administration (e.g., enteral) or topical administration (e.g., transdermal, transcutaneous, patch, suppository) or inhalation (e.g., transmucosal), whereby the compound reaches the target tissue. Administering of the aminopyridine or a pharmaceutically acceptable salt thereof to a patient can be by the patient himself or herself or by a caregiver, such as a medical professional; including the act of ingestion by or application to the patient or the like wherein the compound can exert its effects.

In one embodiment, an aminopyridine or a pharmaceutically acceptable salt thereof is administered locally, i.e, by direct administration by a non-systemic route at or in the vicinity of the site of affliction, disorder, or perceived pain.

In certain embodiments, a patient is treated intravenously beginning within 1, 2, 3, 4, 5, 6, 7, 8, 9, 10, 11, 12, 14, 16, 18, 20, 22 hours, or beginning on day 1, 2, 3, 4, 5, 6 or 7, after the stroke with an aminopyridine (e.g., 4-aminopyridine) or a pharmaceutically acceptable salt thereof at a dose between 0.01 and 1.0 mg/kg per dose, once a day, twice a day, every other day, or once a week, for more than 1, 2, 3, 4, 5, 6, 7, 8, 9, or 10 days, more than 1, 2, 3, 4, 5, 6, 7 or 8 weeks, or more than 1, 2, 3, 4, 5 months (or between 1 day and 5 days, between 2 days and 10 days, between 10 days and 1 month, between 10 days and 6 months, or between 10 days and 1 year).

Alternatively, a patient is treated orally beginning on day 1, day 2, day 3, day 4, day 5, day 6 or day 7 (or after day 1, 2, 3, 4, 5, 6 or 7), beginning, or after, 1 week, 2 weeks, 3 weeks, 4 weeks, 5 weeks, 6 weeks, 7 weeks, or 8 weeks, or beginning, or after, 1 month, 2 months, 3 months, 4 months, 6 months, 8 months, 10 months, or 12 months, post-stroke with an aminopyridine (e.g., 4-aminopyridine) or a pharmaceutically acceptable salt thereof in the amount between 4 mg and 17.5 mg (e.g., 4, 5, 6, 7, 8, 9, 10, 11, 12, 13, 14, 15, 16 or 17 mg), once daily or twice daily, for more than 5, 10, 15, 20 days, more than 1, 2, 3, 4, 5, 6, 7, 8 weeks, more than 1, 2, 3, 4, 5, 6, 9 months, or more than 1, 2, 3, 4, 5, 10, 15, 20 years (or between 10 days and 3 months, between 10 days and 6 months, between 10 days and 1 year, between 3 months and 1 year, between 6 months and 1 year, between 6 months and 5 years, or between 1 year and 50 years). In some embodiments, a patient is treated beginning at 4 weeks post-stroke (or after 4 weeks post-stroke) with 5 mg, 7.5 mg, 10 mg or 12.5 mg 4-aminopyridine twice daily. In other embodiments, a patient is treated beginning at 4 months post-stroke (or after 4 months post-stroke) with 5 mg, 7.5 mg, 10 mg or 12.5 mg 4-aminopyridine twice daily. In yet other embodiments, a patient is treated beginning at, or after, 4, 5, 6, 7, 8 weeks, or 3, 4, 5, 6, 7, or 8 months post-stroke with 8 mg, 10 mg, 12 mg, 12.5 mg, 15 mg, 20 mg or 25 mg of 4-aminopyridine once daily.

### 5.4 Combination treatments

The compositions and methods described herein may be used in the context of a number of therapeutic or prophylactic applications. In order to increase the effectiveness of a treatment with the aminopyridines, or to augment the protection of another therapy (second therapy), it may be desirable to combine these compositions and methods with other agents and methods effective in the treatment of diseases and pathologic conditions, for example, sensorimotor impairments, etc., associated with stroke.

Thus, in a specific embodiment, one can combine an aminopyridine or a pharmaceutically acceptable salt thereof with one or more other agents and/or physical or occupational therapies for the treatment of the stroke-related impairment, for example, a sensorimotor impairment. In some embodiments, an aminopyridine or a pharmaceutically acceptable salt thereof is administered to a patient concomitantly or sequentially with one or more additional drugs or therapies. For example, an aminopyridine or a pharmaceutically acceptable salt thereof can be administered to a patient at the same time, before, or after administration of another drug effective for the stroke-related impairment. Such other drug can be, for example, a cholinesterase inhibitor such as donepezil, rivastigmine, or galantamine, or an immunomodulator such as interferon. In particular embodiments, the combination of an aminopyridine or a pharmaceutically acceptable salt thereof and one, two, or more additional drug(s) is a fixed dose combination. For example, an aminopyridine or a pharmaceutically acceptable salt thereof and one or more additional drug(s) (such as any of those other drugs described above) can be formulated in one composition, such as a pill, a tablet, or a capsule. In other embodiments, an aminopyridine or a pharmaceutically acceptable salt thereof is administered to a patient who has suffered a stroke concomitantly (e.g., at the same time, before or after) with physical therapy, occupational therapy, or speech therapy, etc. In some embodiments, an aminopyridine or a pharmaceutically acceptable salt thereof is administered to a patient who uses a brace, standing frame or other orthotic device such as a rolling walker, or communication aid such as a computer with attached voice synthesizer. In a specific embodiment, the aminopyridine (or salt thereof) and other drug or therapy is administered at the same doctor's visit, or within 1, 2, 3, 4, 5, 6, or 12 hours, or within 1, 2, 3, 4, 5, 6, or 7 days, of each other.

Various combinations may be employed; for example, an aminopyridine or a pharmaceutically acceptable salt thereof, is "A" and the secondary therapy (*e.g.,* cholinesterase inhibitors such as donepezil, rivastigmine, and galantamine and immunomodulators such as interferon, etc.) is "B", nonlimiting combination cycles include:
A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

Administration of a composition of the present invention to a subject will follow general protocols for the administration described herein, and the general protocols for the administration of a particular secondary therapy will also be followed, taking into account the toxicity, if any, of the treatment. It is expected that the treatment cycles would be repeated as necessary. It also is contemplated that various standard therapies may be applied in combination with the described therapies.

In some embodiments, an aminopyridine or a pharmaceutically acceptable salt thereof is administered to a patient concomitantly with Occupational or Physical Therapy. In other embodiments, an aminopyridine or a pharmaceutically acceptable salt thereof is administered to a patient after the patient has been subjected to Occupational or Physical Therapy post-stroke. In another embodiment, an aminopyridine or a pharmaceutically acceptable salt thereof is administered to a patient without Occupational or Physical Therapy. In one embodiment, a patient treated in accordance with the methods described herein does not concomitantly receive Occupational or Physical Therapy. In yet another embodiment, a patient treated in accordance with the methods described herein has not been subjected to Occupational or Physical Therapy post-stroke. In certain embodiments, treatment in accordance with the invention (either with or without use of Occupational or Physical Therapy) is more effective than Occupational or Physical Therapy alone.

### 5.5 Kits

Kits comprise an exemplary embodiment of the invention. The kit can comprise an outer receptacle or container configured to receive one or more inner receptacles/containers, utensils and/or instructions. A utensil in accordance with the invention can comprise item(s) to administer the drug, such as a patch, inhalation apparatus, fluid container cup, syringe or needle. A composition containing an aminopyridine or a pharmaceutically acceptable salt thereof can be comprised within a receptacle of the invention. A receptacle of the invention can contain sufficient quantity of an aminopyridine or a pharmaceutically acceptable salt thereof to be useful for multiple doses, or may be in unit or single dose form. In certain embodiments, a kit comprises a composition containing an aminopyridine or a pharmaceutically acceptable salt thereof in the form of a tablet, a pill, a blister pack, or a capsule.

Kits of the invention generally comprise instructions for administration in accordance with the present invention. The instructions can comprise treating one or more of: ataxia, global body control impairments, coordination or balance impairments, impairment in body sense, endurance impairment, impairment in hand function, fine hand coordination loss or impairment, hyperreflexia, impairment in grip strength, muscle weakness, muscle tone impairment, range of motion impairments, spasticity, strength impairment/weakness, tremor, impairment in limb function, upper extremity function impairment, lower extremity function impairment, impairment in lower extremity muscle strength, walking impairments (e.g., decreased walking speed), dysarthria, impairment in jaw function, impairment in chewing, or impairment in jaw articulation. Any mode of administration set forth or supported herein can constitute some portion of the instructions.

In one embodiment, the instructions indicate that an aminopyridine or a pharmaceutically acceptable salt thereof is to be taken twice-daily. In one embodiment, the instructions indicate that an aminopyridine or a pharmaceutically acceptable salt thereof is to be taken once daily. In one embodiment, the instructions indicate that the composition containing an aminopyridine or a pharmaceutically acceptable salt thereof is to be taken one or more than one times during the acute phase post-stroke. In one embodiment, the instructions indicate that the composition is to be taken one or more than one times during the early chronic phase and/or during the stable chronic phase post-stroke.

The instructions may be affixed to any container/receptacle of the invention. In one embodiment, the instructions indicate that an aminopyridine or a pharmaceutically acceptable salt thereof is to be taken such as to or in order to achieve a therapeutic range in accordance with the present invention. The instructions may be affixed to any container/receptacle of the invention or may be a separate sheet within a container or receptacle of the invention. Alternatively, the instructions can be printed on, embossed in, or formed as a component of a receptacle of the invention. Alternatively, the instructions can be printed on a material that is enclosed within a receptacle or container of the kit of the invention. In one embodiment, a kit has an outer receptacle, such as a box, within which there is a container, such as a bottle; and instructions are provided on and/or within the outer receptacle and/or the bottle. A kit can also include instructions for employing the kit components as well the use of any other reagent not included in the kit; it is contemplated that such reagents are embodiments of kits of the invention. In accordance with the invention, kits are not limited to the particular items identified above and may include any reagent used directly or indirectly in the treatment sought.

### 5.6 Additional Embodiments:

Also described are methods of effectively treating stroke-related sensorimotor impairment, in a patient over a chronic or extended or prolonged or protracted or sustained time period; this is also referred to as a "durable" treatment or a "durable" method of treatment; this is also referred to as a "sustained" treatment or a "sustained" method of treatment. Also described are methods of maintaining improvement of a stroke-related sensorimotor impairment in a patient comprising administering a therapeutically effective amount of an aminopyridine (e.g., 4-aminopyridine) to said patient after previously achieving an improvement of a stroke-related sensorimotor impairment in said patient during contiguous or continuing or prior administration of an aminopyridine. Any of such methods comprise administering a therapeutically effective amount of an aminopyridine (e.g., 4-aminopyridine) to said patient for an extended, prolonged, protracted, sustained or chronic period of time (as used herein, extended, prolonged, protracted, sustained, and chronic are synonyms unless the context clearly indicates otherwise). In certain embodiments, the extended, prolonged, protracted or chronic or sustained period is at least or more than: 8, 9, 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 6, or greater than 5 years. In certain embodiments, the extended, prolonged, protracted, chronic or sustained period is for the lifetime of the patient. These methods can also comprise administering an aminopyridine at or to a therapeutic level (such as Cₘᵢₙₛₛ or an average Cₘᵢₙₛₛ) or range (such as a Cₘᵢₙₛₛ range or a reference range of average Cₘᵢₙₛₛ values) in accordance with the present invention.

In one embodiment, an amount of drug is given to an individual patient (e.g., a dose amount) wherein that dose amount corresponds to a dose that when administered to a normative or reference population obtains an average Cₘᵢₙₛₛ of at least, or more than: 6, 7, 8, 9, 10, 11, 12, 13, 14, 15, 16, 17, 18, 19 or 20 ng/ml.

In certain embodiments, the therapeutically effective amount of 4-aminopyridine is 10 milligrams in a sustained release composition administered twice daily. Methods of administration can also comprise administering the 4-aminopyridine at or to a therapeutic level (such as Cₘᵢₙₛₛ) or range (such as a Cₘᵢₙₛₛ range) in accordance with the present invention.

Also described are methods of maintaining improved sensorimotor function, e.g., global body control, coordination, balance, body sense, endurance, hand function, fine hand coordination, grip strength, muscle tone, range of motion, strength, limb function, upper extremity function, lower extremity function, lower extremity muscle strength, walking (e.g., walking speed), dysarthria, jaw function, chewing, or jaw articulation, in a patient with a stroke-related impairment of one of these sensorimotor functions, comprising administering a therapeutically effective amount of an aminopyridine (e.g., 4-aminopyridine) to said patient over an extended period of time. In certain embodiments, the extended, prolonged, protracted, sustained or chronic period is at least or more than: 8, 9, 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 6, or greater than 5 years. In certain embodiments, the extended, prolonged, protracted, chronic or sustained period is for the lifetime of the patient. This maintenance can be relatively consistent in that there is an essentially uniform percentage improvement relative to a reference or normative population, or this maintenance can be relatively varied in that there is a fluctuating percentage improvement relative to a reference or normative population; when the maintenance is relatively varied this can include periods when the subject patient may do worse relative to a reference or normative population.

Also described are methods of achieving sustained or relatively sustained improvement in any one or more sign or symptom of stroke, such as any one or more sensorimotor impairments induced by or related to a stroke, comprising continuing administration of a therapeutically effective amount of an aminopyridine (e.g., 4-aminopyridine) to said patient over an extended period of time. With regard to a control or standard amount or value, it is understood that sometimes there is progressive decline of sensorimotor function in patients after a stroke, and that an increase or relative increase can properly be considered in regard to the decline in function attendant to the inherent progress of stroke-related sensorimotor pathology. In certain embodiments, the sustained improvement occurs for an extended period, such as for at least or more than: 8, 9, 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 6, or greater than 5 years. In certain embodiments, the extended period is for the lifetime of the patient. In certain embodiments, the therapeutically effective amount of 4-aminopyridine is 10 milligrams in a sustained release composition. In certain embodiments, the sustained release composition can be administered twice daily. In certain embodiments, the sustained release composition may be administered once daily. These methods can also comprise administering an aminopyridine at or to a therapeutic level (such as Cₘᵢₙₛₛ) or range (such as a Cₘᵢₙₛₛ range) in accordance with the present invention. This sustained improvement can be relatively growing in that there is an ongoing growth in a percentage improvement relative to a reference or normative population, or this improvement can be relatively varied in that there is a fluctuating percentage improvement relative to a reference or normative population such that there is a tendency to do better than the reference group; when the improvement is relatively varied this can include periods when the subject patient may do worse relative to a reference or normative population.

In certain embodiments, the therapeutically effective amount of an aminopyridine (e.g., 4-aminopyridine) is a stable or constant or consistent or unchanging or unwavering or unaltered dosing regimen that comprises a therapeutically effective amount of an aminopyridine that is administered at a uniform pattern (e.g., a milligram amount or particular milligram amount at particular times of day, e.g. there may be a higher dose in the morning and a lower dose in the evening or vice versa) and on a uniform schedule (e.g., twice daily), wherein no changes of the dose amount or schedule occurs during the stable or constant or consistent or unchanging or unwavering dosing regimen. As used herein, the terms "stable" or "constant" or "consistent" or "unchanging" or "unwavering" or "unaltered" are synonyms unless the context clearly indicates otherwise. It is to be understood that, e.g., occasional patient noncompliance or deviation from an otherwise stable, constant, consistent, unchanging, unwavering, or unaltered course of treatment is within the definition of such treatment. In certain embodiments, no titration (whether an increase or decrease) of the dose (e.g., milligram amount) of an aminopyridine occurs during the entirety of the stable dosing regimen.

Also described are methods of treating or ameliorating a stroke-related sensorimotor impairment in a patient comprising administering an amount or range of 4-aminopyridine to said patient such that a minimum concentration at steady state (Cₘᵢₙₛₛ) in a range of at least 5 ng/ml to 20 ng/ml, 10 ng/ml to 20 ng/ml, or 12 ng/ml to 20 ng/ml is obtained, or a Cₘᵢₙₛₛ in a range of 20 ng/ml is obtained. Also described are methods of treating or ameliorating a stroke-related sensorimotor impairment in a patient comprising administering an amount or range of 4-aminopyridine to said patient such that an average minimum concentration at steady state (average Cₘᵢₙₛₛ) in a range of at least 7 ng/ml to 20 ng/ml, or 12 ng/ml to 20 ng/ml is obtained, or an average Cₘᵢₙₛₛ in a range of 20 ng/ml is obtained. In certain embodiments, a Cₘᵢₙₛₛ in a range of 20 ng/ml achieves a Cₘᵢₙₛₛ of about 20 ng/ml. In other embodiments, a Cₘᵢₙₛₛ of about 20 ng/ml is obtained; in certain embodiments, a Cₘᵢₙₛₛ in a range of 20 ng/ml comprises a lower limit value of from 11, 12, 13, 14, 15, 16, 17, 18, 19, or 20 ng/ml, and an upper limit value of 20, 21, 22, 23, 24, 25, 26, or 27 ng/ml. In certain embodiments, a Cₘᵢₙₛₛ in a range of at least 12 ng/ml to 15 ng/ml is obtained. In certain embodiments, a Cₘᵢₙₛₛ in a range of at least 13 ng/ml to 15 ng/ml is obtained. In certain embodiments, a Cₘᵢₙₛₛ in a range of at least 15 ng/ml to 25 ng/ml is obtained. In certain embodiments, a Cₘᵢₙₛₛ of at least or more than 11, 12, 13, 14, 15, 16, 17, 18, 19, 20, 21, 22, 23, 24, or 25 ng/ml is obtained. In other embodiments, an average Cₘᵢₙₛₛ of about 20 ng/ml is obtained; in certain embodiments, an average Cₘᵢₙₛₛ in a range of 20 ng/ml comprises an average lower limit value of from 11, 12, 13, 14, 15, 16, 17, 18, 19, or 20 ng/ml, and an average upper limit value of 20, 21, 22, 23, 24, 25, 26, or 27 ng/ml. In certain embodiments, an average Cₘᵢₙₛₛ in a range of at least 12 ng/ml to 15 ng/ml is obtained. In certain embodiments, an average Cₘᵢₙₛₛ in a range of at least 13 ng/ml to 15 ng/ml is obtained. In certain embodiments, an average Cₘᵢₙₛₛ in a range of at least 15 ng/ml to 25 ng/ml is obtained. In certain embodiments, an average Cₘᵢₙₛₛ of at least or more than 11, 12, 13, 14, 15, 16, 17, 18, 19, 20, 21, 22, 23, 24, or 25 ng/ml is obtained.

Alternatively described is a method (e.g., for treating a stroke-related sensorimotor impairment or a method for improving a symptom of a stroke-related sensorimotor impairment in a patient or a method for obtaining a therapeutically effective level of an aminopyridine in a patient with a stroke-related sensorimotor impairment) comprising administering an aminopyridine (e.g., 4-aminopyridine) to said patient such that a Cₘᵢₙₛₛ in a range of 5-12 ng/ml is obtained; a Cₘᵢₙₛₛ in a range of 10-20 ng/ml is obtained; a Cₘᵢₙₛₛ in a range of 15-25 ng/ml is obtained; a Cₘᵢₙₛₛ in a range of 15-30 ng/ml is obtained; a Cₘᵢₙₛₛ in a range of 17-23 ng/ml is obtained; a Cₘᵢₙₛₛ in a range of 18-22 ng/ml is obtained; or a Cₘᵢₙₛₛ is in a range of 19-21 ng/ml is obtained. In specific embodiments, the Cₘᵢₙₛₛ is in a range where the lower value is selected from the group of 5, 6, 7, 8, 9, 10, 11, 12, 13, 14, 15, 16, 17, 18, 19, 20 ng/ml and an upper value is selected from the group of 20, 21, 22, 23, 24, 25, 26 or 27 ng/ml, it being understood that this indicates that any particular combination is contemplated, e.g., without limitation a range of: 16-23 ng/ml, 12-24 ng/ml, 13-27 ng/ml, etc.

In certain embodiments, the therapeutically effective amount of an aminopyridine (e.g., 4-AP) is administered to obtain a Cₘᵢₙₛₛ or an average Cₘᵢₙₛₛ (or respective range thereof) for an extended period, which is at least or more than: 8, 9, 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 6, or greater than 5 years. In certain embodiments, the extended period is for the lifetime of the patient.

Also described is a method of treating a stroke-related sensorimotor impairment or the symptoms thereof comprising administering a therapeutically effective amount of 4-aminopyridine to said patient such that average plasma concentration of about 13 ng/ml to about 15 ng/ml is obtained and the average maximum plasma concentration is not greater than about 15 ng/ml.

Described herein is a method of treating a stroke-related sensorimotor impairment or the symptoms thereof comprising administering a therapeutically effective amount of 4-aminopyridine to said patient such that average plasma concentration at steady state (Cₐᵥₛₛ) of about 15 ng/ml to about 27 ng/ml is obtained. Described herein is a method of treating a stroke-related sensorimotor impairment or the symptoms thereof comprising administering a therapeutically effective amount of 4-aminopyridine to said patient such that average plasma concentration at steady state (Cₐᵥₛₛ) of about 20 ng/ml to about 40 ng/ml is obtained. Described herein is a method of treating a stroke-related sensorimotor impairment or the symptoms thereof comprising administering a therapeutically effective amount of 4-aminopyridine to said patient such that average plasma concentration at steady state (Cₐᵥₛₛ) of about 10 ng/ml to about 20 ng/ml is obtained. Described herein is a method of treating a stroke-related sensorimotor impairment or the symptoms thereof comprising administering a therapeutically effective amount of 4-aminopyridine to said patient such that average plasma concentration at steady state (Cₐᵥₛₛ) of about 5 ng/ml to about 15 ng/ml is obtained.

In certain methods described herein, patients are identified and treated in accordance with the methods described herein, which have or are suspected of having a stroke-related sensorimotor impairment, and which do not have or are not suspected of having multiple sclerosis.

In certain embodiments, the improvement in a stroke-related sensorimotor impairment may be at least about (or more than) 4, 5, 6, 7, 8, 9, 10, 11, 12, 13, 14, 15, 16, 17, 18, 19, or 20%. In certain embodiments, the improvement may be at least about (or more than) 20, 21, 22, 23, 24, 25, 26, 27, 28, 29 or 30%. In certain embodiments, the improvement may be at least about 20%. In certain embodiments, the improvement may be at least about 25%. In certain embodiments, the improvement may be at least about (or more than) 30, 31, 32, 33, 34, 35, 36, 37, 38, 39 or 40%. In certain embodiments, the improvement may be at least about 40%. In certain embodiments, the improvement may be at least about 45%. In certain embodiments, the improvement may be at least about (or more than) 40, 41, 42, 43, 44, 45, 46, 47, 48, 49 or 50%. In certain embodiments, the improvement may be at least about 50%. In certain embodiments, the improvement may be at least about or more than 55%. In certain embodiments, the improvement may be at least about 60%. In certain embodiments, the improvement may be at least about or more than 65%. In certain embodiments, the improvement may be at least about or more than 70%. In certain embodiments, the improvement may be at least about or more than 75%. In certain embodiments, the improvement may be at least about or more than 80%. In certain embodiments, the improvement may be at least about or more than 85%. In certain embodiments, the improvement may be at least about or more than 90%. In certain embodiments, the improvement may be at least about or more than 95%. In certain embodiments, the improvement may be at least about 100%. In certain embodiments, the improvement may be more than about 100%. In certain embodiments, the improvement may be more than about 150%. In certain embodiments, the improvement may be more than about 200%. In certain embodiments, the improvement may be more than about 250%. In certain embodiments, the improvement may be more than about 300%. In certain embodiments, the improvement may be from: 4-100%, 4-20%, 5-20%, 6-20%, 7-20% , 8-20%, 9-20%, 10-20%, 10-30%, 10-60%, 20-30%, 20-40%, 20-50%, 20-60%, 20-100%, 30-100%, 50-100%, 30-150%, 50-150%, 100-150%, 100-200%, 50-250%, 100-250% or 100-300%. Such percent change quantification is preferably applied to assays of sensorimotor function that provide measurements of results in continuous linear scales, such as T25FW, etc.

Also described herein are methods of monotonically improving a stroke-related sensorimotor impairment in a patient comprising administering a therapeutically effective amount of an aminopyridine (e.g., 4-AP) to said patient for an extended period of time. In certain embodiments, the extended period is at least 8, 9, 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 6, or greater than 5 years. In certain embodiments, the extended period is for the lifetime of the patient. As used herein, monotonic increase in a parameter is a consistent increase without any decrease from baseline (i.e., prior to treatment with an aminopyridine).

Various parameters known as quality of life or activities of daily living are known in the art. These parameters can be measured in order to assess the improvement of a condition (e.g., a sensorimotor function) in a patient who had suffered a stroke after a period of treatment in accordance with the invention. These include, e.g., Impact of Impairment on Daily Life:
- Navigate between rooms in one's own home
- Go to the bathroom
- Shower
- Care for one's children
- Cross the street safely
- Stay employed
- Shop for groceries
- Cook a meal
- Climb stairs
- Exercise
- Participate in social activities.

Also described are methods which allow a subject to achieve any of the forgoing where they could not achieve such activity(s) before. Also described are methods which allow a subject to achieve any of the forgoing better, where they were limited in their ability to achieve such activity(s) before.

Also described are methods which allow for maintaining improvement of a symptom, parameter, characteristic, value, finding or manifestation of a stroke-related sensorimotor impairment, where such symptom, parameter, characteristic, value, finding or manifestation was previously effectively addressed by an aminopyridine, by administering a therapeutically effective amount of an aminopyridine to said patient (after previously achieving an improvement of such symptom, parameter, characteristic, value, finding or manifestation). The previous period of efficacy can be 10, 11, 12, 13, 14, 15, 16 , 17 or 18 weeks; 3, 4, 5, 6, 7, 8, 9, 10, 11, 12 or 13 months; 1, 2, 3, 4, 5, 6, 7, 8, 9, 10, or more than 10 years.

### 6. EXAMPLES

### 6.1 Example 1: Rodent Stroke Model

Male rats (about 300-400 g, Sprague-Dawley) undergo surgery to induce brain ischemic injury. Animals are dosed orally with 4-AP (0.1, 0.3 and 1 mg/kg) for single dosing or daily dosing for 2 weeks, initiating at 1 day, 10 days and 4 weeks after ischemic insult. Neurological behaviors are evaluated by performing tests of forelimb placing, hind limb placing, body swing, cylinder test and activity box at 4 hrs [equal to 1 hr after Cmax (∼3 hrs)] after the dosing (for single treatment) or the last dosing (for multiple treatment). Neurological functions are also evaluated after a period of drug withdrawal. Further, small blood samples (100 µL) are taken from the lateral tail veins at multiple time points following the vehicle or 4-AP dose to establish plasma concentrations of 4-AP using HPLC-MS/MS methodology. This blood sampling allows for determination of plasma exposure around the time when animals are being assessed for neurological improvements.

At the end of the experiment, animals are deeply anesthetized with pentobarbital and perfused transcardially with PBS and paraformaldehyde for brain infarction volume measurement and the degree of neuronal damage is assessed with H&E staining and Luxol fast blue staining, respectively.

Table 1 shows a summary of treatment groups and endpoints.

**Table 1. Summary of treatments groups and endpoints**

| Dosing frequency | Dosing Starts at | Dose Level (mg/kg) | Neurological Assessments | Infarction measurement | Histological Assessments |
|---|---|---|---|---|---|
| Single | 1 day, 10 days, 4 weeks | 0, 0.1, 0.3, and 1.0 | forelimb placing, hind limb placing, body swing, cylinder, activity box tests | H&E staining | Myelination by Luxol Fast Blue staining, Axonal survival |
| Multiple (14 consecutive days) | 1 day, 10 days, and 4 weeks | 0, 0.1, 0.3, and 1.0 | forelimb placing, hind limb placing, body swing, cylinder, activity box tests | H&E staining | Myelination by Luxol Fast Blue staining, Axonal survival |

Upon completion of the study, neurologic function following brain infarction is assayed for neurological function improvement, relative infarction reduction, relative myelination and/or axonal survival (as described in Table 1).

### 6.2 Example 2: Effects of Oral Administration of 4-AP: Functional Recovery Following MCA occlusion (MCAO) in Rats. Blinded, vehicle-controlled double crossover study.

4-AP was evaluated for its ability to promote functional sensorimotor improvement following ischemic stroke in rats with stable motor deficits at times remote from their ischemic events. The animal model mimics the conditions in human ischemic stroke and is produced by middle cerebral artery occlusion (MCAO), which results in extensive infarction in cerebral cortex and striatum, including the cortical spinal tract (white matter).

In particular, the MCAO model in Sprague Dawley rats that was utilized in the experiments presented below mimics the conditions in human ischemic stroke. In this model, focal cerebral infarcts were made by permanent occlusion of the proximal right middle cerebral artery (MCA) using a modification of the method of Tamura et al. (No To Shinkei 1986; 38:747-51). Briefly, the temporalis muscle was bisected and reflected through an incision made midway between the eye and the ear canal. The proximal MCA was exposed through a subtemporal craniectomy without removing the zygomatic arch and without transecting the facial nerve. The artery was then occluded by microbipolar coagulation from just proximal to the olfactory tract to the inferior cerebral vein and was transected.

The MCAO model described in this example results in a recovery pattern that in many ways parallels the typical human pattern of neurological recovery after stroke. Following MCAO there is an immediate and complete loss of sensorimotor function at Day 1 after surgery as measured with specific tactile, proprioceptive and sensory tests (forelimb and hindlimb placing and body swing symmetry). This is followed by a relatively rapid partial recovery period over the first several weeks. In the described MCAO model the recovery begins to plateau by 4 weeks after MCAO, at which time there are still measurable deficits in sensorimotor function. A similar, but slower recovery pattern occurs in humans over the first several months after stroke (see Cramer, Ann Neurol 2008; 63:272-87).

### Experimental Design

In this experiment, Sprague Dawley rats were anesthetized, subjected to a surgery resulting in middle cerebral artery occlusion (MCAO), treated with and without 4-aminopyridine and behavioral assessments were performed as described below. Treatment was initiated at 4 weeks after stroke.

Animals: 45 male Sprague Dawley Rats, 300-400 g (obtained from Charles River Laboratories, which arrived 7-10 days before surgery at 250-275 g) were used. Animals were randomly assigned to treatment groups.

Nomenclature: The nomenclature for the days of the study is as follows: Day 0 is the day of the MCAO, and the days following are numbered consecutively (Day 1, Day 2, Day 3, etc.); Day -1 represents the day prior to the MCAO.

Grouping details: The amount of time needed for some procedures in this study necessitated breaking up the 3 treatment groups (as listed below), into 8 working groups (as written in the schedule, see below). Six animals received stroke surgery per day. If an animal died during the 8-day surgical period of the study, it was replaced by a spare. If not, the animal was not replaced. Most animal deaths (<5% overall) occurred in the immediate post-op to 7 day period.

Anesthesia: 1-3% isoflurane in N₂O : O₂ (2:1). Anesthesia was induced in an induction chamber with 2-3% isoflurane in N2O:O2 (2:1), and maintained with 1-1.5% isoflurane via face mask. Adequate depth of anesthesia was assessed by lack of withdrawal to hindlimb pinch and loss of eyeblink reflex. Once anesthetized, animals received cefazolin sodium (40 mg/kg, i.p.) and buprenorphine (0.1 mg/kg, s.c.). Cefazolin was used as a prophylactic antibiotic for this procedure (because it ensures a negligible infection rate). A veterinary ophthalmic ointment, Lacrilube, was applied to the eyes.

Surgical Procedure: A small focal stroke (infarct) was made on the right side of the surface of the brain (cerebral cortex) by middle cerebral artery occlusion (MCAO). The right side of the head was shaved with electric clippers (patch of approx 3 X 5 cm between eye and ear). The region was carefully cleaned with septisol. Using aseptic technique, an incision was made midway between the eye and eardrum canal. The temporalis muscle was isolated, bisected, and reflected. A small window of bone was removed via drill and rongeurs (subtemporal craniectomy) to expose the middle cerebral artery (MCA). Care was taken not to remove the zygomatic arch or to transect the facial nerve that would impair the ability of the animal to chew after surgery. Using a dissecting microscope, the dura was incised, and the MCA was electrocoagulated from just proximal to the olfactory tract to the inferior cerebral vein (taking care not to rupture this vein), using microbipolar electrocauterization. The MCA was then transected. The temporalis muscle was then repositioned, and the incision was closed subcutaneously with sutures. The skin incision was closed with surgical staples (2-3 required). Throughout the procedure, body temperature was maintained at 37.0° ± 1° C, using a self-regulating heating pad connected to a rectal thermometer.

Post-Operative Monitoring: Following surgery, animals remained on a heating pad until they awakened from anesthesia. They were then returned to clean home cages. They were observed frequently on the day of MCAO surgery (Day 0) and at least once daily thereafter.

Handling, surgery, and injections timetable: The animals were housed 2-3 per cage before and after surgery, unless severe aggression was displayed, or death of cage mate(s). The animals were handled for 7 days before the surgery. Cefazolin Sodium i.p. (40 mg/kg) was administered right before surgery. Buprenorphine s.c. (0.1mg/kg) was administered right before surgery.

Dosing and Treatment: Rats were treated in accordance with the dosing schedule shown in Tables 2A, 2B, 3 and Figure 2, with each Phase being a two week period of time. A solution of 4-AP was used in this experiment. Dosing was started at 4 weeks after ischemic event. 4-aminopyridine was dissolved in water for injection (WFI, Cellgro) and sterile filtered. Final concentrations of 0.315 mg/mL or 1.0 mg/mL of 4-aminopyridine were delivered at 2 mL/kg by gastric gavage, resulting in final doses of 0.63 mg/kg and 2 mg/kg respectively. Vehicle control treatment was WFI delivered at 2 mL/kg by gastric gavage. This study was divided into three treatment phases (1-3) with each randomized cohort of animals receiving a different dose level during each of the treatment phases. Starting on day 30 after MCAO (Day 30, start of phase 1), the animals received gavage dosing of solutions (2mL/kg) approximately 12 hours apart, for a total of five doses. The same schedule was repeated with different treatment on Day 44 and Day 58 for phase 2 and 3 of the study, respectively. Animals were not treated during the 10 days between phases (washout period).

**Table 2A:**

| **Treatment ID** | **Treatment (doses TBD)** |
|---|---|
| V | Vehicle (water) |
| L | Low 4-AP (0.63 mg/kg, b.i.d, p.o) |
| H | High 4-AP (2.0 mg/kg, b.i.d, p.o) |

**Table 2B:**

| **Group (n=15)** | **Phase 1 treatment** | **Phase 2 treatment** | **Phase 3 treatment** |
|---|---|---|---|
| 1 | H | L | V |
| 2 | L | V | H |
| 3 | V | H | L |

Treatment groups: Dosing was started 4 weeks after MCAO surgery. During these 4 weeks, weekly behavioral assessments, as defined below, were performed. Two dose levels of 4-AP plus vehicle control were assessed, with treatment starting at 4 weeks after ischemic event. All dosing was via gastric gavage, volume not to exceed 2 mL/kg. Animals were given the first dose and behavioral assessments were performed starting 60 minutes after dosing. Animals then received the second dose that day at the appropriate time and b.i.d (preferably every 12 hours) thereafter for 2 more days (3 total days of dosing, 5 total doses). One hour after the 5^{th} dose, animals were subjected to behavioral assessments as defined below. Following the final behavioral assessment, drug was withdrawn for 10 or 11 days, behavior was re-assessed, and then animals were re-challenged with a cross-over treatment (Phase 2 of Table 2B) as described in Tables 2A, 2B and 3, followed by the same behavioral testing and dosing regimen. This cross-over was repeated one more time as well (Phase 3 of Table 2B) (see Figure 2).

Behavioral test details: Behavioral evaluations were done by evaluators blinded to treatment assignment. Blinded assessments of sensorimotor function were performed just prior to MCAO surgery, 24 hours after MCAO surgery and weekly thereafter until the first phase of dosing using limb placing and body swing behavioral tests. As described above, behavioral assessments were timed exactly with dosing times. Animals were tested one hour after the 1^{st} and 5^{th} doses of each phase (i.e., Days 30 and 32 of the first phase, Days 44 and 46 of the second phase; and Days 58 and 60 of the third phase); animals were also tested during the washout periods on Days 42 and 56. Animals were given the first dose, behavioral assessments were performed starting 60 minutes later, and blood was collected 90 minutes after dosing. Animals then received the second dose that day at the appropriate time and b.i.d (preferably every 12 hours) thereafter for 2 more days (3 total days of dosing, 5 total doses). One hour after the 5^{th} dose, animals were subjected to behavioral testing. Following the final behavioral assessment, drug was withdrawn for 10 or 11 days, behavior was re-assessed, and then animals were re-challenged with a cross-over treatment as described in Tables 2A, 2B, 3 and Figure 2, followed by the same behavioral testing and dosing regimen.

Limb Placing: Evaluated at Day -1 (pre-operation), Day 1, Day 7, Day 14, Day 21, Day 28, Day 30, Day 32, Day 42, Day 44, Day 46, Day 56, Day 58, Day 60. The limb placing tests were divided into forelimb and hindlimb tests. For the forelimb-placing test, the examiner held the rat close to a tabletop and scored the rat's ability to place the forelimb on the tabletop in response to whisker, visual, tactile, or proprioceptive stimulation. Similarly, for the hindlimb placing test, the examiner assessed the rat's ability to place the hindlimb on the tabletop in response to tactile and proprioceptive stimulation. Together, these tests reflect function and recovery in the sensorimotor systems (De Ryck et al., Brain Res 1992; 573:44-60). Separate sub -scores were obtained for each mode of sensory input and added to give total scores (for the forelimb placing test: 0 = normal, 12 = maximally impaired; for the hindlimb placing test: 0 = normal; 6 = maximally impaired). Scores were given in half-point increments (see below). Typically, there is a slow and steady recovery of limb placing behavior during the first month after stroke. Fore
limb placing test (0-12):
   whisker placing (0-2);
   visual placing (forward (0-2), sideways (0-2))
   tactile placing (dorsal (0-2), lateral (0-2))
   proprioceptive placing (0-2).
Hindlimb placing test (0-6):
   tactile placing (dorsal (0-2), lateral (0-2))
   proprioceptive placing (0-2).
For each subtest, animals are scored as followed:
   0.0 = immediate response
   0.5 = response within 2 seconds
   1.0 = response of 2-3 seconds
   1.5 = response of >3 seconds
   2.0 = no response

Body Swing: Evaluated at Day -1 (pre-operation), Day 1, Day 7, Day 14, Day 21, Day 28, Day 30, Day 32, Day 42, Day 44, Day 46, Day 56, Day 58, Day 60. Body swing was evaluated by counting head movements to one side or the other when suspended by tail. For this test, the rat was held approximately one (1) inch from the base of its tail. It was then elevated to an inch above a surface of a table. The rat was held in the vertical axis, defined as no more than 10° to either the left or the right side. A swing was recorded whenever the rat moved its head out of the vertical axis to either side. Before attempting another swing, the rat had to return to the vertical position for the next swing to be counted. Thirty (30) total swings were counted. The test reflects symmetry of striatal function (Borlongan et al., J. Neurosci 1995; 15:5372-8), and a normal rat typically has an equal number of swings to either side. Following focal ischemia, the rat tends to swing to the contralateral side (left side in this case). Body swing scores were expressed as a percentage of rightward over total swings. There is a spontaneous partial recovery of body swing scores (toward 50%) during the first month after stroke. The body swing test was performed at the same time as the limb placing test.

Cylinder Test: Evaluation was performed at Day -1 (pre-operation), Day 7, Day 21, Day 30, Day 32, Day 44, Day 46, Day 58, Day 60. This test assessed limb use asymmetry. Rats were placed in a transparent cylinder (20 cm in diameter and 30 cm in height) for 3-6 minutes. A mirror was placed behind the cylinder to determine forelimb movements when animal was turned away from the camera. The extent of fore limb-use asymmetry displayed by the animals was determined by counting the number of times the left or right forelimbs contact the wall during a full rear. Simultaneous use of both left and right forelimbs while contacting the wall during a full rear was also scored. A total number of 20 forelimb placings were counted during a trial. Data was expressed in terms of percent use of the non-impaired and or impaired forelimb relative to the total number of limb use observations for wall movements.

Blood sampling: After the on-drug behavior assessments were completed (Days 30, 32, 44, 46, 58 and 60), approximately 300 microliter blood sample was taken exactly 90 min after dosing to assess 4-AP plasma levels at that time. Blood was collected from the saphenous vein of each animal. Blood was collected into K3 EDTA tubes and centrifuged at 10,000 rpm for 10 minutes at 4 degrees C. Plasma was obtained, frozen and stored at approximately -80C. Samples were analyzed for 4-AP levels. 4-AP concentration was determined using a validated liquid chromatography with tandem mass spectrometric detection method in positive electrospray mode.

Euthanasia and post-mortem processing: Infarct Volume analysis. After the last behavioral assessment, rats were anesthetized deeply with ketamine/xylazine (100 mg/kg ketamine, 10 mg/kg xylazine, i.p.) and perfused transcardially with normal saline (with heparin 2 unit/ml) followed by 4% paraformaldehyde or formalin on Day 63. Brains were harvested and processed for histological assessment. A subset of 10 brains per group was processed for infarct volume measurement (H&E staining).

Infarct measurement: Brains were embedded in paraffin and 5 micron thick coronal sections were cut using a microtome. Sections were stained with hematoxylin and eosin (H&E), using standard methods. Seven coronal sections (+4.7, +2.7, +0.7, -1.3, -3.3, -5.3 and -7.3, compared to bregma respectively) from each brain were photographed by a digital camera, and the infarct area on each slice was determined by NIH Image (Image J) using the "indirect method" (area of the intact contralateral [left] hemisphere - area of intact regions of the ipsilateral [right] hemisphere) to correct for brain edema. Infarct areas were then summed among slices and multiplied by slice thickness (the distance between sections) to give total infarct volume, which was expressed as a percentage of intact contralateral hemispheric volume.

Regulatory Compliance: This study was conducted in a non-GLP environment, in an AAALAC accredited facility and in accordance with standard good scientific principles and practices.

Quality Assurance (QA): During the progress of the study, data collected was verified by a second scientist in the laboratory who was not involved in collection of that data set. This verification was documented within the raw data, and was maintained with the data package for this study. At the completion of the study, the entire data package (all raw data, measurements, notebooks and calculations) was verified and checked against the final report.

### Statistical Methods

Statistical Methods: Change from baseline behavior values were calculated for each treatment group in each phase. Baseline is defined as the behavioral value measured while animals receive no treatment prior to starting a dosing phase (Day 28, 42 and 56 for phase 1, 2 and 3, respectively). Mean behavioral parameter data for each entire dosing phase were subject to Analysis of Variance (ANOVA). Data were also subjected to mixed model analyses examining dose, sequence, carry-over effect and phase of the experiment as covariates using SAS Pair-wise comparisons between each pair of treatments using a difference in least squares from the mean method. Values of p<0.005 were considered statistically significant.

Within each phase, the baseline was defined as the measurement on the first day of that phase, which is Day 28, Day 42 and Day 56. Change from baseline was taken as the difference from the measurements on other days to the baseline. The average change from baseline for each subject was calculated by summing the two changes from baseline values within each phase (e.g., Day 30 and Day 32 in phase 1) and divided by two.

Descriptive statistics (mean and standard deviation) were presented in the excel sheets for different behavioral endpoints. Change from baseline was calculated at different phases per treatment group (N=15), regardless of which day within the phase the measurement was taken. Within a phase, one-way ANOVA with treatment as the only covariate was used to compare the means under different treatments. The null hypothesis was the means were all the same under different treatments. With a p-value <0.01 (for body swing) and <0.0001 (for hind and fore limb), the statistical significance was strongly demonstrated. The null hypotheses was rejected at the 99% confidence level and, thus, it was concluded that the three dose levels show the significantly different treatment effects on the studied muscle functions.

Two sets of mixed models have been used to further investigate other effects on the outcome. In the first set of mixed models, the outcome variable was the average change from the two post baseline measurements within a phase. Fixed effects included the covariates: "dose", "seq", "co" and "phase", "dose" referred to the three treatments, and "seq" referred to the treatment sequence assigned to each group (i.e., "high-low-vehicle"). "co" was the carry-over effect, defined as the dose from previous phase, in which the carry-over effect for phase 1 was set to 0. There was only one random effect, id, which was the subject id nested in sequence. In the second set of mixed models, the outcome variable was the raw change (without taking the average of the two post baseline measurements) from the baseline within a phase. Day as a fixed effect was added into the model so that fixed effects included the covariates: "dose", "seq", "co", "phase" and "day". "day" was the day when the measurement was taken, and it was nested within the phase. The rest of the fixed effects are the same as those in the previous mixed model. There was only one random effect, id, which was the subject id nested in sequence. In both mixed models, there were two parts of outputs from SAS presented. The first part is the "Type 3 Tests of Fixed Effects". With a statistical significant p-value (<=0.05), a conclusion could be made that the effect is significantly predicting the outcome. The second part was "Differences of Least Squares Means", in which the pair-wise comparison was conducted between each pair of treatments, i.e., high vs. low. p-value less than or equal to 0.05 claims a statistical significance in difference of the outcome under different treatments.

For forelimb function, the first model demonstrated that phase, dose and carry-over effect were significant effects but not sequence. High dose significantly improved the forelimb function compared to low (p=0.0334) and vehicle (p=0.001); while low didn't show statistical significant improvement compared to vehicle at level 0.05. In the second mixed model, day was shown as another significant effect and all three treatments were shown significant difference to each other (i.e. high vs. low, high vs. vehicle, and low vs. vehicle) with p<0.0001. Overall, for forelimb function, both models demonstrated that phase, dose and carryover effect were significant effects but not sequence. Both models demonstrated that high dose would significantly improve the forelimb function compared to lower dose and vehicle. The second mixed model appeared to be more sensitive to detect the treatment effects based on the smaller p-values.

The same analysis procedure was applied for each outcome measurements, including forelimb, hindlimb and body swing.

For hindlimb function, the first model demonstrated that phase and dose were significant effects but sequence or carry-over effects were not significant. High dose significantly improved the hindlimb function compared to vehicle (p<0.0001), and low dose significantly improved the hindlimb function compared to vehicle as well (p=0.0027), while high does didn't show the statistical significance at level 0.05, compare to low dose. In the second mixed model, all effects except sequence showed significant effect, and all three treatments were shown significant difference to each other (i.e. high vs. low, high vs. vehicle, and low vs. vehicle) with p<0.0001. Overall, for hindlimb function, both models demonstrated that phase and dose were significant effects. Both models also demonstrated that high dose and low dose would significantly improve the hindlimb function compared to vehicle. The second mixed model appeared to be more sensitive to detect the treatment effects based on the smaller p-values.

For body swing function, the first model demonstrated that only dose was significant effect. High dose significantly improved the body swing function compared to vehicle (p=0.0131), and low dose significantly improved the body swing function compared to vehicle as well (p=0.033), while high does didn't show the statistical significance at level 0.05, compare to low dose. In the second mixed model, phase was shown as another significant effect. High dose significantly improved the body swing function compared to low (p=0.006) and vehicle (p<0.0001); while low didn't show the statistical significant improvement compared to vehicle at level 0.05. Overall, for body swing function, both models have shown that dose was significant effects.

The mixed model is change from baseline = dose seq co phase day, with id nested in seq as random effect. Baseline is defined as Day 28, Day 46 and Day 56 for each phase respectively. Dose has three fixed levels, high, low and vehicle; seq(sequence) has three fixed values as three different dosing sequence, namely "hlv", "lvh" and "vhl"; co(carry-over effect) is defined as the dose from previous phase, in which the carry-over effect for phase 1 is set to 0. Id is the rat id from the data. * indicates statistically significant based on two sided test base on α=0.05.

### Results

Table 3 shows the distribution of animals between the treatment groups. Tables 4-6 show the Forelimb Placing Test Total Score for each of Groups 1-3. Tables 7-9 show Hindlimb Placing Test Total Score for each of Groups 1-3. Tables 10-12 show Body Swing Test Total Score for each of Groups 1-3. Tables 13-15 show the weight of animals at time points tested in each of Groups 1-3. Tables 16-18 show Cylinder Test Total Score (% of overall asymmetry) for each of Groups 1-3. Tables 19-21 show Cylinder Test Total Movement Score for each of Groups 1-3.

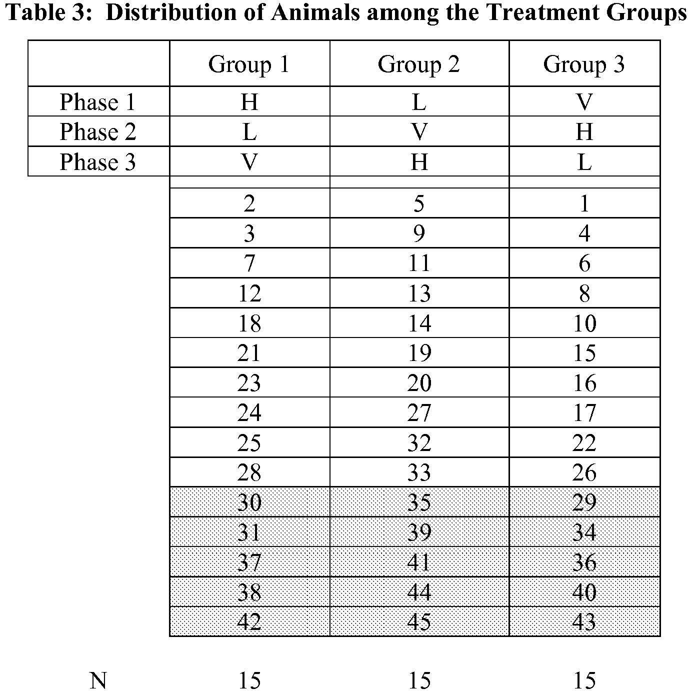

**Table 4: Forelimb Placing Total Score Group 1**

| **Group 1** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 2 | 0.00 | 12.00 | 9.00 | 8.50 | 7.00 | 6.50 | 4.50 | 4.00 | 6.00 | 4.00 | 3.00 | 3.50 | 4.50 | 6.00 |
| No. 3 | 0.00 | 11.50 | 9.00 | 7.50 | 6.50 | 6.00 | 4.50 | 3.00 | 5.00 | 3.00 | 2.50 | 2.50 | 2.50 | 2.50 |
| No. 7 | 0.00 | 11.00 | 7.00 | 6.00 | 6.00 | 6.00 | 3.50 | 3.50 | 4.50 | 0.00 | 0.00 | 2.00 | 3.00 | 3.00 |
| No. 12 | 0.00 | 11.50 | 6.00 | 4.50 | 4.50 | 4.00 | 1.50 | 0.50 | 1.50 | 0.50 | 0.00 | 1.50 | 1.50 | 1.50 |
| No. 18 | 0.00 | 12.00 | 7.00 | 6.50 | 5.50 | 4.50 | 3.00 | 2.00 | 4.00 | 1.00 | 1.00 | 2.50 | 2.00 | 2.00 |
| No. 21 | 0.00 | 12.00 | 9.50 | 7.00 | 6.50 | 6.00 | 4.50 | 3.00 | 5.00 | 3.50 | 3.50 | 4.00 | 4.00 | 3.00 |
| No. 23 | 0.00 | 11.50 | 6.00 | 5.00 | 5.00 | 5.00 | 3.50 | 2.00 | 2.50 | 1.50 | 1.50 | 1.50 | 1.50 | 1.50 |
| No. 24 | 0.00 | 11.50 | 7.50 | 7.00 | 6.50 | 6.00 | 4.00 | 3.50 | 4.50 | 4.00 | 3.00 | 3.00 | 3.50 | 3.50 |
| No. 25 | 0.00 | 11.50 | 7.00 | 6.00 | 6.00 | 6.00 | 5.50 | 2.00 | 6.00 | 4.00 | 1.50 | 5.50 | 5.50 | 5.50 |
| No. 28 | 0.00 | 11.50 | 8.00 | 7.00 | 6.00 | 6.00 | 4.50 | 3.00 | 5.00 | 3.00 | 3.50 | 5.00 | 5.50 | 4.00 |
| No. 30 | 0.00 | 12.00 | 7.50 | 6.50 | 6.00 | 6.00 | 3.00 | 3.00 | 5.50 | 4.00 | 4.00 | 4.50 | 4.00 | 4.00 |
| No. 31 | 0.00 | 11.50 | 7.00 | 6.50 | 6.00 | 6.00 | 3.50 | 2.50 | 5.00 | 2.00 | 2.50 | 3.50 | 3.50 | 3.00 |
| No. 37 | 0.00 | 12.00 | 6.50 | 6.00 | 6.00 | 6.00 | 3.50 | 3.50 | 6.00 | 3.50 | 3.00 | 5.00 | 5.50 | 5.50 |
| No. 38 | 0.00 | 10.50 | 7.00 | 5.00 | 3.50 | 3.50 | 0.50 | 0.50 | 2.00 | 1.50 | 1.50 | 1.50 | 1.50 | 1.50 |
| No. 42 | 0.00 | 12.00 | 7.00 | 6.50 | 4.00 | 3.50 | 1.00 | 0.50 | 2.00 | 0.00 | 0.00 | 0.00 | 0.00 | 0.00 |
| | | | | | | | | | | | | | | |
| N | 15 | 15 | 15 | 15 | 15 | 15 | 15 | 15 | 15 | 15 | 15 | 15 | 15 | 15 |
| Mean | 0.00 | 11.60 | 7.40 | 6.37 | 5.67 | 5.40 | 3.37 | 2.43 | 4.30 | 2.37 | 2.03 | 3.03 | 3.20 | 3.10 |
| Sem | 0.00 | 0.11 | 0.27 | 0.26 | 0.26 | 0.26 | 0.36 | 0.30 | 0.40 | 0.39 | 0.35 | 0.41 | 0.44 | 0.44 |
| SD | 0.00 | 0.43 | 1.06 | 1.03 | 0.99 | 1.02 | 1.41 | 1.16 | 1.56 | 1.52 | 1.36 | 1.59 | 1.69 | 1.70 |

**Table 5: Forelimb Placing Total Score Group 2**

| **Group 2** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 5 | 0.00 | 11.50 | 9.50 | 6.50 | 6.00 | 5.50 | 4.00 | 3.00 | 3.50 | 3.50 | 3.00 | 2.50 | 0.50 | 0.50 |
| No. 9 | 0.00 | 11.00 | 8.50 | 7.00 | 6.50 | 6.00 | 5.00 | 4.50 | 6.00 | 6.00 | 5.00 | 5.50 | 3.00 | 2.50 |
| No. 11 | 0.00 | 12.00 | 7.00 | 6.00 | 5.50 | 5.50 | 3.00 | 3.00 | 4.50 | 4.00 | 3.50 | 3.50 | 1.00 | 0.00 |
| No. 13 | 0.00 | 11.50 | 8.50 | 7.00 | 6.00 | 6.00 | 4.50 | 4.00 | 5.00 | 4.50 | 4.00 | 4.00 | 2.50 | 2.50 |
| No. 14 | 0.00 | 12.00 | 9.00 | 6.50 | 7.00 | 6.00 | 6.00 | 5.50 | 6.00 | 6.00 | 6.00 | 5.50 | 3.50 | 3.00 |
| No. 19 | 0.00 | 12.00 | 7.00 | 6.00 | 6.00 | 6.00 | 5.50 | 5.50 | 6.00 | 5.00 | 5.00 | 5.00 | 2.50 | 1.00 |
| No. 20 | 0.00 | 12.00 | 9.00 | 6.00 | 4.00 | 4.00 | 4.00 | 2.00 | 2.00 | 1.50 | 1.00 | 0.50 | 0.00 | 0.00 |
| No. 27 | 0.00 | 12.00 | 7.50 | 6.00 | 6.00 | 6.00 | 3.50 | 2.00 | 5.00 | 4.50 | 4.50 | 4.50 | 1.50 | 1.00 |
| No. 32 | 0.00 | 12.00 | 8:00 | 6.50 | 6.50 | 6.50 | 5.50 | 4.00 | 5.00 | 4.50 | 3.50 | 5.00 | 2.50 | 1.50 |
| No. 33 | 0.00 | 12.00 | 8.50 | 6.50 | 6.00 | 6.00 | 5.00 | 5.00 | 6.00 | 6.00 | 5.50 | 5.00 | 3.00 | 0.00 |
| No. 35 | 0.00 | 12.00 | 7.50 | 6.50 | 6.00 | 6.00 | 4.00 | 3.50 | 3.00 | 3.00 | 3.00 | 3.00 | 1.50 | 1.00 |
| No. 39 | 0.00 | 11.00 | 7.00 | 6.00 | 6.00 | 6.00 | 4.50 | 4.50 | 4.50 | 5.00 | 5.00 | 5.00 | 5.00 | 3.50 |
| No. 41 | 0.00 | 11.00 | 6.00 | 4.50 | 4.50 | 4.00 | 1.50 | 1.00 | 2.50 | 3.00 | 3.00 | 2.50 | 0.00 | 0.00 |
| No. 44 | 0.00 | 11.00 | 6.50 | 4.50 | 4.50 | 3.00 | 1.50 | 1.00 | 1.00 | 1.00 | 1.00 | 1.00 | 0.00 | 0.00 |
| No. 45 | 0.00 | 11.00 | 6.00 | 6.00 | 6.00 | 6.00 | 5.50 | 3.00 | 6.00 | 6.00 | 6.00 | 6.00 | 2.00 | 1.00 |
| | | | | | | | | | | | | | | |
| N | 15 | 15 | 15 | 15 | 15 | 15 | 15 | 15 | 15 | 15 | 15 | 15 | 15 | 15 |
| Mean | 0.00 | 11.60 | 7.70 | 6.10 | 5.77 | 5.50 | 4.20 | 3.43 | 4.40 | 4.23 | 3.93 | 3.90 | 1.90 | 1.17 |
| Sem | 0.00 | 0.12 | 0.29 | 0.19 | 0.21 | 0.26 | 0.35 | 0.38 | 0.42 | 0.41 | 0.41 | 0.43 | 0.38 | 0.31 |
| SD | 0.00 | 0.47 | 1.11 | 0.74 | 0.82 | 1.00 | 1.37 | 1.47 | 1.64 | 1.59 | 1.58 | 1.68 | 1.45 | 1.19 |

**Table 6: Forelimb Placing Total Score Group 3**

| **Group 3** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 1 | 0.00 | 12.00 | 9.00 | 7.00 | 6.50 | 6.00 | 6.00 | 6.00 | 6.00 | 5.00 | 1.00 | 4.50 | 3.00 | 3.00 |
| No. 4 | 0.00 | 12.00 | 7.00 | 6.00 | 6.00 | 6.00 | 4.00 | 4.00 | 4.00 | 1.50 | 1.00 | 3.00 | 1.50 | 1.00 |
| No. 6 | 0.00 | 11.50 | 8.00 | 5.00 | 5.00 | 5.00 | 5.00 | 5.00 | 4.50 | 3.00 | 1.50 | 3.50 | 3.00 | 3.00 |
| No. 8 | 0.00 | 11.00 | 6.00 | 4.50 | 4.50 | 4.50 | 3.00 | 3.00 | 1.50 | 0.00 | 0.00 | 0.00 | 0.00 | 0.00 |
| No. 10 | 0.00 | 11.50 | 8.00 | 6.00 | 6.00 | 6.00 | 6.00 | 6.00 | 5.50 | 4.50 | 2.50 | 4.50 | 3.00 | 2.50 |
| No. 15 | 0.00 | 12.00 | 8.00 | 7.00 | 6.00 | 6.00 | 6.00 | 6.00 | 5.50 | 4.00 | 3.00 | 4.00 | 3.00 | 3.00 |
| No. 16 | 0.00 | 12.00 | 7.00 | 6.00 | 5.50 | 5.00 | 3.50 | 3.00 | 2.00 | 1.00 | 0.00 | 0.50 | 0.50 | 1.00 |
| No. 17 | 0.00 | 11.50 | 8.50 | 7.00 | 6.50 | 6.00 | 5.50 | 6.00 | 5.50 | 4.00 | 2.00 | 3.00 | 2.00 | 2.00 |
| No. 22 | 0.00 | 12.00 | 9.00 | 7.00 | 6.50 | 6.50 | 6.50 | 6.50 | 6.00 | 4.00 | 3.00 | 6.00 | 3.50 | 3.50 |
| No. 26 | 0.00 | 12.00 | 9.00 | 6.00 | 5.00 | 5.50 | 5.00 | 4.00 | 3.00 | 2.50 | 1.50 | 3.00 | 1.50 | 0.50 |
| No. 29 | 0.00 | 12.00 | 8.50 | 7.00 | 6.50 | 6.50 | 6.00 | 6.00 | 6.00 | 3.00 | 0.00 | 6.00 | 2.50 | 2.00 |
| No. 34 | 0.00 | 12.00 | 8.50 | 6.50 | 6.00 | 6.00 | 6.00 | 6.00 | 6.00 | 4.50 | 1.50 | 6.00 | 4.00 | 3.00 |
| No. 36 | 0.00 | 12.00 | 7.50 | 6.50 | 5.00 | 5.50 | 4.50 | 4.50 | 6.00 | 3.00 | 3.00 | 4.00 | 2.50 | 2.50 |
| No. 40 | 0.00 | 11.00 | 6.50 | 6.00 | 5.00 | 4.00 | 3.00 | 1.50 | 2.00 | 0.00 | 0.00 | 0.00 | 0.00 | 0.00 |
| No. 43 | 0.00 | 11.50 | 6.00 | 5.00 | 4.00 | 3.00 | 1.50 | 1.50 | 1.00 | 0.00 | 0.00 | 0.00 | 0.00 | 0.00 |
| | | | | | | | | | | | | | | |
| N | 15 | 15 | 15 | 15 | 15 | 15 | 15 | 15 | 15 | 15 | 15 | 15 | 15 | 15 |
| Mean | 0.00 | 11.73 | 7.77 | 6.17 | 5.60 | 5.43 | 4.77 | 4.60 | 4.30 | 2.67 | 1.33 | 3.20 | 2.00 | 1.80 |
| Sem | 0.00 | 0.10 | 0.27 | 0.21 | 0.21 | 0.25 | 0.38 | 0.44 | 0.49 | 0.45 | 0.30 | 0.56 | 0.35 | 0.33 |
| SD | 0.00 | 0.37 | 1.05 | 0.82 | 0.81 | 0.98 | 1.47 | 1.70 | 1.89 | 1.76 | 1.18 | 2.18 | 1.35 | 1.26 |

**Table 7: Hindlimb Placing Total Score Group 1**

| **Group 1** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 2 | 0.00 | 6.00 | 3.50 | 3.00 | 3.00 | 3.00 | 1.50 | 1.50 | 2.50 | 2.50 | 2.00 | 2.50 | 2.50 | 2.50 |
| No. 3 | 0.00 | 6.00 | 3.50 | 3.00 | 3.00 | 2.50 | 1.50 | 0.00 | 0.00 | 0.00 | 0.00 | 0.00 | 0.00 | 0.00 |
| No. 7 | 0.00 | 6.00 | 3.50 | 3.50 | 3.00 | 2.50 | 1.50 | 1.50 | 2.50 | 0.00 | 0.00 | 1.50 | 1.50 | 1.50 |
| No. 12 | 0.00 | 5.00 | 3.00 | 3.00 | 2.50 | 2.00 | 0.50 | 0.00 | 1.50 | 0.50 | 0.50 | 0.50 | 1.00 | 1.00 |
| No. 18 | 0.00 | 6.00 | 4.00 | 3.50 | 3.00 | 3.00 | 1.50 | 0.50 | 2.50 | 1.00 | 1.00 | 1.50 | 1.50 | 1.50 |
| No. 21 | 0.00 | 6.00 | 4.50 | 3.00 | 3.00 | 3.00 | 1.50 | 1.00 | 2.50 | 2.00 | 2.00 | 2.00 | 2.00 | 2.00 |
| No. 23 | 0.00 | 6.00 | 4.50 | 3.50 | 3.50 | 3.00 | 3.00 | 3.00 | 3.00 | 3.00 | 3.00 | 2.00 | 3.00 | 2.00 |
| No. 24 | 0.00 | 6.00 | 3.50 | 3.00 | 3.00 | 3.00 | 1.50 | 1.50 | 3.00 | 2.00 | 1.50 | 1.50 | 2.00 | 2.00 |
| No. 25 | 0.00 | 6.00 | 4.00 | 3.00 | 3.00 | 3.00 | 2.50 | 1.50 | 3.00 | 2.00 | 0.00 | 2.00 | 2.50 | 2.50 |
| No. 28 | 0.00 | 6.00 | 4.00 | 3.50 | 3.00 | 3.00 | 3.00 | 1.50 | 2.50 | 1.50 | 1.50 | 2.50 | 3.00 | 2.50 |
| No. 30 | 0.00 | 6.00 | 3.50 | 3.00 | 3.00 | 3.00 | 1.50 | 0.00 | 2.50 | 2.00 | 2.00 | 3.00 | 3.00 | 3.00 |
| No. 31 | 0.00 | 6.00 | 4.00 | 3.00 | 3.00 | 3.00 | 2.50 | 0.50 | 3.00 | 1.50 | 1.50 | 1.50 | 1.50 | 1.50 |
| No. 37 | 0.00 | 6.00 | 4.00 | 3.00 | 3.00 | 3.00 | 2.00 | 1.50 | 2.50 | 1.50 | 1.50 | 2.50 | 2.50 | 2.50 |
| No. 38 | 0.00 | 5.50 | 3.50 | 3.00 | 3.00 | 3.00 | 1.00 | 0.50 | 1.50 | 0.50 | 0.50 | 0.50 | 0.50 | 0.50 |
| No. 42 | 0.00 | 6.00 | 3.00 | 3.00 | 3.00 | 2.50 | 0.00 | 0.00 | 1.50 | 0.00 | 0.00 | 0.00 | 0.00 | 0.00 |
| | | | | | | | | | | | | | | |
| N | 15 | 15 | 15 | 15 | 15 | 15 | 15 | 15 | 15 | 15 | 15 | 15 | 15 | 15 |
| Mean | 0.00 | 5.90 | 3.73 | 3.13 | 3.00 | 2.83 | 1.67 | 0.97 | 2.27 | 1.33 | 1.13 | 1.57 | 1.77 | 1.67 |
| Sem | 0.00 | 0.07 | 0.12 | 0.06 | 0.05 | 0.08 | 0.22 | 0.22 | 0.21 | 0.25 | 0.24 | 0.24 | 0.27 | 0.24 |
| SD | 0.00 | 0.28 | 0.46 | 0.23 | 0.19 | 0.31 | 0.84 | 0.85 | 0.82 | 0.96 | 0.93 | 0.94 | 1.03 | 0.94 |

**Table 8: Hindlimb Placing Total Score Group 2**

| **Group 2** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 5 | 0.00 | 6.00 | 3.50 | 3.00 | 3.00 | 3.00 | 2.00 | 1.00 | 2.50 | 2.00 | 2.00 | 2.00 | 0.50 | 0.50 |
| No. 9 | 0.00 | 6.00 | 3.50 | 3.00 | 3.00 | 3.00 | 1.50 | 1.50 | 2.50 | 3.00 | 3.00 | 3.00 | 1.50 | 0.00 |
| No. 11 | 0.00 | 6.00 | 3.50 | 2.50 | 2.50 | 2.00 | 1.50 | 1.50 | 1.50 | 1.50 | 2.00 | 1.50 | 0.00 | 0.00 |
| No. 13 | 0.00 | 6.00 | 4.00 | 3.00 | 3.00 | 3.00 | 2.00 | 2.00 | 2.50 | 2.50 | 2.50 | 2.50 | 2.00 | 0.50 |
| No. 14 | 0.00 | 5.50 | 3.00 | 3.00 | 3.00 | 3.00 | 3.00 | 1.50 | 2.00 | 2.00 | 2.00 | 2.00 | 0.50 | 0.00 |
| No. 19 | 0.00 | 6.00 | 3.50 | 3.00 | 3.00 | 3.00 | 2.50 | 2.00 | 3.00 | 2.50 | 2.50 | 2.50 | 2.00 | 0.50 |
| No. 20 | 0.00 | 6.00 | 4.50 | 3.00 | 3.00 | 3.00 | 1.50 | 1.00 | 2.50 | 1.50 | 0.50 | 0.50 | 0.00 | 0.00 |
| No. 27 | 0.00 | 6.00 | 3.00 | 3.00 | 2.50 | 2.50 | 2.00 | 1.50 | 2.00 | 2.00 | 2.00 | 2.00 | 1.00 | 1.00 |
| No. 32 | 0.00 | 6.00 | 4.00 | 3.00 | 3.00 | 2.50 | 2.00 | 1.50 | 2.00 | 2.00 | 2.00 | 2.00 | 1.50 | 0.50 |
| No. 33 | 0.00 | 6.00 | 4.50 | 3.50 | 3.00 | 3.00 | 2.00 | 1.50 | 3.00 | 3.00 | 3.00 | 3.00 | 1.50 | 0.00 |
| No. 35 | 0.00 | 6.00 | 3.50 | 3.00 | 2.50 | 2.50 | 1.50 | 1.50 | 1.50 | 1.50 | 1.50 | 0.50 | 0.00 | 0.00 |
| No. 39 | 0.00 | 5.50 | 3.00 | 3.00 | 3.00 | 3.00 | 1.50 | 1.00 | 1.50 | 2.00 | 1.50 | 1.50 | 1.50 | 0.50 |
| No. 41 | 0.00 | 5.00 | 3.00 | 2.50 | 2.50 | 2.00 | 1.00 | 1.00 | 1.50 | 1.50 | 1.50 | 1.00 | 0.00 | 0.00 |
| No. 44 | 0.00 | 6.00 | 3.50 | 3.00 | 3.00 | 1.50 | 0.50 | 0.50 | 1.00 | 1.00 | 1.00 | 1.00 | 0.00 | 0.00 |
| No. 45 | 0.00 | 6.00 | 3.50 | 3.00 | 3.00 | 3.00 | 1.50 | 1.50 | 2.00 | 2.00 | 2.00 | 2.00 | 0.50 | 0.00 |
| | | | | | | | | | | | | | | |
| N | 15 | 15 | 15 | 15 | 15 | 15 | 15 | 15 | 15 | 15 | 15 | 15 | 15 | 15 |
| Mean | 0.00 | 5.87 | 3.57 | 2.97 | 2.87 | 2.67 | 1.73 | 1.37 | 2.07 | 2.00 | 1.93 | 1.80 | 0.83 | 0.23 |
| Sem | 0.00 | 0.08 | 0.13 | 0.06 | 0.06 | 0.13 | 0.15 | 0.10 | 0.15 | 0.15 | 0.18 | 0.21 | 0.20 | 0.08 |
| SD | 0.00 | 0.30 | 0.50 | 0.23 | 0.23 | 0.49 | 0.59 | 0.40 | 0.59 | 0.57 | 0.68 | 0.80 | 0.77 | 0.32 |

**Table 9: Hindlimb Placing Total Score Group 3**

| **Group 3** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 1 | 0.00 | 6.00 | 4.00 | 4.00 | 3.50 | 3.00 | 3.00 | 3.00 | 3.00 | 3.00 | 1.50 | 3.00 | 1.50 | 1.50 |
| No. 4 | 0.00 | 6.00 | 3.50 | 3.00 | 3.00 | 3.00 | 2.50 | 2.00 | 2.00 | 0.00 | 0.00 | 1.50 | 0.00 | 0.00 |
| No. 6 | 0.00 | 6.00 | 3.50 | 3.00 | 3.00 | 3.00 | 3.00 | 3.00 | 2.50 | 0.50 | 0.00 | 0.50 | 0.50 | 0.50 |
| No. 8 | 0.00 | 5.50 | 4.00 | 3.50 | 2.50 | 2.50 | 2.50 | 1.50 | 2.00 | 0.00 | 0.00 | 0.00 | 0.00 | 0.00 |
| No. 10 | 0.00 | 5.50 | 3.00 | 3.00 | 3.00 | 3.00 | 3.00 | 3.00 | 2.50 | 1.50 | 0.50 | 2.00 | 1.00 | 1.00 |
| No. 15 | 0.00 | 6.00 | 3.50 | 3.50 | 3.00 | 3.00 | 3.00 | 3.00 | 3.00 | 2.00 | 1.00 | 2.00 | 1.50 | 1.50 |
| No. 16 | 0.00 | 6.00 | 3.00 | 3.00 | 3.00 | 3.00 | 2.50 | 2.00 | 1.50 | 0.00 | 0.00 | 1.00 | 0.50 | 1.00 |
| No. 17 | 0.00 | 5.50 | 3.50 | 3.00 | 3.00 | 3.00 | 3.00 | 3.00 | 3.00 | 2.00 | 0.50 | 1.50 | 1.00 | 1.00 |
| No. 22 | 0.00 | 6.00 | 4.50 | 3.00 | 3.00 | 3.00 | 3.00 | 3.00 | 3.00 | 1.50 | 0.50 | 1.50 | 0.50 | 0.50 |
| No. 26 | 0.00 | 6.00 | 3.50 | 3.00 | 2.50 | 3.00 | 2.50 | 2.00 | 1.50 | 1.00 | 0.50 | 1.50 | 1.00 | 0.50 |
| No. 29 | 0.00 | 6.00 | 4.50 | 3.50 | 3.00 | 3.00 | 3.00 | 3.00 | 3.00 | 1.00 | 0.00 | 3.00 | 3.00 | 2.00 |
| No. 34 | 0.00 | 6.00 | 3.50 | 3.00 | 3.00 | 3.00 | 2.00 | 2.00 | 2.50 | 0.50 | 0.00 | 2.50 | 1.00 | 1.00 |
| No. 36 | 0.00 | 6.00 | 3.00 | 2.50 | 2.50 | 2.50 | 1.50 | 1.50 | 2.00 | 0.50 | 0.00 | 1.50 | 0.50 | 0.00 |
| No. 40 | 0.00 | 5.50 | 4.00 | 3.00 | 2.50 | 2.50 | 2.00 | 1.50 | 1.50 | 0.00 | 0.00 | 0.00 | 0.00 | 0.00 |
| No. 43 | 0.00 | 5.50 | 3.50 | 3.00 | 3.00 | 3.00 | 2.00 | 1.50 | 1.00 | 0.00 | 0.00 | 1.00 | 0.00 | 0.00 |
| | | | | | | | | | | | | | | |
| N | 15 | IS | 15 | 15 | 15 | 15 | 15 | 15 | 15 | 15 | 15 | 15 | 15 | 15 |
| Mean | 0.00 | 5.83 | 3.63 | 3.13 | 2.90 | 2.90 | 2.57 | 2.33 | 2.27 | 0.90 | 0.30 | 1.50 | 0.80 | 0.70 |
| Sem | 0.00 | 0.06 | 0.12 | 0.09 | 0.07 | 0.05 | 0.13 | 0.17 | 0.18 | 0.24 | 0.12 | 0.24 | 0.21 | 0.17 |
| SD | 0.00 | 0.24 | 0.48 | 0.35 | 0.28 | 0.21 | 0.50 | 0.67 | 0.68 | 0.93 | 0.46 | 0.93 | 0.80 | 0.65 |

**Table 10: Body Swing Test (% right Swing) Group 1**

| **Group 1** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 2 | 50.00 | 0.00 | 6.67 | 13.33 | 23.33 | 36.67 | 33.33 | 33.33 | 40.00 | 46.67 | 53.33 | 50.00 | 46.67 | 43.33 |
| No. 3 | 46.67 | 0.00 | 3.33 | 6.67 | 10.00 | 33.33 | 26.67 | 30.00 | 23.33 | 30.00 | 20.00 | 16.67 | 26.67 | 40.00 |
| No. 7 | 50.00 | 0.00 | 10.00 | 16.67 | 10.00 | 13.33 | 36.67 | 40.00 | 16.67 | 40.00 | 20.00 | 26.67 | 13.33 | 20.00 |
| No. 12 | 50.00 | 6.67 | 33.33 | 30.00 | 26.67 | 36.67 | 36.67 | 33.33 | 26.67 | 30.00 | 33.33 | 36.67 | 26.67 | 36.67 |
| No. 18 | 50.00 | 0.00 | 0.00 | 23.33 | 26.67 | 33.33 | 46.67 | 50.00 | 30.00 | 43.33 | 40.00 | 43.33 | 30.00 | 26.67 |
| No. 21 | 53.33 | 0.00 | 0.00 | 6.67 | 13.33 | 20.00 | 33.33 | 43.33 | 26.67 | 33.33 | 36.67 | 33.33 | 30.00 | 26.67 |
| No. 23 | 50.00 | 0.00 | 20.00 | 46.67 | 46.67 | 46.67 | 53.33 | 50.00 | 46.67 | 56.67 | 43.33 | 43.33 | 40.00 | 43.33 |
| No. 24 | 50.00 | 0.00 | 0.00 | 6.67 | 23.33 | 16.67 | 33.33 | 53.33 | 40.00 | 43.33 | 43.33 | 40.00 | 36.67 | 36.67 |
| No. 25 | 46.67 | 0.00 | 6.67 | 26.67 | 10.00 | 16.67 | 40.00 | 53.33 | 33.33 | 26.67 | 33.33 | 23.33 | 30.00 | 33.33 |
| No. 28 | 50.00 | 0.00 | 6.67 | 3.33 | 6.67 | 33.33 | 30.00 | 26.67 | 33.33 | 33.33 | 36.67 | 40.00 | 33.33 | 33.33 |
| No. 30 | 50.00 | 0.00 | 0.00 | 13.33 | 30.00 | 23.33 | 36.67 | 43.33 | 33.33 | 33.33 | 36.67 | 30.00 | 30.00 | 30.00 |
| No. 31 | 46.67 | 0.00 | 0.00 | 10.00 | 6.67 | 16.67 | 36.67 | 33.33 | 33.33 | 26.67 | 30.00 | 23.33 | 33.33 | 43.33 |
| No. 37 | 50.00 | 0.00 | 0.00 | 26.67 | 20.00 | 13.33 | 43.33 | 53.33 | 50.00 | 50.00 | 43.33 | 33.33 | 33.33 | 33.33 |
| No. 38 | 46.67 | 0.00 | 0.00 | 10.00 | 6.67 | 33.33 | 40.00 | 50.00 | 50.00 | 43.33 | 43.33 | 40.00 | 40.00 | 36.67 |
| No. 42 | 50.00 | 0.00 | 0.00 | 3.33 | 3.33 | 13.33 | 36.67 | 40.00 | 43.33 | 40.00 | 33.33 | 33.33 | 36.67 | 30.00 |
| | | | | | | | | | | | | | | |
| N | 15 | 15 | 15 | 15 | 15 | 15 | 15 | 15 | 15 | 15 | 15 | 15 | 15 | 15 |
| Mean | 49.33 | 0.44 | 5.78 | 16.22 | 17.56 | 25.78 | 37.56 | 42.22 | 35.11 | 38.44 | 36.44 | 34.22 | 32.44 | 34.22 |
| Sem | 0.48 | 0.44 | 2.44 | 3.15 | 3.08 | 2.81 | 1.70 | 2.38 | 2.54 | 2.30 | 2.29 | 2.33 | 1.96 | 1.77 |
| SD | 1.87 | 1.72 | 9.47 | 12.21 | 11.92 | 10.87 | 6.60 | 9.23 | 9.83 | 8.90 | 8.86 | 9.04 | 7.61 | 6.84 |

**Table 11: Body Swing Test (% right Swing) Group 2**

| **Group 2** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 5 | 46.67 | 0.00 | 3.33 | 3.33 | 0.00 | 33.33 | 30.00 | 43.33 | 26.67 | 30.00 | 30.00 | 33.33 | 36.67 | 40.00 |
| No. 9 | 50.00 | 0.00 | 6.67 | 13.33 | 20.00 | 30.00 | 43.33 | 46.67 | 33.33 | 33.33 | 36.67 | 40.00 | 50.00 | 40.00 |
| No. 11 | 46.67 | 0.00 | 36.67 | 36.67 | 36.67 | 33.33 | 53.33 | 43.33 | 36.67 | 40.00 | 43.33 | 43.33 | 46.67 | 50.00 |
| No. 13 | 50.00 | 3.33 | 0.00 | 20.00 | 16.67 | 26.67 | 26.67 | 33.33 | 10.00 | 23.33 | 30.00 | 26.67 | 33.33 | 40.00 |
| No. 14 | 46.67 | 0.00 | 0.00 | 23.33 | 26.67 | 30.00 | 26.67 | 30.00 | 20.00 | 26.67 | 33.33 | 33.33 | 43.33 | 50.00 |
| No. 19 | 50.00 | 0.00 | 6.67 | 16.67 | 26.67 | 23.33 | 20.00 | 33.33 | 33.33 | 33.33 | 33.33 | 36.67 | 46.67 | 43.33 |
| No. 20 | 46.67 | 0.00 | 16.67 | 26.67 | 26.67 | 13.33 | 40.00 | 33.33 | 36.67 | 33.33 | 30.00 | 30.00 | 33.33 | 40.00 |
| No. 27 | 53.33 | 0.00 | 0.00 | 16.67 | 16.67 | 16.67 | 16.67 | 33.33 | 26.67 | 16.67 | 33.33 | 23.33 | 26.67 | 33.33 |
| No. 32 | 53.33 | 0.00 | 3.33 | 6.67 | 10.00 | 16.67 | 30.00 | 33.33 | 33.33 | 33.33 | 30.00 | 26.67 | 40.00 | 46.67 |
| No. 33 | 50.00 | 0.00 | 0.00 | 16.67 | 16.67 | 23.33 | 36.67 | 33.33 | 30.00 | 20.00 | 20.00 | 33.33 | 33.33 | 36.67 |
| No. 35 | 46.67 | 0.00 | 0.00 | 26.67 | 23.33 | 13.33 | 33.33 | 40.00 | 43.33 | 26.67 | 26.67 | 43.33 | 43.33 | 50.00 |
| No. 39 | 50.00 | 0.00 | 23.33 | 23.33 | 26.67 | 30.00 | 23.33 | 43.33 | 46.67 | 43.33 | 43.33 | 43.33 | 46.67 | 50.00 |
| No. 41 | 50.00 | 6.67 | 6.67 | 13.33 | 16.67 | 13.33 | 33.33 | 30.00 | 33.33 | 26.67 | 33.33 | 26.67 | 46.67 | 40.00 |
| No. 44 | 50.00 | 0.00 | 13.33 | 6.67 | 20.00 | 30.00 | 53.33 | 46.67 | 46.67 | 50.00 | 46.67 | 50.00 | 50.00 | 46.67 |
| No. 45 | 50.00 | 0.00 | 0.00 | 0.00 | 3.33 | 26.67 | 26.67 | 43.33 | 46.67 | 30.00 | 26.67 | 23.33 | 33.33 | 33.33 |
| | | | | | | | | | | | | | | |
| N | 15 | 15 | 15 | 15 | 15 | 15 | 15 | 15 | 15 | 15 | 15 | 15 | 15 | 15 |
| Mean | 49.33 | 0.67 | 7.78 | 16.67 | 19.11 | 24.00 | 32.89 | 37.78 | 33.56 | 31.11 | 33.11 | 34.22 | 40.67 | 42.67 |
| Sem | 0.58 | 0.48 | 2.75 | 2.56 | 2.47 | 1.93 | 2.81 | 1.58 | 2.65 | 2.25 | 1.82 | 2.14 | 1.90 | 1.53 |
| SD | 2.25 | 1.87 | 10.67 | 9.92 | 9.55 | 7.47 | 10.90 | 6.13 | 10.27 | 8.70 | 7.07 | 8.31 | 7.37 | 5.94 |

**Table 12: Body Swing Test (% right Swing) Group 3**

| **Group 3** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 1 | 50.00 | 0.00 | 20.00 | 23.33 | 30.00 | 23.33 | 30.00 | 33.33 | 33.33 | 43.33 | 43.33 | 33.33 | 30.00 | 26.67 |
| No. 4 | 50.00 | 3.33 | 30.00 | 23.33 | 16.67 | 16.67 | 13.33 | 16.67 | 26.67 | 40.00 | 20.00 | 30.00 | 20.00 | 16.67 |
| No. 6 | 46.67 | 0.00 | 6.67 | 10.00 | 3.33 | 10.00 | 13.33 | 10.00 | 6.67 | 23.33 | 23.33 | 26.67 | 20.00 | 23.33 |
| No. 8 | 50.00 | 6.67 | 30.00 | 20.00 | 43.33 | 33.33 | 20.00 | 43.33 | 40.00 | 46.67 | 40.00 | 46.67 | 46.67 | 50.00 |
| No. 10 | 53.33 | 0.00 | 0.00 | 23.33 | 20.00 | 30.00 | 26.67 | 30.00 | 26.67 | 33.33 | 30.00 | 33.33 | 33.33 | 36.67 |
| No. 15 | 50.00 | 0.00 | 6.67 | 33.33 | 33.33 | 36.67 | 30.00 | 36.67 | 36.67 | 40.00 | 40.00 | 36.67 | 30.00 | 30.00 |
| No. 16 | 46.67 | 0.00 | 30.00 | 30.00 | 36.67 | 40.00 | 33.33 | 20.00 | 26.67 | 43.33 | 43.33 | 43.33 | 46.67 | 43.33 |
| No. 17 | 50.00 | 6.67 | 16.67 | 43.33 | 36.67 | 36.67 | 36.67 | 40.00 | 36.67 | 46.67 | 40.00 | 43.33 | 40.00 | 36.67 |
| No. 22 | 53.33 | 0.00 | 0.00 | 10.00 | 26.67 | 13.33 | 16.67 | 33.33 | 30.00 | 53.33 | 50.00 | 43.33 | 40.00 | 36.67 |
| No. 26 | 53.33 | 0.00 | 6.67 | 33.33 | 33.33 | 36.67 | 30.00 | 26.67 | 33.33 | 33.33 | 30.00 | 26.67 | 30.00 | 33.33 |
| No. 29 | 50.00 | 0.00 | 6.67 | 6.67 | 10.00 | 16.67 | 26.67 | 16.67 | 20.00 | 13.33 | 53.33 | 10.00 | 20.00 | 33.33 |
| No. 34 | 50.00 | 0.00 | 0.00 | 30.00 | 23.33 | 16.67 | 33.33 | 33.33 | 36.67 | 36.67 | 43.33 | 33.33 | 33.33 | 36.67 |
| No. 36 | 50.00 | 3.33 | 20.00 | 36.67 | 36.67 | 40.00 | 40.00 | 46.67 | 46.67 | 50.00 | 53.33 | 46.67 | 53.33 | 50.00 |
| No. 40 | 46.67 | 10.00 | 13.33 | 20.00 | 33.33 | 46.67 | 53.33 | 50.00 | 46.67 | 50.00 | 53.33 | 46.67 | 46.67 | 43.33 |
| No. 43 | 46.67 | 0.00 | 6.67 | 10.00 | 10.00 | 16.67 | 13.33 | 40.00 | 40.00 | 40.00 | 43.33 | 33.33 | 33.33 | 30.00 |
| | | | | | | | | | | | | | | |
| N | 15 | 15 | 15 | 15 | 15 | 15 | 15 | 15 | 15 | 15 | 15 | 15 | 15 | 15 |
| Mean | 49.78 | 2.00 | 12.89 | 23.56 | 26.22 | 27.56 | 27.78 | 31.78 | 32.44 | 39.56 | 40.44 | 35.56 | 34.89 | 35.11 |
| Sem | 0.61 | 0.85 | 2.83 | 2.84 | 3.07 | 3.06 | 2.90 | 3.05 | 2.67 | 2.74 | 2.72 | 2.60 | 2.72 | 2.39 |
| SD | 2.35 | 3.29 | 10.97 | 11.02 | 11.88 | 11.85 | 11.25 | 11.81 | 10.35 | 10.61 | 10.53 | 10.05 | 10.53 | 9.25 |

**Table 13: Weight (g) Group 1**

| **Group 1** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 2 | 301 | 284 | 319 | 340 | 355 | 358 | 360 | 351 | 392 | 400 | 386 | 419 | 426 | 415 |
| No. 3 | 305 | 297 | 322 | 358 | 388 | 413 | 422 | 404 | 436 | 440 | 442 | 457 | 463 | 470 |
| No. 7 | 318 | 301 | 334 | 380 | 405 | 407 | 425 | 412 | 444 | 456 | 446 | 463 | 477 | 470 |
| No. 12 | 326 | 323 | 359 | 421 | 457 | 487 | 496 | 477 | 490 | 494 | 490 | 516 | 516 | 513 |
| No. 18 | 295 | 277 | 301 | 334 | 357 | 366 | 372 | 360 | 387 | 390 | 379 | 400 | 405 | 396 |
| No. 21 | 305 | 291 | 328 | 375 | 399 | 427 | 425 | 411 | 448 | 455 | 448 | 476 | 480 | 475 |
| No. 23 | 317 | 317 | 361 | 403 | 419 | 451 | 450 | 441 | 491 | 501 | 490 | 509 | 520 | 511 |
| No. 24 | 311 | 306 | 359 | 394 | 416 | 436 | 435 | 417 | 449 | 447 | 440 | 452 | 460 | 456 |
| No. 25 | 332 | 315 | 375 | 421 | 444 | 484 | 490 | 470 | 499 | 511 | 494 | 523 | 530 | 527 |
| No. 28 | 340 | 323 | 369 | 436 | 494 | 525 | 544 | 515 | 565 | 566 | 570 | 612 | 625 | 615 |
| No. 30 | 324 | 294 | 340 | 382 | 428 | 457 | 470 | 454 | 479 | 480 | 488 | 496 | 512 | 509 |
| No. 31 | 330 | 317 | 366 | 422 | 469 | 506 | 512 | 496 | 543 | 545 | 552 | 576 | 585 | 587 |
| No. 37 | 313 | 300 | 335 | 382 | 380 | 423 | 434 | 422 | 447 | 454 | 457 | 467 | 473 | 467 |
| No. 38 | 322 | 315 | 359 | 404 | 440 | 466 | 482 | 453 | 499 | 502 | 494 | 524 | 530 | 521 |
| No. 42 | 320 | 311 | 344 | 395 | 450 | 469 | 480 | 461 | 497 | 502 | 491 | 500 | 515 | 505 |
| | | | | | | | | | | | | | | |
| N | 15 | 15 | 15 | 15 | 15 | 15 | 15 | 15 | 15 | 15 | 15 | 15 | 15 | 15 |
| Mean | 317.3 | 304.7 | 344.7 | 389.8 | 420.1 | 445.0 | 453.1 | 436.3 | 471.1 | 476.2 | 471.1 | 492.7 | 501.1 | 495.8 |
| Sem | 3.2 | 3.7 | 5.5 | 7.7 | 10.4 | 12.3 | 13.0 | 11.9 | 12.7 | 12.5 | 13.4 | 14.3 | 14.6 | 14.7 |
| SD | 12.4 | 14.2 | 21.4 | 30.0 | 40.3 | 47.6 | 50.2 | 46.1 | 49.2 | 48.6 | 51.9 | 55.2 | 56.6 | 57.1 |

**Table 14: Weight (g) Group 2**

| **Group 2** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 5 | 303 | 278 | 329 | 364 | 409 | 418 | 434 | 424 | 460 | 470 | 465 | 486 | 499 | 488 |
| No. 9 | 304 | 299 | 334 | 364 | 389 | 405 | 420 | 403 | 430 | 440 | 431 | 445 | 456 | 444 |
| No. 11 | 311 | 305 | 330 | 370 | 398 | 415 | 416 | 412 | 454 | 455 | 447 | 464 | 470 | 447 |
| No. 13 | 311 | 309 | 318 | 328 | 359 | 379 | 380 | 374 | 401 | 405 | 394 | 428 | 432 | 417 |
| No. 14 | 316 | 304 | 339 | 381 | 399 | 413 | 431 | 428 | 460 | 455 | 461 | 480 | 483 | 471 |
| No. 19 | 322 | 307 | 353 | 406 | 438 | 479 | 474 | 473 | 492 | 505 | 496 | 524 | 530 | 517 |
| No. 20 | 296 | 278 | 315 | 333 | 364 | 373 | 380 | 369 | 394 | 402 | 391 | 415 | 425 | 410 |
| No. 27 | 319 | 297 | 341 | 383 | 394 | 410 | 414 | 410 | 428 | 441 | 432 | 455 | 461 | 444 |
| No. 32 | 330 | 292 | 338 | 388 | 418 | 434 | 444 | 426 | 458 | 460 | 448 | 472 | 477 | 460 |
| No. 33 | 307 | 286 | 325 | 360 | 390 | 405 | 410 | 406 | 429 | 426 | 427 | 443 | 455 | 441 |
| No. 35 | 332 | 308 | 353 | 401 | 436 | 466 | 480 | 455 | 500 | 505 | 499 | 529 | 540 | 519 |
| No. 39 | 345 | 332 | 388 | 454 | 494 | 525 | 540 | 541 | 577 | 596 | 591 | 620 | 635 | 614 |
| No. 41 | 323 | 322 | 362 | 412 | 444 | 467 | 475 | 467 | 500 | 510 | 510 | 530 | 535 | 508 |
| No. 44 | 318 | 315 | 358 | 424 | 472 | 491 | 512 | 508 | 533 | 540 | 545 | 550 | 570 | 535 |
| No. 45 | 338 | 342 | 362 | 402 | 443 | 470 | 480 | 476 | 515 | 521 | 522 | 538 | 546 | 530 |
| | | | | | | | | | | | | | | |
| N | 15 | 15 | 15 | 15 | 15 | 15 | 15 | 15 | 15 | 15 | 15 | 15 | 15 | 15 |
| Mean | 318.3 | 304.9 | 343.0 | 384.7 | 416.5 | 436.7 | 446.0 | 438.1 | 468.7 | 475.4 | 470.6 | 491.9 | 500.9 | 483.0 |
| Sem | 3.5 | 4.7 | 5.1 | 8.6 | 9.8 | 11.3 | 12.0 | 12.4 | 13.1 | 13.8 | 14.5 | 14.3 | 14.9 | 14.1 |
| SD | 13.7 | 18.1 | 19.6 | 33.5 | 37.9 | 43.8 | 46.4 | 48.0 | 50.6 | 53.5 | 56.1 | 55.5 | 57.6 | 54.5 |

**Table 15: Weight (g) Group 3**

| **Group 3** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 1 | 311 | 306 | 345 | 384 | 418 | 438 | 445 | 445 | 455 | 463 | 446 | 480 | 492 | 468 |
| No. 4 | 303 | 289 | 309 | 357 | 389 | 403 | 419 | 412 | 440 | 442 | 430 | 455 | 462 | 453 |
| No. 6 | 307 | 290 | 337 | 372 | 403 | 413 | 429 | 427 | 440 | 445 | 430 | 452 | 469 | 456 |
| No. 8 | 306 | 316 | 332 | 362 | 392 | 405 | 419 | 416 | 444 | 453 | 436 | 460 | 476 | 470 |
| No. 10 | 306 | 287 | 309 | 350 | 381 | 397 | 411 | 413 | 438 | 445 | 412 | 446 | 461 | 447 |
| No. 15 | 312 | 298 | 335 | 375 | 397 | 425 | 422 | 418 | 451 | 460 | 440 | 461 | 475 | 466 |
| No. 16 | 319 | 316 | 341 | 371 | 392 | 414 | 427 | 425 | 451 | 461 | 436 | 467 | 486 | 479 |
| No. 17 | 311 | 317 | 338 | 376 | 394 | 425 | 440 | 441 | 454 | 469 | 455 | 476 | 485 | 484 |
| No. 22 | 315 | 296 | 339 | 382 | 405 | 430 | 430 | 430 | 455 | 451 | 435 | 462 | 467 | 463 |
| No. 26 | 296 | 284 | 333 | 369 | 376 | 404 | 405 | 401 | 415 | 423 | 402 | 425 | 440 | 430 |
| No. 29 | 307 | 298 | 327 | 371 | 405 | 430 | 436 | 429 | 460 | 462 | 442 | 466 | 472 | 465 |
| No. 34 | 323 | 303 | 346 | 394 | 414 | 425 | 433 | 433 | 460 | 463 | 435 | 483 | 490 | 484 |
| No. 36 | 332 | 317 | 370 | 422 | 467 | 498 | 500 | 504 | 515 | 532 | 502 | 535 | 550 | 550 |
| No. 40 | 346 | 338 | 401 | 456 | 508 | 521 | 540 | 530 | 530 | 572 | 567 | 613 | 625 | 613 |
| No. 43 | 318 | 297 | 350 | 412 | 458 | 487 | 500 | 496 | 523 | 538 | 497 | 553 | 573 | 549 |
| | | | | | | | | | | | | | | |
| N | 15 | 15 | 15 | 15 | 15 | 15 | 15 | 15 | 15 | 15 | 15 | 15 | 15 | 15 |
| Mean | 314.1 | 303.5 | 340.8 | 383.5 | 413.3 | 434.3 | 443.7 | 441.3 | 462.1 | 471.9 | 451.0 | 482.3 | 494.9 | 485.1 |
| Sem | 3.2 | 3.8 | 5.8 | 7.1 | 9.4 | 9.7 | 9.9 | 9.8 | 8.6 | 10.7 | 10.7 | 12.5 | 12.8 | 12.5 |
| SD | 12.4 | 14.8 | 22.4 | 27.7 | 36.5 | 37.5 | 38.5 | 37.9 | 33.4 | 41.4 | 41.6 | 48.6 | 49.5 | 48.4 |

**Table 16: Cylinder Test Score (Overall Asymmetry (%)) Group 1**

| **Group 1** | D-1 | D7 | D21 | D30 | D32 | D44 | D46 | D58 | D60 |
|---|---|---|---|---|---|---|---|---|---|
| | | | | | | | | | |
| No. 2 | 24.49 | 36.51 | 53.85 | 50.00 | | 40.00 | 50.00 | 28.57 | 50.00 |
| No. 3 | 28.36 | -38.89 | | | 50.00 | 33.33 | 33.33 | 43.75 | 53.13 |
| No. 7 | 0.97 | -6.45 | -56.25 | -100.00 | -100.00 | 9.30 | 7.69 | 10.87 | -10.81 |
| No. 12 | 11.90 | 60.87 | -32.00 | 80.00 | 16.67 | 10.00 | -8.33 | 0.00 | -9.52 |
| No. 18 | -11.76 | 34.62 | 28.13 | | -56.52 | -28.57 | | 75.00 | 28.57 |
| No. 21 | 25.00 | 51.91 | 52.94 | | 36.36 | 60.71 | 63.64 | 57.14 | 71.43 |
| No. 23 | 0.00 | 40.00 | 24.49 | 9.33 | 24.07 | 25.81 | 14.89 | 27.74 | 32.10 |
| No. 24 | -1.23 | 48.05 | 41.94 | 0.00 | 22.73 | 39.58 | 30.23 | 33.33 | 39.39 |
| No. 25 | -5.77 | 22.81 | 28.36 | 50.00 | 5.13 | 22.81 | 33.33 | 32.10 | 29.55 |
| No. 28 | 6.00 | 24.46 | 43.60 | | -30.00 | 31.91 | 36.96 | 39.62 | 42.86 |
| No. 30 | -9.43 | -8.57 | 28.57 | 16.67 | -100.00 | 33.33 | -8.33 | 12.50 | 11.11 |
| No. 31 | 0.00 | 36.84 | 34.29 | 50.00 | 50.00 | 39.02 | 33.33 | 43.24 | 36.47 |
| No. 37 | -7.14 | 26.67 | -12.50 | 38.46 | -60.00 | -45.45 | 25.00 | | |
| No. 38 | -6.45 | 37.50 | -1.92 | | | -60.00 | | 9.09 | -33.33 |
| No. 42 | -26.09 | 7.14 | -22.73 | -16.67 | -65.79 | -56.06 | -100.00 | -65.38 | -100.00 |
| | | | | | | | | | |
| N | 15 | 15 | 14 | 10 | 13 | 15 | 13 | 14 | 14 |
| Mean | 1.92 | 24.90 | 15.05 | 17.78 | -15.95 | 10.38 | 16.29 | 24.83 | 17.21 |
| Sem | 3.90 | 6.84 | 9.15 | 15.94 | 15.28 | 10.00 | 11.25 | 8.76 | 11.76 |
| SD | 15.09 | 26.48 | 34.24 | 50.40 | 55.10 | 38.73 | 40.58 | 32.79 | 43.99 |

**Table 17: Cylinder Test Score (Overall Asymmetry (%)) Group 2**

| **Group 2** | D-1 | D7 | D21 | D30 | D32 | D44 | D46 | D58 | D60 |
|---|---|---|---|---|---|---|---|---|---|
| | | | | | | | | | |
| No. 5 | -2.70 | 14.29 | -25.00 | 20.00 | -33.33 | -20.00 | 2.86 | | -100.00 |
| No. 9 | -5.66 | 31.82 | 16.67 | 12.50 | 9.09 | 0.00 | -11.54 | 42.86 | 0.00 |
| No. 11 | -9.52 | 40.54 | 0.00 | 6.90 | 50.00 | -9.76 | 10.00 | -6.67 | 23.08 |
| No. 13 | -14.08 | 20.00 | -19.35 | 23.53 | 4.35 | 2.63 | -16.67 | 33.33 | -22.22 |
| No. 14 | -10.59 | 57.89 | 60.00 | 38.10 | 0.00 | 0.00 | 42.86 | | -100.00 |
| No. 19 | -15.58 | 55.47 | 56.25 | 51.72 | 63.29 | 58.33 | 56.67 | 29.17 | 64.00 |
| No. 20 | -1.96 | 28.85 | -23.17 | 0.00 | 18.18 | 14.04 | 28.57 | 50.00 | 20.00 |
| No. 27 | -24.39 | 25.00 | 66.67 | 4.17 | 33.33 | 38.96 | 34.85 | 66.67 | 14.29 |
| No. 32 | 2.38 | 51.56 | 49.06 | 56.64 | 54.78 | 52.53 | 31.40 | 54.76 | 62.50 |
| No. 33 | 0.00 | 50.00 | 46.43 | 27.59 | 22.73 | 11.54 | 19.05 | 30.00 | 10.00 |
| No. 35 | 1.65 | 58.33 | 66.67 | 75.00 | 62.50 | 54.93 | 29.33 | 88.00 | 76.47 |
| No. 39 | -2.20 | 42.36 | 25.32 | 20.99 | 16.00 | 7.14 | 40.35 | -7.69 | 13.95 |
| No. 41 | -7.58 | 28.57 | 47.83 | 27.50 | 20.00 | 18.18 | 46.15 | | |
| No. 44 | 21.05 | 43.14 | 22.22 | 23.33 | 3.85 | -27.08 | 16.95 | -16.67 | 0.00 |
| No. 45 | 8.45 | 24.32 | 17.86 | 16.67 | 27.66 | 17.65 | 42.86 | 62.50 | 0.00 |
| | | | | | | | | | |
| N | 15 | 15 | 15 | 15 | 15 | 15 | 15 | 12 | 14 |
| Mean | -4.05 | 38.14 | 27.16 | 26.98 | 23.50 | 14.61 | 24.91 | 35.52 | 4.43 |
| Sem | 2.78 | 3.75 | 8.37 | 5.35 | 6.81 | 6.82 | 5.51 | 9.35 | 13.97 |
| SD | 10.76 | 14.51 | 32.41 | 20.70 | 26.38 | 26.40 | 21.35 | 32.38 | 52.26 |

**Table 18: Cylinder Test Score (Overall Asymmetry (%)) Group 3**

| **Group 3** | D-1 | D7 | D21 | D30 | D32 | D44 | D46 | D58 | D60 |
|---|---|---|---|---|---|---|---|---|---|
| | | | | | | | | | |
| No. 1 | -17.65 | 28.57 | 25.00 | 35.59 | 16.67 | 48.28 | 19.23 | 23.91 | 16.00 |
| No. 4 | 6.41 | 40.00 | 41.67 | 50.00 | 42.11 | 36.36 | 0.00 | 5.00 | 31.25 |
| No. 6 | 0.00 | 28.13 | 12.96 | 21.57 | 42.11 | | 36.36 | 14.29 | -6.67 |
| No. 8 | 0.00 | 25.81 | 8.33 | 8.57 | 0.00 | -33.33 | 6.90 | 21.43 | -5.71 |
| No. 10 | 10.00 | 0.00 | 0.00 | -7.41 | 57.14 | 28.57 | -22.22 | 14.29 | 0.00 |
| No. 15 | -4.05 | 54.47 | 46.30 | 28.57 | 34.15 | 56.67 | 32.35 | 24.24 | 6.25 |
| No. 16 | 4.82 | 46.67 | -80.00 | -21.31 | -46.15 | -16.67 | -28.57 | 5.56 | 9.09 |
| No. 17 | 3.75 | 41.89 | 15.52 | 30.89 | 29.68 | 40.00 | 32.73 | 17.95 | 16.13 |
| No. 22 | -7.55 | 44.74 | 64.81 | 56.82 | 46.88 | 20.00 | 50.00 | 55.56 | 52.70 |
| No. 26 | -4.55 | 36.51 | 0.00 | -17.74 | 20.00 | 37.50 | | 0.00 | -23.33 |
| No. 29 | -20.00 | 42.35 | 46.94 | 49.18 | 23.91 | 100.00 | -60.00 | 21.74 | -25.00 |
| No. 34 | 8.33 | 51.09 | 42.86 | 47.69 | 15.00 | 0.00 | 55.56 | 0.00 | 0.00 |
| No. 36 | -6.25 | 58.46 | 30.00 | 35.21 | 15.22 | 35.90 | 17.07 | 26.03 | 22.45 |
| No. 40 | -2.80 | 18.18 | -10.87 | -9.09 | -10.81 | 10.53 | -7.14 | -8.82 | -36.36 |
| No. 43 | 0.97 | 26.09 | 21.74 | 2.27 | 28.57 | | | -36.36 | -8.33 |
| | | | | | | | | | |
| N | 15 | 15 | 15 | 15 | 15 | 13 | 13 | 15 | 15 |
| Mean | -1.90 | 36.20 | 17.68 | 20.72 | 20.96 | 27.98 | 10.17 | 12.32 | 3.23 |
| Sem | 2.23 | 3.96 | 8.84 | 6.80 | 6.63 | 9.37 | 9.25 | 5.24 | 5.93 |
| SD | 8.65 | 15.32 | 34.25 | 26.33 | 25.68 | 33.78 | 33.36 | 20.29 | 22.96 |

**Table 19: Cylinder Test (Total Movement) Group 1**

| **Group 1** | D-1 | D7 | D21 | D30 | D32 | D44 | D46 | D58 | D60 |
|---|---|---|---|---|---|---|---|---|---|
| | | | | | | | | | |
| No. 2 | 49.00 | 63.00 | 13.00 | 4.00 | 0.00 | 25.00 | 2.00 | 7.00 | 4.00 |
| No. 3 | 67.00 | 18.00 | 0.00 | 0.00 | 2.00 | 6.00 | 6.00 | 16.00 | 32.00 |
| No. 7 | 103.00 | 31.00 | 16.00 | 5.00 | 1.00 | 43.00 | 39.00 | 46.00 | 37.00 |
| No. 12 | 42.00 | 46.00 | 25.00 | 5.00 | 6.00 | 20.00 | 24.00 | 16.00 | 21.00 |
| No. 18 | 68.00 | 26.00 | 32.00 | 0.00 | 23.00 | 7.00 | 0.00 | 32.00 | 14.00 |
| No. 21 | 64.00 | 131.00 | 51.00 | 0.00 | 11.00 | 28.00 | 11.00 | 14.00 | 14.00 |
| No. 23 | 152.00 | 130.00 | 98.00 | 75.00 | 54.00 | 93.00 | 47.00 | 137.00 | 81.00 |
| No. 24 | 81.00 | 77.00 | 31.00 | 2.00 | 22.00 | 48.00 | 43.00 | 18.00 | 33.00 |
| No. 25 | 52.00 | 57.00 | 67.00 | 10.00 | 39.00 | 57.00 | 48.00 | 81.00 | 44.00 |
| No. 28 | 100.00 | 139.00 | 172.00 | 0.00 | 10.00 | 47.00 | 46.00 | 53.00 | 28.00 |
| No. 30 | 53.00 | 35.00 | 28.00 | 6.00 | 8.00 | 9.00 | 12.00 | 16.00 | 9.00 |
| No. 31 | 47.00 | 57.00 | 70.00 | 10.00 | 14.00 | 123.00 | 63.00 | 74.00 | 85.00 |
| No. 37 | 14.00 | 15.00 | 8.00 | 13.00 | 15.00 | 11.00 | 4.00 | 0.00 | 0.00 |
| No. 38 | 31.00 | 32.00 | 52.00 | 0.00 | 0.00 | 5.00 | 0.00 | 11.00 | 3.00 |
| No. 42 | 23.00 | 14.00 | 22.00 | 12.00 | 38.00 | 66.00 | 13.00 | 26.00 | 6.00 |
| | | | | | | | | | |
| N | 15 | 15 | 15 | 15 | 15 | 15 | 15 | 15 | 15 |
| Mean | 63.07 | 58.07 | 45.67 | 9.47 | 16.20 | 39.20 | 23.87 | 36.47 | 27.40 |
| Sem | 9.10 | 11.12 | 11.33 | 4.83 | 4.20 | 8.95 | 5.56 | 9.50 | 6.80 |
| SD | 35.25 | 43.05 | 43.89 | 18.71 | 16.27 | 34.66 | 21.53 | 36.78 | 26.32 |

**Table 20: Cylinder Test (Total Movement) Group 2**

| **Group 2** | D-1 | D7 | D21 | D30 | D32 | D44 | D46 | D58 | D60 |
|---|---|---|---|---|---|---|---|---|---|
| | | | | | | | | | |
| No. 5 | 74.00 | 14.00 | 8.00 | 35.00 | 9.00 | 35.00 | 35.00 | 0.00 | 1.00 |
| No. 9 | 53.00 | 44.00 | 6.00 | 8.00 | 11.00 | 31.00 | 26.00 | 7.00 | 1.00 |
| No. 11 | 42.00 | 37.00 | 14.00 | 29.00 | 2.00 | 41.00 | 30.00 | 15.00 | 13.00 |
| No. 13 | 71.00 | 50.00 | 31.00 | 17.00 | 23.00 | 38.00 | 24.00 | 3.00 | 9.00 |
| No. 14 | 85.00 | 19.00 | 5.00 | 21.00 | 1.00 | 6.00 | 7.00 | 0.00 | 1.00 |
| No. 19 | 77.00 | 137.00 | 64.00 | 87.00 | 79.00 | 60.00 | 60.00 | 24.00 | 25.00 |
| No. 20 | 51.00 | 104.00 | 82.00 | 17.00 | 11.00 | 57.00 | 35.00 | 12.00 | 10.00 |
| No. 27 | 41.00 | 4.00 | 15.00 | 24.00 | 3.00 | 77.00 | 66.00 | 9.00 | 7.00 |
| No. 32 | 126.00 | 64.00 | 53.00 | 143.00 | 115.00 | 99.00 | 86.00 | 42.00 | 16.00 |
| No. 33 | 46.00 | 24.00 | 56.00 | 29.00 | 22.00 | 26.00 | 21.00 | 10.00 | 20.00 |
| No. 35 | 182.00 | 60.00 | 21.00 | 28.00 | 32.00 | 71.00 | 75.00 | 25.00 | 17.00 |
| No. 39 | 91.00 | 144.00 | 79.00 | 81.00 | 75.00 | 84.00 | 114.00 | 13.00 | 43.00 |
| No. 41 | 66.00 | 14.00 | 46.00 | 40.00 | 35.00 | 22.00 | 26.00 | 0.00 | 0.00 |
| No. 44 | 95.00 | 51.00 | 108.00 | 120.00 | 78.00 | 48.00 | 59.00 | 6.00 | 28.00 |
| No. 45 | 71.00 | 37.00 | 28.00 | 24.00 | 47.00 | 51.00 | 21.00 | 8.00 | 3.00 |
| | | | | | | | | | |
| N | 15 | 15 | 15 | 15 | 15 | 15 | 15 | 15 | 15 |
| Mean | 78.07 | 53.53 | 41.07 | 46.87 | 36.20 | 49.73 | 45.67 | 11.60 | 12.93 |
| Sem | 9.49 | 11.16 | 8.23 | 10.59 | 9.08 | 6.54 | 7.68 | 2.95 | 3.17 |
| SD | 36.77 | 43.22 | 31.87 | 41.02 | 35.18 | 25.34 | 29.74 | 11.41 | 12.26 |

**Table 21: Cylinder Test (Total Movement) Group 3**

| **Group 3** | D-1 | D7 | D21 | D30 | D32 | D44 | D46 | D58 | D60 |
|---|---|---|---|---|---|---|---|---|---|
| | | | | | | | | | |
| No. 1 | 51.00 | 14.00 | 12.00 | 59.00 | 18.00 | 29.00 | 52.00 | 46.00 | 50.00 |
| No. 4 | 78.00 | 50.00 | 36.00 | 80.00 | 57.00 | 11.00 | 11.00 | 20.00 | 16.00 |
| No. 6 | 52.00 | 32.00 | 54.00 | 51.00 | 19.00 | 0.00 | 11.00 | 28.00 | 30.00 |
| No. 8 | 36.00 | 31.00 | 24.00 | 35.00 | 52.00 | 27.00 | 29.00 | 28.00 | 35.00 |
| No. 10 | 20.00 | 16.00 | 3.00 | 27.00 | 7.00 | 7.00 | 9.00 | 7.00 | 5.00 |
| No. 15 | 74.00 | 123.00 | 54.00 | 63.00 | 82.00 | 60.00 | 34.00 | 33.00 | 32.00 |
| No. 16 | 83.00 | 15.00 | 10.00 | 61.00 | 13.00 | 12.00 | 7.00 | 18.00 | 11.00 |
| No. 17 | 80.00 | 74.00 | 58.00 | 123.00 | 155.00 | 60.00 | 55.00 | 78.00 | 31.00 |
| No. 22 | 53.00 | 38.00 | 54.00 | 44.00 | 32.00 | 5.00 | 4.00 | 63.00 | 74.00 |
| No. 26 | 22.00 | 63.00 | 71.00 | 62.00 | 10.00 | 8.00 | 0.00 | 12.00 | 30.00 |
| No. 29 | 85.00 | 85.00 | 49.00 | 61.00 | 46.00 | 4.00 | 5.00 | 23.00 | 4.00 |
| No. 34 | 48.00 | 92.00 | 7.00 | 65.00 | 60.00 | 6.00 | 9.00 | 11.00 | 10.00 |
| No. 36 | 32.00 | 65.00 | 10.00 | 71.00 | 46.00 | 39.00 | 41.00 | 73.00 | 49.00 |
| No. 40 | 107.00 | 22.00 | 46.00 | 22.00 | 37.00 | 19.00 | 14.00 | 34.00 | 11.00 |
| No. 43 | 103.00 | 23.00 | 23.00 | 44.00 | 28.00 | 0.00 | 0.00 | 11.00 | 12.00 |
| | | | | | | | | | |
| N | 15 | 15 | 15 | 15 | 15 | 15 | 15 | 15 | 15 |
| Mean | 61.60 | 49.53 | 34.07 | 57.87 | 44.13 | 19.13 | 18.73 | 32.33 | 26.67 |
| Sem | 7.18 | 8.54 | 5.80 | 6.25 | 9.64 | 5.17 | 4.80 | 5.90 | 5.12 |
| SD | 27.82 | 33.06 | 22.46 | 24.22 | 37.32 | 20.03 | 18.58 | 22.86 | 19.83 |

MCAO resulted in substantial acute loss of sensorimotor function which recovered partially, approaching a plateau of stable deficits in all animals by the end of the 4-week pre-treatment period.

All groups (1-3) demonstrated a typical recovery response to the MCAO-induced ischemia with normal scores of 0 just prior to the surgery (Day -1) followed by a complete loss in function (score 12, forelimb; 6, hindlimb) within 24 hours after the occlusion (Day 1). During the next 4 weeks, untreated phase, forelimb and hindlimb scores improved to approximately 5.5 and 3, respectively, and approached a plateau level of recovery (Figures 3 and 4). In the body swing test, animals displayed less than 5 percent swings to the right the day after surgery and had recovered to approximately 25 percent right swings by the end of the 4 week untreated period (Figure 5). While not significant, baseline behavioral measures improved slightly relative to pretreatment levels during the drug-free periods between phases 1 and 2 and phases 2 and 3. This may be due to slow continued endogenous recovery, training effects of repeated behavioral assessments and possibly carry-over effects of treatment.

All animals received each of the treatments by the end of the study. 4-AP was administered to rats twice a day (in this study and in the study presented in Example 17). With a half life of 1-1.5 hours (Hayes et al., J. Clin. Pharmacol. 2003; 43:379-85) this regimen did not sustain long term plasma levels of the compound, but it did allow for repeated daily exposure in the animals. Behavioral evaluations were performed at 1 hour after dosing to ensure adequate exposure during the time of assessment and the three day interval for each dosing phase may have helped adapt the animals to the stress of oral gavage prior to conducting behavioral assessments. Blood was drawn 30 minutes later to confirm a dose-associated level of dalfampridine in the animals upon completion of behavioral assessments (Table 22). It is noted that it is not possible to equate the doses used here or the plasma concentrations obtained with what would be expected in patients treated with a sustained release formulation of the drug, where the pharmacokinetics are very different. There is also a delay in the peak concentration measured in cerebrospinal fluid compared to that in the blood, which is approximately an hour in human subjects (Donovan et al., Spinal Cord 2000; 38:7-15). Therefore, the concentration of 4-AP achieved in the central nervous system for a given plasma level is likely to be much less for a transient plasma peak following gavage compared to a similar concentration maintained for a longer period of time.

The forelimb placing test shows the effect of the treatment on forelimb function. Figure 3 indicates that the treatment with either low dose or high dose 4-aminopyridine, 4 weeks post ischemic brain injury, is effective to improve forelimb function in rats. Figure 3 also indicates that the effect is dose-responsive. This effect is also reversible as it diminishes upon withdrawal of the drug.

The hindlimb placing test shows the effect of the treatment on hindlimb function. Figures 4A-D indicate that the treatment with either low dose or high dose 4-aminopyridine, 4 weeks post ischemic brain injury, is effective to improve hindlimb function in rats. Figures 4A-D also indicate that the effect is reversible. Notably, the effect is dose responsive as the treatment with a higher dose results in an improved behavioral score, relative to the treatment with a lower dose or vehicle control.

The body swing test shows the effect of the treatment on global body control. Figure 5 shows that the treatment with either low dose or high dose 4-aminopyridine is effective to improve the percentage of rightward over total swings in rats, and thus, effective to improve one of the symptoms of ischemic stroke. Thus, Figure 5 shows that 4-aminopyridine is effective to improve global body control in rats. Figure 5 also demonstrates that this effect is reversible and dose-dependent.

Group 1 animals (Figures 3-5) which received 4-aminopyridine at 2 mg/kg during the first dosing phase showed significant improvements in forelimb, hind limb and body swing scores compared to pre-treatment baseline scores (Day 28 vs. Day 32; p values < 0.05). Between dosing phases 1 and 2 (washout period, Days 33-42), the effects on limb placing returned to near baseline levels. During the second dosing phase, animals in Group 1 received 4-aminopyridine at 0.63 mg/kg. All behavioral scores were significantly improved compared to scores during the washout just prior to dosing (Day 42 vs. Day 46; p's<0.05), though they did not achieve the same degree of improvement as during the first higher dose phase. During the washout between the second and third phases (Days 47-56) the behavioral scores declined to a level similar to baseline scores (Day 56). Animals in this group received vehicle during the third dosing phase and saw no change in behavioral scores compared with the day immediately prior to dosing (on Day 56).

Group 2 animals (Figure 3-5) receiving 4-aminopyridine at 0.63 mg/kg during the first dosing phase showed significantly improved behavioral scores in all measures compared to pre-treatment baseline scores (Day 28 vs. Day 32; p values < 0.05). Between dosing phases 1 and 2, while animals were not on drug, the effects on behavior declined to levels similar to pre-phase dosing (Day 42). During dosing phase 2, animals in this group received vehicle, and demonstrated no change in behavioral testing scores. They remained at that baseline level of function during the washout between phases 2 and 3 (Days 47-56). Animals in this group received 4-aminopyridine at 2 mg/kg during phase 3 of dosing and all behavioral testing scores were significantly improved compared to pre-phase baseline scores (Day 56 vs. Day 60; p values < 0.05).

Animals in Group 3 (Figures 3-5) had results similar to those seen in Group 1 and 2 during the different treatment phases. These animals received vehicle during the phase 1. There was no change in any behavioral score and animals stayed at this level of function through the washout between phases 1 and 2. 4-aminopyridine treatment at 2 mg/kg during phase 2 and 0.63 mg/kg during phase 3 produced significant improvements in limb placing compared to the off-drug assessments just prior to each phase (Day 42 vs. Day 46 and Day 56 vs. Day 60, respectively; p values < 0.05). Body swing scores were improved during the high dose treatment in the phase 2 (Day 42 vs. Day 46; p<0.05), but were unchanged with the low dose treatment during the third treatment phase. There was a return to baseline behavior during the washout between phases 2 and 3 (Day 56).

Taken together, all animals responded similarly to the respective treatments regardless of the order in which they were treated. In all cases, the highest dose during any dosing phase resulted in significant improvements (p values < 0.05) compared to vehicle and the lower dose, and the lower dose was statistically better or trended toward significance compared to vehicle, depending on the statistical model employed (ANOVA or mixed-model analysis, see Statistical Methods, above).

In addition to being evaluated weekly prior to treatment, assessments were performed twice during any given dosing phase (after the 1^{st} and 5^{th} doses). Slight improvements between these scores were noted (for example, Group 3, between Day 30 and Day 32, when animals received vehicle treatment). This could have been due to acclimation to the stress of oral gavage, or perhaps are indicative of a learning response as the animals become familiar with and anticipate the tests. This effect was not observed in the study presented in Example 17 (see vehicle group, Day 56 and on) where the animals were tested just once during each of the 3 day dosing periods. As the baseline was still slightly improving and as all possible dosing sequences were not tested, it was not possible to determine if a previous exposure to 4-AP pre-disposes animals to greater or lesser response when dosed with 4-AP at a later phase. To eliminate this potential carry-over effect from dose order variability, the study presented in Example 17 was designed as a dose escalation study without washout periods.

The cylinder test shows the effect of the treatment on aspects of the global body control such as body symmetry and coordination. Figure 7 shows that the treatment with 4-aminopyridine is effective to improve the asymmetry in limb usage resulting from the stroke by showing increase in percentage of use of the impaired forelimb relative to the total limb use in rats. Thus, Figure 7 shows that 4-aminopyridine is effective to improve body symmetry and coordination in rats. Figure 7 also demonstrates that this effect is reversible and dose-dependent.

Figure 9 shows that no differences in infarct volume were observed between Groups 1-3. In particular, mean infarct volumes (% of contralateral hemisphere) were not different between any of the groups. Mean infarct volume (%) in Group 1 was 45.0 (±1.8), in Group 2 was 41.4 (±2.3), and in Group 3 was 39.0 (±3.3).

4-aminopyridine plasma levels: Blood samples drawn when the animals were receiving vehicle treatment had levels of 4-aminopyridine below the lower limit of quantitation for the method. Samples drawn when animals received 4-aminopyridine confirmed exposure at the time of behavioral testing appropriately related to dose level. 4-aminopyridine plasma levels are shown in Table 22.

**Table 22: 4-aminopyridine plasma levels.**

| Group | Mean (SE) 4-aminopyridine Plasma Level (ng/mL) | | |
|---|---|---|---|
| | Treatment Phase | | |
| | Phase 1 | Phase 2 | Phase 3 |
| Group 1--H, L, V | 142.4 (6.7) | 64.0 (2.3) | BLOQ* |
| Group 2--L, V, H | 78.1 (10.3) | BLOQ* | 144.1 (7.4) |
| Group 3--V, H, L | BLOQ* | 128.6 (5.6) | 61.8 (3.3) |

| | | | |
|---|---|---|---|
| SE, standard error *BLOQ=below lower limit of quantitation (<1.0 ng/mL) | | | |

The data show that during each separate treatment phase and overall, 4-aminopyridine treatment resulted in significant improvement in forelimb, hindlimb and body-swing function. Further, several cross over statistical models utilized by the inventors demonstrated that the high dose was significantly better (p<0.0001 for limb placing tests, and p<0.001 for body swing) than both the vehicle control and the low dose on a consistent basis. The low dose showed either a strong trend or reached significance for improvement compared with vehicle control. Additionally, scores during the second assessment within a dosing phase were significantly better than the first on-drug assessment. Thus, this example demonstrates significant reversible and dose dependent improvements in forelimb and hind limb sensorimotor function during times when 4-AP was at detectable plasma levels in the animals. The body swing test data also indicates dose dependent effect on recovery of postural function. This may be evidence of effects on tracts in the striatum, or perhaps effects on subcortical white matter areas. Further, this example demonstrates clear and dose-dependent response to treatment with 4-AP within each group and between groups at each phase.

In addition, the results in Figures 3-8 show that continued treatment with 4-aminopyridine may yield further improvement in sensorimotor behavioral outcome. In particular, behavioral scores after administration of multiple doses of 4-aminopyridine are, on average, improved relative to behavioral scores after a single dose of 4-aminopyridine.

These results indicate that treatment with 4-aminopyridine is effective to improve sensorimotor functions in mammals suffering from stroke-related impairment of such functions. These results also demonstrate improvements in stroke-related sensorimotor impairments when the treatment is initiated during a chronic period, with stable motor deficits, following the stroke event. Based on this data it can be concluded that 4-aminopyridine significantly improves chronic sensorimotor deficits post-stroke.

### 6.3 Example 3: Treatment of Ischemic Stroke

A patient presents to a medical facility with signs and symptoms of an ischemic stroke. The patient is revascularized with tPA or other therapy to restore blood flow. Although blood flow has been restored, some level of brain injury has occurred. Three days after the stroke, the patient is assessed neurologically and shown to have measurable sensorimotor deficits. Beginning on day 4, after day 2 and after day 3, this patient is treated with 4-aminopyridine at a dose between 0.01 and 1.0 mg/kg per dose, intravenously for 10 days to 3 months. During treatment and after treatment, sensorimotor function is evaluated.

### 6.4 Example 4: Treatment of Stroke and Resulting Paralysis of the Right Hand

A patient presents to the Emergency Department with paralysis of the right hand. Following evaluation and imaging it is determined that the patient has suffered an ischemic stroke. The patient receives tPA according to approved methods, and blood flow is restored through the thrombosis. However, a week after tPA treatment, the patient has residual paralysis of the right hand as measured by standard neurological measures of hand motor activity. This patient begins to be treated with 4-aminopyridine 1, 2, 3, 4, 5, 6, 7, 8, 9, 10, 11, 12, 13, 14, 15, 16, 17, 18, 19, 20, or 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, or 66 months; 0.5, 1, 1.5, 2, 2.5, 3, 3.5, 4, 4.5, 5, 5.5, 6 or 6.5 years or longer after the stroke event (0.01 to 1.0 mg/kg, IV) once per week for 4 weeks. Improvement in hand function is measured periodically by a neurologist or other physician with standard neurological testing including dynamometer and other strength testing. During treatment and after treatment, sensorimotor function in the right hand is evaluated.

### 6.5 Example 5: Treatment of Ischemic Stroke

A patient presents to a medical facility with signs and symptoms of an ischemic stroke. They are found to have paralysis of their left side. The patient does not arrive in time for revascularization therapy. Upon clinical evaluation it is found that some brain injury has occurred. Three days after the stroke the patient is assessed neurologically and shown to have measurable sensorimotor deficits. This patient begins to be treated with 4-aminopyridine 1, 2, 3, 4, 5, 6, 7, 8, 9, 10, 11, 12, 13, 14, 15, 16, 17, 18, 19, 20, or 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, or 66 months; 0.5, 1, 1.5, 2, 2.5, 3, 3.5, 4, 4.5, 5, 5.5, 6 or 6.5 years or longer after the stroke event at a dose between 0.01 and 1.0 mg/kg per dose, intravenously each day for four weeks; thereafter they receive weekly doses for six months. They also receive physical therapy. During treatment (e.g., after 2 weeks) and after treatment, sensorimotor function of the left side is evaluated.

### 6.6 Example 6: Treatment of Ischemic Stroke

A patient presents to the Emergency Department with paralysis of the left hand. The patient reports that the problem with their hand began "over a week ago". Following evaluation and imaging it is determined that the patient has suffered an ischemic stroke. The patient does not receive tPA. Upon neurological exam it is found that the patient has residual paralysis of the left hand as measured by standard neurological measures of hand motor activity; the patient has a sensory deficit as well. The patient refuses to participate in physical or occupational therapy. This patient begins to be treated with 4-aminopyridine 1, 2, 3, 4, 5, 6, 7, 8, 9, 10, 11, 12, 13, 14, 15, 16, 17, 18, 19, 20, or 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, or 66 months; 0.5, 1, 1.5, 2, 2.5, 3, 3.5, 4, 4.5, 5, 5.5, 6 or 6.5 years or longer after the stroke event (0.01 to 1.0 mg/kg, IV) once per week for 12 weeks. Improvement in hand function is measured periodically by a neurologist or other physician with the standard neurological testing including dynamometer and other strength testing. During treatment (e.g., after 2 weeks of treatment) and after treatment, sensorimotor function in the left hand is evaluated.

### 6.7 Example 7: Treatment of Hemorrhagic Stroke

A patient presents to a medical facility with signs and symptoms consistent with an ischemic stroke or cerebral hemorrhage. The patient is stabilized. Upon neurological assessment it is found that some level of brain injury has occurred. One week after the stroke the patient is again assessed neurologically and shown to have measurable sensorimotor deficits. This patient begins to be treated with 4-aminopyridine 1, 2, 3, 4, 5, 6, 7, 8, 9, 10, 11, 12, 13, 14, 15, 16, 17, 18, 19, 20, or 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, or 66 months; 0.5, 1, 1.5, 2, 2.5, 3, 3.5, 4, 4.5, 5, 5.5, 6 or 6.5 years or longer after the stroke event at a dose between 0.01 and 1.0 mg/kg daily, intravenously for 10 days, followed by administration of this dose weekly for 2 months, at which point all treatment is discontinued. The sensorimotor function is evaluated (e.g., after 1, 2, 3, 4, 5, 6 weeks, and/or 1, 2, 3, 4, 5, 6, 7, 8, 9, 10, 11, or 12 months post-stroke and/or from the start of therapy).

### 6.8 Example 8: 4-aminopyridine Treatment of Stroke-related Injury including Treatment in Chronic Periods

For a comprehensive trial, inclusion criteria include: adults, male and female, with clinical evidence of neural injury.

Indications to be explored:
Ischemic stroke with thrombolytics,
Ischemic stroke without thrombolytics,
Hemorrhagic stroke.

Dose Ranges to be explored:
0.001 mg/kg to 10.0 mg/kg per dose.

Dose Frequencies to be explored:
daily
on alternate days
every fourth day
once per week
once every other week
once per month.

Mixed Periodicity Regimens:
- daily for one or two weeks and then weekly, biweekly, or monthly for the remainder of the study
- on alternate days for one or two weeks and then weekly, biweekly or monthly thereafter.

Initiation of treatment to be explored:
1, 2, 3, 4, 5, 6, 7, 8, 9, 10, 11, 12, 13, 14, 15, 16, 17, 18, 19, 20, or 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, or 66 months; 0.5, 1, 1.5, 2, 2.5, 3, 3.5, 4, 4.5, 5, 5.5, 6 or 6.5 years or longer after the stroke event.

Treatment Duration to be explored:
treatment for 1, 2, 4, 10, 30 weeks.
treatment for 1, 2, 3, 4, 5, 6, 7, 8, 9, 10, 11, 12 months
treatment for 1, 2, 3, 4, 5, 6, 7, 8, 9, 10 years.

Function to be explored:
hand motor function,
face motor function,
survival
time to return to work.

Recovery is measured by standard neurological measures.

Results: Upon treatment as described above, sensorimotor function is evaluated in patients treated with 4-aminopyridine and in patients treated with placebo using methodologies known in the art, and the test results are compared.

In alternative embodiments, combinations of less than all of the above parameters are explored.

### 6.9 Example 9: 4-aminopyridine Treatment of Ischemic Stroke with unilateral hand weakness and/or paralysis (without thrombolysis)

Inclusion criteria include: adults, male and female, evidence of stroke based on loss of consciousness, disorientation, speech difficulty, facial or limb paralysis. Ischemic stroke confirmed with radiographic imaging.

Patients are selected for those with unilateral hand weakness and/or paralysis that are not candidates for tPA (or other thrombolytic) or who did not previously receive tPA for any reason. Consents are obtained from the patients and/or someone with authority to sign for the patients.

Patients are enrolled and randomized to receive 4-aminopyridine or placebo 1, 2, 3, 4, 5, 6, 7, 8, 9, 10, 11, 12, 13, 14, 15, 16, 17, 18, 19, 20, or 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, or 66 months; 0.5, 1, 1.5, 2, 2.5, 3, 3.5, 4, 4.5, 5, 5.5, 6 or 6.5 years or longer after the stroke event and starting as soon as they present to a medical facility including hospital or physician's office, diagnosis, imaging is obtained.

For this trial, treatment is initiated between 1 hour and 7 days after injury. Treatment is continued for 3 months with dosing on alternate days for 1 week and then weekly for the remainder of the treatment period. Patients are dosed with 0.0001 to 1.0 mg per kg, intravenously, intramuscularly, or subcutaneously.

Recovery is measured by standard neurological measures of hand sensorimotor activity every other week for the duration of the study.

Results: Upon treatment as described above, hand function is evaluated in patients treated with 4-aminopyridine and patients treated with placebo using methodologies known in the art, and the test results are compared.

### 6.10 Example 10: 4-aminopyridine Treatment of Stroke with Unilateral Facial Paralysis without Thrombolytics

Patients are selected for those with unilateral facial paralysis who did not or cannot receive thrombolytics. Function is assessed by methodologies known in the art, on alternate weeks during the 3 month dosing period. Consents are obtained from the patients and/or someone with authority to sign for the patients.

Patients are enrolled and randomized to receive 4-aminopyridine or placebo. Treatment is initiated 1, 2, 3, 4, 5, 6, 7, 8, 9, 10, 11, 12, 13, 14, 15, 16, 17, 18, 19, 20, or 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, or 66 months; 0.5, 1, 1.5, 2, 2.5, 3, 3.5, 4, 4.5, 5, 5.5, 6 or 6.5 years or longer after the stroke event.. Treatment is continued for 3 months with dosing on alternate days for 1 week and then weekly for the remainder of the treatment period. Patients are dosed with 0.0001 to 1.0 mg per kg, intravenously, intramuscularly, or subcutaneously.

Results: Upon treatment as described above, facial movement is assessed in patients treated with 4-aminopyridine and patients treated with placebo; the test results are compared.

### 6.11 Example 11: 4-aminopyridine Treatment of Ischemic stroke (with thrombolysis)

Inclusion criteria include: adults, male and female, evidence of stroke based on loss of consciousness, disorientation, speech difficulty, facial or limb paralysis. Ischemic stroke is confirmed with radiographic imaging.

Patients are selected for those with unilateral hand weakness and/or paralysis that have been treated with tPA or other thrombolytic. Consents are obtained from the patients and/or someone with authority to sign for the patients

Patients are enrolled and randomized to receive 4-aminopyridine or placebo starting as soon as they present to a medical facility, diagnosis, and imaging is completed.

Treatment is initiated 1, 2, 3, 4, 5, 6, 7, 8, 9, 10, 11, 12, 13, 14, 15, 16, 17, 18, 19, 20, or 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, or 66 months; 0.5, 1, 1.5, 2, 2.5, 3, 3.5, 4, 4.5, 5, 5.5, 6 or 6.5 years or longer after the stroke event. Treatment is continued for 3 months with dosing on alternate days for 1 week and then weekly for the remainder of the treatment period. Patients are dosed with 0.0001 to 1.0 mg per kg, intravenously, intramuscularly, or subcutaneously.

Recovery is measured by standard neurological measures of hand sensorimotor activity every other week for the duration of the study.

Results: Upon treatment as described above, hand function in patients treated with 4-aminopyridine is measured by methodologies known in the art and compared to that in patients treated with placebo.

### 6.12 Example 12: 4-aminopyridine Treatment of Stroke with Dysarthria without Thrombolytics

Patients are selected for those with dysarthria who did not or cannot receive thrombolytics. Function is assessed by methodologies known in the art on alternate weeks during the 3 month dosing period. Consents are obtained from the patients and/or someone with authority to sign for the patients.

Patients are enrolled and randomized to receive 4-aminopyridine or placebo. Treatment is initiated 1, 2, 3, 4, 5, 6, 7, 8, 9, 10, 11, 12, 13, 14, 15, 16, 17, 18, 19, 20, or 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, or 66 months; 0.5, 1, 1.5, 2, 2.5, 3, 3.5, 4, 4.5, 5, 5.5, 6 or 6.5 years or longer after the stroke event. Treatment is continued for 3 months with dosing on alternate days for 1 week and then weekly for the remainder of the treatment period. Patients are dosed with 0.0001 to 1.0 mg per kg, intravenously, intramuscularly or subcutaneously.

Results: Upon treatment as described above, speech impairment due to dysarthria in patients treated with 4-aminopyridine is assessed using methodologies known in the art and compared to that in patients treated with placebo.

### 6.13 Example 13: 4-aminopyridine Treatment of Patients with Dysarthria (With Thrombolytics)

Inclusion criteria include: adults, male and female, evidence of stroke based on loss of consciousness, disorientation, speech difficulty, facial or limb paralysis. Ischemic stroke is confirmed with radiographic imaging. Patients are selected for those with dysarthria and who did receive thrombolytics.

Treatment is initiated 1, 2, 3, 4, 5, 6, 7, 8, 9, 10, 11, 12, 13, 14, 15, 16, 17, 18, 19, 20, or 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, or 66 months; 0.5, 1, 1.5, 2, 2.5, 3, 3.5, 4, 4.5, 5, 5.5, 6 or 6.5 years or longer after the stroke event. Treatment is continued for 3 months with dosing on alternate days for 1 week and then weekly for the remainder of the treatment period. Patients are dosed with 0.0001 to 1.0 mg per kg, intravenously, intramuscularly or subcutaneously.

Function is assessed by methodologies known in the art, on alternate weeks during the 3 month dosing period.

Results: Upon treatment as described above, speech impairment due to dysarthria in patients treated with 4-aminopyridine and patients treated with placebo is assessed and compared.

### 6.14 Example 14: 4-aminopyridine Treatment of Patients with Hemorrhagic Stroke

Inclusion criteria include: adults, male and female, evidence of stroke based on loss of consciousness, disorientation, speech difficulty, facial or limb paralysis. Hemorrhagic stroke confirmed with radiographic imaging. Consents are obtained from the patients and/or someone with authority to sign for the patients.

Patients are selected for those with unilateral hand weakness.

Patients are enrolled and randomized to receive 4-aminopyridine or placebo starting as soon as they present to a medical facility including hospital or physician's office, diagnosis, imaging and consent is obtained.

Treatment is initiated 1, 2, 3, 4, 5, 6, 7, 8, 9, 10, 11, 12, 13, 14, 15, 16, 17, 18, 19, 20, or 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, or 66 months; 0.5, 1, 1.5, 2, 2.5, 3, 3.5, 4, 4.5, 5, 5.5, 6 or 6.5 years or longer after the stroke event. Treatment is continued for 3 months with dosing on alternate days for 1 week and then weekly for the remainder of the treatment period. Patients are dosed with 0.0001 to 1.0 mg per kg, intravenously, intramuscularly, or subcutaneously.

Recovery is measured by standard neurological measures of hand sensorimotor activity every other week for the duration of the study.

Results: Upon treatment as described above, hand function in patients treated with 4-aminopyridine is measured by methodologies known in the art and compared to that in patients treated with placebo.

### 6.15 Example 15: 4-aminopyridine Treatment of Patients with Hemorrhagic Stroke

In humans, the effects of administering a single dose or multiple daily doses of 4AP at dose levels of 0.05 and 0.1 on functional outcome following thrombotic stroke are evaluated using the protocol below.

Purpose: To assess the efficacy and safety of orally or intravenously administered 4AP in chronic ischemic stroke.

Design: multicenter, randomized, double-blind, placebo controlled, safety and efficacy study.

Inclusion Criteria: Patients with ischemic stroke and limb weakness and full functional independence before stroke.

Exclusion Criteria: Patients with a severe illness with life expectancy less than 6 months, known severe kidney disorder, current known alcohol or illicit drug abuse or dependence. Patients with hemorrhagic stroke are excluded.

Patient Involvement: Patients are randomized to receive a single dose of 4AP or placebo, or 2 weeks' daily dose of 4AP or placebo beginning 1, 2, 3, 4, 5, 6, 7, 8, 9, 10, 11, 12, 13, 14, 15, 16, 17, 18, 19, 20, or 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, or 66 months; 0.5, 1, 1.5, 2, 2.5, 3, 3.5, 4, 4.5, 5, 5.5, 6 or 6.5 years or longer after the stroke event. Blood samples are collected at multiple time points after 4AP treatment to determine plasma drug concentrations around the time when sensorimotor function outcome is evaluated.

Primary Outcome: Overall recovery and recovery of sensorimotor function at 2 days (single dosing) or 2 weeks (multiple dosing) after treatment is measured by the modified Rankin Scale and the NIH scale.

Upon treatment with 4-AP, sensorimotor function in stroke patients following single or multiple administration is measured and compared to baseline function and placebo treatment.

### 6.16 Example 16: A Study of Dalfampridine 10 mg Extended Release Tablet in Subjects with Chronic Deficits after Ischemic Stroke

### 6.16.1 LIST OF ABBREVIATIONS

The following abbreviations and specialist terms are used in this study protocol:

| **Abbreviation or Specialist Term** | **Explanation** |
|---|---|
| ADL | Activities of daily living |
| AE | Adverse event |
| AST | Aspartate aminotransferase |
| BDI | Beck Depression Inventory |
| BMI | Body mass index |
| BUN | Blood urea nitrogen |
| C | Celsius |
| CFB | Change from baseline |
| CGI | Clinician Global Impression |
| CP | Cerebral palsy |
| CRF | Case Report Form |
| EOS | Early onset seizure |
| ER | Extended release |
| F | Fahrenheit |
| FAP | Full Analysis Population |
| FIM | Functional Independence Measure |
| FMA | Fugl-Meyer Assessment |
| GCP | Good Clinical Practice |
| HDPE | High density polyethylene |
| HEENT | Head Ears Eyes Nose Throat |
| ICH | International Conference on Harmonization |
| IEC | Independent Ethics Committee |
| IND | Investigational New Drug |
| INN | International Nonproprietary Name |
| IRB | Institutional Review Board |
| LOS | Late onset seizure |
| LEMMT | Lower Extremity Manual Muscle Testing |
| mg | Milligram |
| MRI | Magnetic resonance imaging |
| MS | Multiple sclerosis |
| MSWS-12 | 12-item Multiple Sclerosis Walking Scale |
| PPP | Per-Protocol Population |
| RBC | Red blood cell |
| SAE | Serious adverse event |
| SGI | Subject Global Impression |
| SSRI | Serotonin reuptake inhibitors |
| T25FW | Timed 25 Foot Walk |
| TMS | Transcranial magnetic stimulation |
| UPT | Urine pregnancy test |
| US | United States |
| USAN | United States Adopted Name |
| UTI | Urinary tract infection |
| WBC | White blood cell |

### 6.16.2 STUDY OBJECTIVES

The examine the effects of dalfampridine-ER 10 mg (i.e., a sustained release formulation of 10 mg 4-aminopyridine) administered twice daily approximately 12 hours apart on the following clinical functions:
- Walking speed as measured by the Timed 25 Foot Walk test (T25FW)
- Manual dexterity as measured by the Box and Block Test
- Hand strength as measured by the grip test and pinch tests
- Motor and sensory function as measured by Fugl-Meyer Assessment (FMA)
- Optionally, upper limb spasticity as measured by the Disability Assessment Scale (DAS)
- Assistance required to perform activities of daily living (ADL) by the Functional Independence Measure (FIM) scale
- Subject Global Impression (SGI) scale
- Clinician Global Impression (CGI) scale
- Optionally, depression as measured by the Beck Depression Inventory (BDI) (to rule out severe depression)

### 6.16.3 INVESTIGATION PLAN

This is a study of dalfampridine-ER 10 mg taken twice daily, approximately 12 hours apart, in subjects with chronic stable sensorimotor deficits after ischemic stroke. The study is designed as a double-blind, placebo-controlled, 2-period crossover study. It will be conducted at multiple sites, with 66 subjects planned. The study duration of 8 weeks includes a 2-week screening period, 2 weeks of treatment in the first period, a 1-week washout period, 2 weeks of treatment in the second period, and a 1-week post-treatment follow-up call. Adverse events will be monitored through the duration of the study. Additionally, brief physical examinations and vital sign measurements will be performed during each study period to assess potential changes from baseline. A set of functional and subjective clinical assessments, described below, will also be administered.

After obtaining subject informed consent, eligibility will be determined at a screening visit (Visit 1) through a review of medical history, results from prior brain imaging, SMA-12 chemistry tests including an estimate of creatinine clearance, urinalysis, and a urine pregnancy test (UPT) for women of childbearing potential. The Beck Depression Inventory (BDI) will be administered to rule out severe depression. Also at screening, measurements of walking speed (T25FW), manual dexterity (Box and Block), hand strength (grip and pinch tests), motor function in upper and lower extremities (FMA), upper limb motor spasticity (DAS), and assistance required to perform activities of daily living (FIM) will be administered. The screening period (day -14 to day -1) will be completed at Visit 2, which marks the beginning of Period 1 for qualified subjects.

At Visit 2 (day 1), subjects will be randomized in a 2:1 ratio to one of two blinded treatment sequences: placebo followed by dalfampridine-ER (Sequence A) or dalfampridine-ER followed by placebo (Sequence B). The same clinical assessments administered in the previous visit will be performed. Subjects will be discharged home with a one-week supply of their assigned investigational treatment. They will be instructed to take their first dose that evening and one dose approximately every 12 hours until the morning of their next visit. They will have two additional visits at one week intervals during this period: Visit 3 (day 8) and Visit 4 (day 15). At each of these visits, a similar battery of clinical evaluations will be performed, adding a subject global impression (SGI) and clinician global impression (CGI). See Table 23 for a detailed schedule of assessments at each visit. A new supply of investigational product will be dispensed at the completion of study procedures at Visit 3.

At the completion of study procedures at Visit 4, which is the end of Period 1, a one-week washout period will begin during which all subjects will take placebo. Subjects will be discharged home with a supply single-blind placebo and instructed to take their first dose in the evening and one dose approximately every 12 hours until the morning of their next visit.

Visit 5 (day 22) marks the beginning of Period 2 of the study for both groups. During this visit, subjects will have a new set of clinical assessments, after which they will start their crossover treatment according to the sequence established per each group. A similar set of evaluations will be performed at two additional weekly visits during this period: Visit 6 (day 29) and Visit 7 (day 36). Investigational product will be dispensed at the completion of study procedures at each visit, except at Visit 7, the end of Period 2. Dosing instructions will be the same as at previous visits.

A follow-up telephone visit, Visit 8 (day 43), will occur a week later to evaluate adverse events. Participation in the study will be completed after the follow-up call.

Subjects will be instructed to have a dosing schedule targeted towards taking a morning dose of investigational product 2 hours prior to the start of scheduled study assessments during the treatment evaluation visits (Visits 3, 4, 6 and 7), to correspond to approximate peak plasma concentration of dalfampridine-ER.

To monitor treatment compliance, blood samples will be obtained for determination of plasma study drug concentration at all clinic visits after the screening visit.

The study design is displayed graphically in Figure 10. The by-visit schedule of study procedures is presented in Table 23 and descriptions of the procedures can be found below.

**Table 23: Schedule of Assessments**

| | | **Period 1** | | | **Washou t** | **Period 2** | | | |
|---|---|---|---|---|---|---|---|---|---|
| **Procedure** | **Screenin g** | **Placebo or Dalfampridine-ER** | | | **Placebo** | **Placebo or Dalfampridine-ER** | | | **Follow -up call** |
| | **Visit 1** | **Visit 2** | **Visit 3** | **Visit 4** | | **Visit 5** | **Visit 6** | **Visit 7** | **Visit 8** |
| | **Day -14** | **Day 1¹** | **Day 8±1** | **Day 15±1** | | **Day 22¹±1** | **Day 29± 1** | **Day 36± 1** | **Day 43±1** |
| Written Informed Consent | X | | | | | | | | |
| Inclusion/Exclusion criteria | X | | | | | | | | |
| Medical History | X | | | | | | | | |
| Review of prior imaging findings (MRI/CAT scan) to confirm ischemic stroke/ | X | | | | | | | | |
| Physical Examination including vital signs² | X | X | X | X | | X | X | X | |
| Concomitant Meds/Therapy | X | X | X | X | | X | X | X | |
| SMA-12³ | X | | | X | | | | X | |
| Urinalysis^{3,4} | X | | | | | | | | |
| Urine Pregnancy Test³ | X | X | | | | | | | |
| Randomize | | X | | | | | | | |
| SGI | | | X | X | | X | X | X | |
| T25FW | X | X | X | X | | X | X | X | |
| Box and Block⁵ | X | X | X | X | | X | X | X | |
| Grip and pinch tests⁶ | X | X | X | X | | X | X | X | |
| FMA | X | X | X | X | | X | X | X | |
| DAS | X | X | X | X | | X | X | X | |
| FIM | X | X | X | X | | X | X | X | |
| CGI | | | X | X | | X | X | X | |
| BDI | X | X | | X | | X | | X | |
| Plasma dalfampridine concentration³ | | X | X | X | | X | X | X | |
| AE review | X | X | X | X | | X | X | X | X |
| Dispense investigational product⁷ | | X | X | X | | X | X | | |
| Drug accountability | | | X | X | | X | X | X | |
| Final status assessment⁸ | | | | | | | | | X |

| | | | | | | | | | |
|---|---|---|---|---|---|---|---|---|---|
| ¹All measurements will be done prior to start of first treatment on Day 1 and start of crossover treatment on Day 22 ²Full physical, height and weight at screening only; brief physical at subsequent visits. ³A11 sampling is to be done after functional clinical assessments have been performed ⁴If result is positive, confirmatory culture is needed ⁵Test each hand, the dominant hand first ⁶Three trials for each hand of the grip test, tip pinch, key pinch and palmar tests ⁷Dispense a one-week supply of investigational product and instruct subject to take one dose approximately every 12 hours at home, beginning in the evening of the visit ⁸A telephone follow- up visit to evaluate for adverse events | | | | | | | | | |

### 6.16.4 SELECTION AND WITHDRAWAL OF SUBJECTS

### (a) Inclusion Criteria

Subjects may be included in the study if they meet all the following criteria:
1. History of a stable sensorimotor deficit due to an ischemic stroke, as confirmed by the Evaluator with supportive prior imaging findings (MRI/CT scan)
2. ≥ 6 months post-stroke
3. Men or women aged 18 to 85 years inclusive
4. Have a body mass index (BMI) ranging between 18.0 - 35 kg/m², inclusive
5. No previous use of Ampyra, dalfampridine, fampridine or 4-aminopyridine (4AP)
6. Have sufficient ambulatory ability to complete T25FW at Screening Visit and every other visit as required
7. Lower extremity motor Fugl-Meyer score of ≤27
8. Ability to perform all the required study procedures
9. Adequate cognitive ability to provide informed consent, as determined by the Evaluator.
10. Stable concomitant medication therapy regimen within 4 weeks of screening visit.

### (b) Exclusion Criteria

Subjects who meet any of the following exclusion criteria are not eligible for participation in the study:
1. Sexually active woman of childbearing potential who is not surgically sterile, less than two years postmenopausal, or not using an effective birth control method
2. Pregnant or breastfeeding
3. History of seizures, except simple febrile seizures
4. Moderate or severe renal impairment as defined by a calculated creatinine clearance of ≤ 50 mL/minute using the Cockcroft-Gault Equation
5. Evidence of an active urinary tract infection (UTI) at the Screening Visit or within the 4 weeks prior to the Screening Visit
6. Initiation of a prescription medication regimen or therapy within four weeks prior to the Screening Visit, and/or concomitant medication regimen or concomitant therapy is expected to change during the course of the study
7. Initiation of baclofen or tizanidine within four weeks prior to the Screening Visit or any change in dosing regimen within four weeks prior to the Screening Visit
8. Initiation of serotonin reuptake inhibitors (SSRIs) within 3 months prior to the Screening Visit, or any change in dosing regimen within 3 months prior to the Screening Visit
9. Botulinum toxin use within two months prior to the Screening Visit
10. History of drug or alcohol abuse within the past year
11. Orthopedic surgical procedures in any of the extremities within the past 6 months
12. Subject has an abnormal laboratory value that, in the Evaluator's judgment, is both, clinically significant and has the potential to affect the subject's ability to safely complete the study
13. Unstable angina, uncontrolled hypertension or any other significant cardiovascular abnormality as deemed by the Evaluator
14. Severe depression as indicated by a score of ≥30 on the Beck Depression Inventory (BDI)
15. Any other medical condition, per Evaluator's judgment, that would interfere with conduct of study or interpretation of study result
16. Participation in an investigational interventional trial within four weeks prior to Screening Visit
17. Diagnosis of multiple sclerosis

### (c) Subject Withdrawal Criteria

The withdrawal criteria, which are optional, include one or more of the following reasons:
- Subject experiences an adverse event (such as a seizure)
- Pregnancy
- Subject is non-compliant with the protocol
- Subject is lost to follow-up
- Subject abuses alcohol or drugs or no longer meets another eligibility criterion

### 6.16.5 TREATMENT OF SUBJECTS

### (a) Treatments to be Administered

Each subject will receive 28 doses of (A) dalfampridine-ER 10 mg, and 42 doses of (B) placebo (including 14 doses during the placebo washout period). The tablets will be taken at home, with water. The order of treatment will be determined as described in Section (b), below.

Investigational product will be dispensed to the subject at Visits 2, 3, 4, 5 and 6, after assessments have been completed. Subjects will be instructed to take the first dose in the evening of the visit, and the next dose the following morning, approximately 12 hours later. Subjects will be instructed to continue dosing every 12 hours at times that are as consistent as possible. Subjects will be told that they must not make up for missed doses.

The last dose from each dispensed supply will be a dose taken in the morning of the next scheduled visit. Subjects will be instructed to have a dosing schedule targeted towards taking the morning dose of investigational product 2 hours prior to the start of scheduled study assessments during the treatment evaluation visits: Visits 3, 4, 6 and 7.

### (b) Method of Assigning Subject to Treatment Group

Subjects will be randomized at Visit 1 to one of two blinded treatment sequences (A or B) in a 2:1 ratio respectively, according to a randomization created prior to the start of the study:
A: placebo then dalfampridine-ER
B: dalfampridine-ER then placebo

### (c) Blinding

Drug administration will be double-blind, meaning that the treatment sequence is not known to the subject or the study site personnel.

The washout period will be single-blind, meaning that the study site personnel, but not the subject, will know that placebo is being administered during this period.

### (d) Treatment Compliance

Subjects will be encouraged to take all doses as prescribed. Treatment compliance will be monitored through inventory of tablet count in returned bottles, and by obtaining blood samples for determination of plasma dalfampridine concentration during each treatment period. Any reasons for non-compliance will be documented.

### (e) Prior and Concomitant Medications

The following medications are excluded for the duration of the study, and also for some period of time prior to the study, for the purpose of maintaining stable symptoms:
- Baclofen or tizanidine initiated or dosing modified less than four weeks before the Screening Visit
- SSRI initiated or dosing modified less than 3 months before the Screening Visit
- Botulinum toxin administered less than two months before the Screening Visit
- Other prescription medications (or therapies) initiated or changed less than 4 weeks before the Screening Visit

No changes will be made to the concomitant treatment during the study, except as required for the safety of the subject.

### 6.16.6 DESCRIPTION OF INVESTIGATIONAL PRODUCT

*Active:* Commercial drug will be used. AMPYRA (dalfampridine) Extended Release tablets are a white to off-white, biconvex, oval shaped, film-coated, non-scored tablet with flat edge, debossed with "A10" on one side, containing 10 mg of dalfampridine. Inactive ingredients consist of colloidal silicon dioxide, hydroxypropyl methylcellulose, magnesium stearate, microcrystalline cellulose, polyethylene glycol, and titanium dioxide.

*Placebo:* The placebo tablets will be identical in appearance to the AMPYRA tablets and contain the same inactive ingredients.

### 6.16.7 STUDY PROCEDURES

The following sections describe the baseline and clinical functional measurements that will be obtained in this study. A detailed schedule of procedures by study visit is provided below and summarized in Table 23.

Prior to engaging in any study procedure, subjects must provide written informed consent.

### (a) Plasma Dalfampridine Concentration

Blood samples for determination of plasma dalfampridine concentration will be obtained after the completion of all functional clinical assessments. The purpose of these measurements is for assessment of treatment compliance. A minimum of 7 mL of whole blood will be collected into an appropriately labeled heparin tube and kept cold (i.e., on wet ice) until centrifuged. Immediately after collection, the tube will be centrifuged at low speed and approximately 3 mL of plasma will be transferred from each sample into a labeled tube. The plasma will be stored at -20° C until the shipment to the central laboratory is requested. At that time, frozen plasma samples will be collected together and sent in an insulated container, on dry ice, overnight by express carrier to the designated central laboratory.

### (b) Clinical Assessments

### Timed 25 Foot Walk (T25FW)

The T25FW test is a quantitative measure of ambulatory function. The subject is instructed to walk as quickly as he or she can from one end to the other end of a clearly marked, unobstructed, 25-foot course. The T25FW will be performed according to the detailed instructions provided in the Administration and Scoring Manual published by the National Multiple Sclerosis Society (Fischer J, et al., National Multiple Sclerosis Society. 2001; 1-410). The subject will stand with the tip of their shoes on a marked starting line, and timing will begin when any part of the subject's foot crosses the line. Timing will end when any part of the subject's foot crosses the marked finish line. Time will be recorded in seconds and rounded to the nearest tenth of a second using a stopwatch provided for this study. The task is administered again, with a maximum five-minute rest period allowed between the two trials, by having the subject walk back the same distance. If required, the subject may use an appropriate assistive device. The subject must be instructed to maintain his or her normal activities without rehearsal or practice measures to unfairly improve their performance scores between visits. Every effort will be made to use the same testing room and the same designated area for the T25FW at each assessment. Potential for external distractions will be kept to a minimum as much as possible.

Normative data for walking speed are available (Bohannon R., Age and Ageing. 1997; 26: 15-19). For subjects ≥18 years of age and <20 years of age, the normative data for the 20s decade age group will be used.

### Box and Block Test

The Box and Block Test (Mathiowetz V, et al., Am J Occup Ther. 1985; 36(6): 386-391) has been used as a valid and reliable measure of manual dexterity. The subject is instructed to quickly pick up blocks one at a time from one side of a box, transport each block over a partition to the other side of the box, and drop it. The test was originally developed to evaluate the gross manual dexterity of adults with cerebral palsy. Data for normal adults are available (Bohannon R., Age and Ageing. 1997; 26: 15-19). For subjects ≥18 years of age and <20 years of age, the normative data for the 20-24 years age group will be used.

The Box and Block test will be consistently performed before the Pinch and Grip tests to minimize the effects of fatigue. Both dominant and non-dominant hands will be tested, starting with the dominant hand.

### Hand Strength by the Grip and Pinch tests

The Grip Test (Mathiowetz V, et al., Arch Phys Med Rehabil. 1985; 66:69-72) is used as a simple, valid and reliable measure to identify hand strength problems, to detect the change which may result from an occupational therapy program, the course of a disease or injury, or to show the relation of the patient's strength to the general population. Hand strength is measured using a dynamometer.

The Pinch Tests (Mathiowetz V, et al., Arch Phys Med Rehabil. 1985; 66:69-72) are used as a simple, valid and reliable measure to indentify pinch strength problems, to detect the change which may result from an occupational therapy program, the course of a disease or injury, or to show the relation of the patient's strength to the general population. It has three components, the tip, key and palmar pinch. Pinch strength is measured using a pinch gauge.

There will be three trials for each hand of the grip test, tip pinch, key pinch and palmar tests each time they are measured.

Normative data on the Grip and Pinch tests in adults are available (Mathiowetz V, et al., Arch Phys Med Rehabil. 1985; 66:69-72). For subjects ≥18 years of age and <20 years of age, the normative data for the 20-24 years age group will be used.

### Fugl-Meyer Assessment (FMA)

The FMA is a performance-based impairment assessment that was designed to evaluate motor functioning, balance, sensation and joint functioning in patients with post-stroke hemiplegia (Fugl-Meyer AR, et al., Scand J Rehabil Med. 1975; 7(1):13-31). For this study, the domains of upper extremity (UE) motor function, lower extremity (LE) motor function and sensation will be assessed (see Table 24).

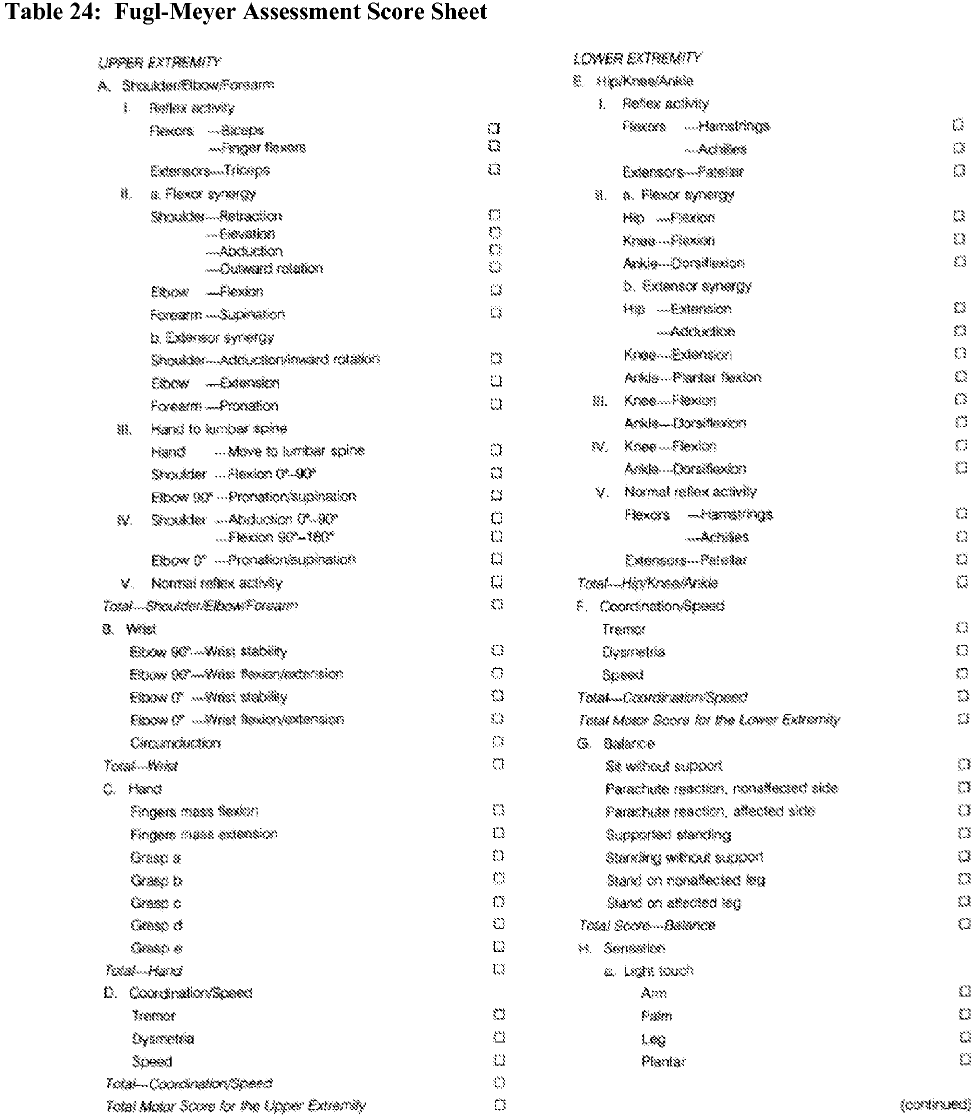

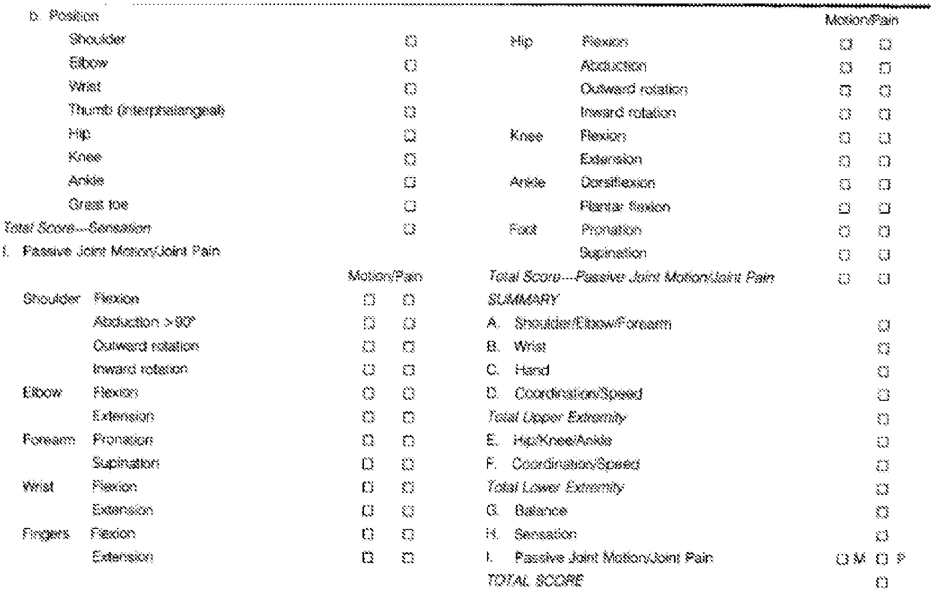

Items are rated on a 3-point scale of severity. A total score can be determined for each domain separately, and the total UE and LE scores can be combined for a total motor score.

### Disability Assessment Scale (DAS)

Optionally, DAS is performed.

The DAS was developed to assess impairment in 4 functional areas commonly affected in patients with post-stroke upper limb spasticity: personal hygiene, dressing, pain, and limb position. The clinician will rate the subject's level of impairment in each of these domains using a 4-point scale ranging from "no disability" to "severe disability." Assessment of the 4 functional domains will performed according to the following guidelines (Brashear A, et al., Arch Phys Med Rehabil. 2002; 83(10):1349-54):

Hygiene: The rater will assess the extent of maceration, ulceration, and/or palmar infection; palm and hand cleanliness; ease of cleanliness; ease of nail trimming; and the degree of interference caused by hygiene-related disability in the patient's daily life.

Dressing: The rater will assess the difficulty or ease with which the patient could put on clothing (e.g., shirts, jackets, gloves) and the degree of interference caused by dressing related disability in the patient's daily life.

Limb position: The rater will assess the amount of abnormal position of the upper limb.

Pain: The rater will assess the intensity of pain or discomfort related to upper-limb spasticity.

Each of the 4 functional domains will be rated using the following scale: **0** = no disability; **1** = mild disability (noticeable but does not interfere significantly with normal activities); **2** = moderate disability (normal activities require increased effort and/or assistance); **3** = severe disability (normal activities limited).

### Functional Independence Measurement (FIM)

The FIM scale is a widely used assessment of disability that measures how much assistance is needed for the individual to conduct activities of daily living (ADL). It is comprised of 18 items: 13 in the physical domain and 5 in the cognitive domain. Ratings are based on the clinician's direct observation, with each item scored on a 7-point scale ranging from "total assistance" to "complete independence." Dimensions assessed are: eating, grooming, bathing, upper body dressing, lower body dressing, toileting, bladder management, bowel management, bed to chair transfer, toilet transfer, shower transfer, locomotion (ambulatory or wheelchair level), stairs, cognitive comprehension, expression, social interaction, problem solving, memory.

Scoring Criteria are as follows (see Rehabilitation Measures Database webpage):

| | |
|---|---|
| **7** | Complete Independence |
| **6** | Modified Independence |
| **5** | Supervision or Setup |
| **4** | Minimal Contact Assistance (patient can perform 75% or more at task) |
| **3** | Moderate Assistance (patient can perform 50% to 74% of task) |
| **2** | Maximal Assistance (patient can perform 25% to 49% of task) |
| **1** | Total assistance |

### Subject Global Impression (SGI)

The SGI is a commonly used measure of treatment response that asks the subject to rate the effects of the investigational drug on his or her physical well-being during the preceding week, using a 7-point scale ranging from "terrible" to "delighted."

The subjects is given a form to complete stating the following: "We want to find out how you feel about the effects of the study medication on your physical well-being. How do you feel about the effects of the study medication over the past 7 days?" The subject is given the following choices for a response: "terrible," "unhappy," "mostly dissatisfied," "neural/mixed," "mostly satisfied," "pleased," and "delighted." The subject is asked to explain the response given in their own words.

### Clinician Global Impression (CGI)

The CGI is a commonly used measure of treatment response that asks the clinician to provide an overall impression of the changes in the subject's neurological status and general state of health following treatment with the investigational product, as compared to the subject's condition at baseline (and not compared to the preceding visit). The CGI is rated according to a 7-point scale ranging from "very much improved" to "very much worse."

The clinician is given a form to complete stating the following: "Overall, taking into account the subject's symptoms and other neurological functions, how would you rate the subject's neurological status today, relative to their Screening Visit? Please consider neurological changes only, without respect to other factors." The clinician is given the following choices for a response: "very much improved," "much improved," "somewhat improved," "no change," "somewhat worse," "much worse," and "very much worse." The clinician is asked to explain any indication of change, if possible.

### Beck Depression Inventory (BDI)

The BDI (see Table 25) is a widely used self-report depression questionnaire measuring the severity of depression symptoms. Each of 21 items is rated on a 4-point scale ranging from minimal to severe. Subjects with severe depression as indicated by a score of ≥30 at screening will be excluded from participating in the study. The BDI will also be administered at other visits as one of the clinical assessments.

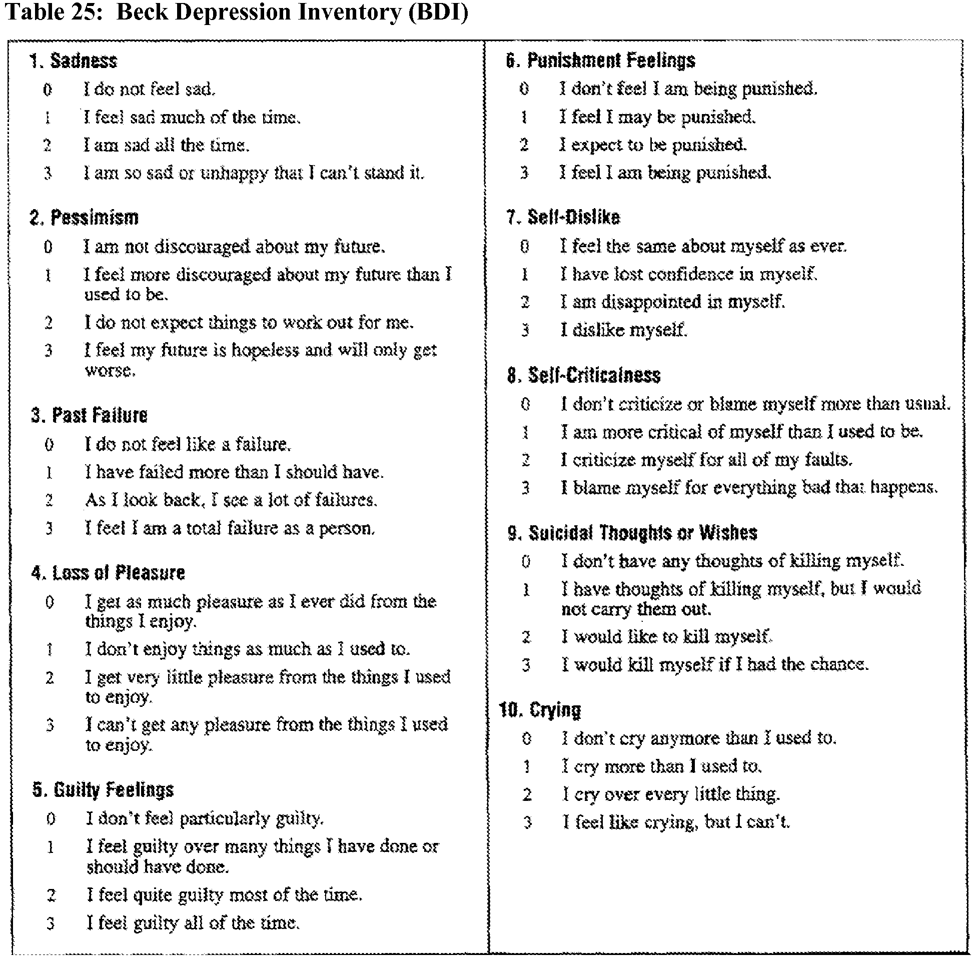

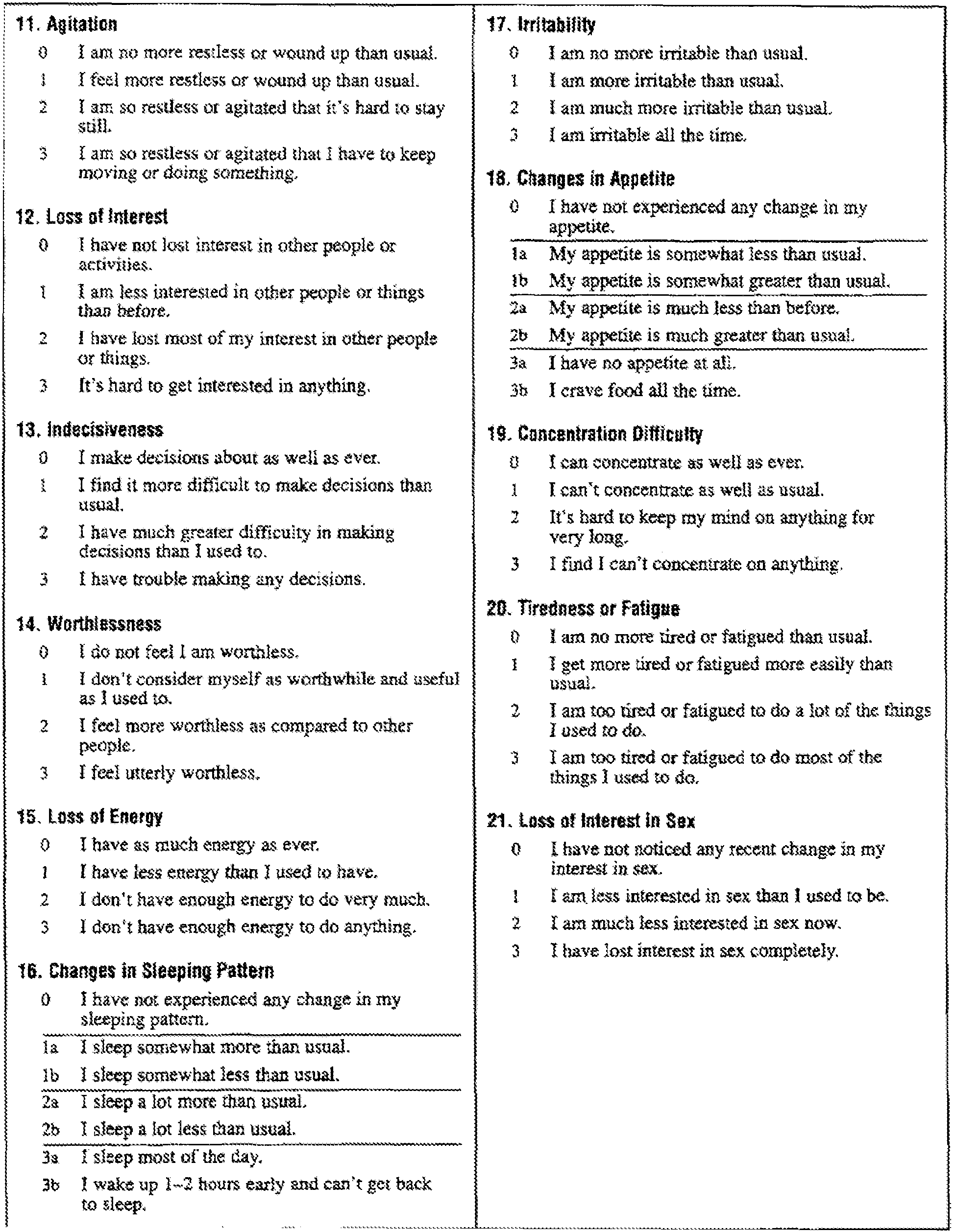

### (c) Study Sequence

The following sections describe the assessments to be performed at each clinic visit during the study.

### Visit 1, day -14 to day -1 (Screening Visit)

The Evaluator will assess eligibility for the study after the following procedures have been performed. These procedures will be completed within 14 days prior to the randomization visit. They will be performed in the order outlined below.
- Obtain signed informed consent
- Complete medical history, including demographic information
- Review prior and concomitant medications
- Subject to complete the BDI
- Complete a full physical examination
- Perform routine sitting vital sign measurements, including height and weight.
- Calculate BMI
- Administer the T25FW
- Administer the Box and Block test (dominant and non-dominant hand, the dominant first)
- Administer the grip test, tip pinch, key pinch and palmar pinch tests. These tests will be administered three times with each hand.
- Administer the FMA, DAS, and FIM
- Take blood and urine samples for laboratory evaluations (SMA-12, calculated creatinine clearance, urinalysis, and urine pregnancy test for women of childbearing potential)
- Review adverse events
- If subject qualifies, schedule subject to return to the investigational center within 14 days.

### Visit 2, day 1 (randomization visit, start of Period 1)

The following assessments and procedures will be performed in the order outlined below:
- Complete a brief physical examination
- Perform sitting vital sign measurements
- Administer the T25FW
- Administer the Box and Block test (dominant and non-dominant hand, the dominant first)
- Administer the grip test, tip pinch, key pinch and palmar pinch tests. These tests will be administered three times with each hand.
- Administer the FMA, DAS, and FIM
- Subject to complete the BDI
- Obtain blood sample for plasma dalfampridine concentration
- Urine pregnancy test for women of childbearing potential
- Review adverse events and concomitant medications
- Randomize to one of two treatment sequences
- Dispense a one-week supply of assigned double-blind investigational product with instructions to take the first dose that evening. See Section 6.16.5(a) for further instructions to the subject on dosing regimen
- Discharge subject and schedule a date and time for the next visit to occur in one week (± 1 day).

### Visit 3, day 8

The following assessments and procedures will be performed in the order outlined below:
- Complete brief physical examination
- Perform sitting vital sign measurements
- Subject to complete the SGI
- Administer the T25FW
- Administer the Box and Block test (dominant and non-dominant hand, the dominant first)
- Administer the grip test, tip pinch, key pinch and palmar pinch tests. These tests will be administered three times with each hand.
- Administer the FMA, DAS, and FIM
- Complete the CGI
- Obtain blood sample for plasma dalfampridine concentration
- Review adverse events and concomitant medications
- Collect investigational product from last visit, and perform drug accountability
- Dispense a new one-week supply of assigned double-blind investigational product with instructions to take dose that evening, approximately 12 hours post last dose. See Section 6.16.5(a) for further instructions to the subject on dosing regimen
- Discharge subject and schedule a date and time for the next visit to occur in one week (± 1 day)

### Visit 4, day 15 (end of Period 1, start of washout)

The following assessments and procedures will be performed in the order outlined below:
- Complete brief physical examination
- Perform sitting vital sign measurements
- Subject to complete the SGI
- Administer the T25FW
- Administer the Box and Block test (dominant and non-dominant hand, the dominant first)
- Administer the grip test, tip pinch, key pinch and palmar pinch tests. These tests will be administered three times with each hand.
- Administer the FMA, DAS, and FIM
- Complete the CGI
- Subject to complete the BDI
- Obtain blood sample for plasma dalfampridine concentration and SMA-12
- Review adverse events and concomitant medications
- Collect investigational product from last visit, and perform drug accountability
- Dispense a one-week supply of single-blind placebo with instructions to take first dose that evening. See Section 6.16.5(a) for further instructions to the subject on dosing regimen
- Discharge subject and schedule a date and time for the next visit to occur in one week (± 1 day)

### Visit 5, day 22 (end of washout, start of Period 2)

The following assessments and procedures will be performed in the order outlined below:
- Complete brief physical examination
- Perform sitting vital sign measurements
- Subject to complete the SGI
- Administer the T25FW
- Administer the Box and Block test (dominant and non-dominant hand, the dominant first)
- Administer the grip test, tip pinch, key pinch and palmar pinch tests. These tests will be administered three times with each hand.
- Administer the FMA, DAS, and FIM
- Complete the CGI
- Subject to complete the BDI
- Obtain blood sample for plasma dalfampridine concentration
- Review adverse events and concomitant medications
- Collect investigational product from last visit, and perform drug accountability
- Dispense a one-week supply of crossover treatment with instructions to take the first dose that evening. See Section 6.16.5(a) for further instructions to the subject on dosing regimen
- Discharge subject and schedule a date and time for the next visit to occur in one week (± 1 day)

### Visit 6, day 29

The following assessments and procedures will be performed in the order outlined below:
- Complete brief physical examination
- Perform sitting vital sign measurements
- Subject to complete the SGI
- Administer the T25FW
- Administer the Box and Block test (dominant and non-dominant hand, the dominant first)
- Administer the grip test, tip pinch, key pinch and palmar pinch tests. These tests will be administered three times with each hand.
- Administer the FMA, DAS, and FIM
- Complete the CGI Obtain blood sample for plasma dalfampridine concentration
- Review adverse events and concomitant medications
- Collect investigational product from last visit, and perform drug accountability
- Dispense a new one-week supply of crossover treatment with instructions to take dose that evening, approximately 12 hours post last dose. See Section 6.16.5(a) for further instructions to the subject on dosing regimen
- Discharge subject and schedule a date and time for the next visit to occur in one week (± 1 day)

### Visit 7, day 36 (end of Period 2)

The following assessments and procedures will be performed in the order outlined below:
- Complete brief physical examination
- Perform sitting vital sign measurements
- Subject to complete the SGI
- Administer the T25FW
- Administer the Box and Block test (dominant and non-dominant hand, the dominant first)
- Administer the grip test, tip pinch, key pinch and palmar pinch tests. These tests will be administered three times with each hand.
- Administer the FMA, DAS, and FIM
- Complete the CGI
- Subject to complete the BDI
- Obtain blood sample for plasma dalfampridine concentration and SMA-12
- Review adverse events and concomitant medications
- Collect investigational product from last visit, and perform drug accountability. No investigational product to be dispensed.
- Discharge subject and schedule a date and time for the next visit, a telephone visit, to occur in one week (± 1 day)

### Telephone Follow-up, day 43 ± 1

The site will make a follow-up telephone call to review any adverse events or changes in medication. The follow-up call may be performed up to 2 days before or after day 43 to account for the weekends/holidays.
- Final status assessment

### 6.16.8 STATISTICS

### (a) Statistical Power

Sixty-six (66) subjects will be randomized in a 2:1 ratio to one of two treatment sequences: placebo followed by dalfampridine-ER (Sequence A) or dalfampridine-ER followed by placebo (Sequence B). This sample size will provide adequate planning estimates to aid in the design of future studies.

### (b) Derived Endpoints and Data Handling

Baseline for analyses will be defined as the last non-missing assessment prior to the first dose of double-blind medication.

Age and time since ischemic stroke will be computed based on the date at informed consent:

Age will be calculated as: Age = [Date of informed consent - Date of birth] / 365.25 rounded down to the previous integer.

Days since ischemic stroke will be calculated: Days = Date of informed consent - Date of stroke.

The derivation of baseline measures will follow the derivations outlined below in the section entitled "Derived Variables and Data Handling."

### (c) Analysis of Functional Assessments

The computational details for the derivation of all applicable variables in this section can be found below in the section entitled "Derived Variables and Data Handling."

### Functional Assessments

The functional assessments that will be explored in this study are:
- Walking speed as measured by the Timed 25 Foot Walk test (T25FW)
- Manual dexterity as measured by the Box and Block Test
- Hand strength as measured by the grip test and pinch tests
- Global motor function score on the Fugl-Meyer Assessment (FMA) and the individual motor scores:
   - Upper extremity function
   - Lower extremity function
- Upper limb spasticity as measured by the Disability Assessment Scale (DAS)
- Assistance required to perform activities of daily living (ADL) by the Functional Independence Measure (FIM) scale
- Subject Global Impression (SGI) scale
- Clinician Global Impression (CGI) scale
- Depression as measured by the Beck Depression Inventory (BDI)

### Derived Variables and Data Handling

Baseline for the analyses will be defined as the last non-missing assessment prior to the first dose of double-blind medication.

### Walking Speed

At each visit, there will be two trials of the T25FW test. Walking speed for an individual trial will be derived (in feet per second) by multiplying the reciprocal of the time to complete the walk (in seconds) by 25 (feet). The walking speed for a particular study visit will be derived by calculating the average of the walking speeds for Trial 1 and Trial 2 from that study visit. If either trial is missed, then the walking speed for that visit will be the walking speed from the non-missing trial.

### Grip and Pinch Tests

At each visit, there will be three trials for each of the grip and pinch tests. The response for a particular study visit is the average of the three trials for that particular test. The grip test and the pinch tests will be summarized by dominant hand and non-dominant hand separately.

### Box and Block Test

The response for the Box and Block Test is the number of blocks transported to the other side of the partition in 60 seconds. There is no derivation needed for the response variable. The Box and Block Test will be summarized by dominant hand and non-dominant hand separately.

### Fugl-Meyer Assessment (FMA)

The FMA is a measure of upper extremity and lower extremity motor and sensory impairment, consisting of 155 items in 5 domains. Subscores can be determined for each domain by summing the scores on the associated individual items. A global FMA score can be created by summing the individual domain subscores. The FMA global score and the individual domain subscores will be summarized separately.

### Disability Assessment Scale (DAS)

The DAS is used to assess impairment in 4 functional areas commonly affected in patients with post-stroke upper limb spasticity. The four functional areas will be analyzed separately. There is no derivation needed for the response variable.

### Functional Independence Measure (FIM) scale

The FIM scale is an assessment of physical and cognitive disability comprised of 18 items. For each visit, the response on the FIM scale is the sum of the individual responses to the 18 items. The total score can range from 18 (lowest level of function) to 126 (highest level of function).

### Subject Global Impression (SGI) scale

For each visit, the response on the SGI is the subject's rating of the investigational product on his or her physical well-being during the preceding week. There is no derivation needed for the response variable.

### Clinician Global Impression (CGI) scale

For each visit, the response on the CGI is the clinician's overall impression of the changes in the subject's neurological status and general state of health following treatment with the investigational product, as compared to baseline. There is no derivation needed for the response variable.

### Beck Depression Inventory (BDI)

The BDI is a self-reported 21-item depression questionnaire measuring the severity of depression symptoms. For each visit, the response on the BDI is the sum of the individual responses from the 21 items. The total score can range from 0 to 63.

### Statistical Methods

Analyses will be performed to determine the effect of dalfampridine-ER on the functional assessments. For each of the functional assessments, except the SGI and CGI, the intra-subject change from baseline will be calculated within each treatment period:
Period 1: Visit 4 assessment - Visit 2 assessment
Period 2: Visit 7 assessment - Visit 5 assessment

For SGI and CGI, the Visit 4 assessment and the Visit 7 assessment will be used in the analyses.

For any functional assessment, if the Visit 4 assessment is missing, it will be imputed using the Visit 3 assessment. If the Visit 7 assessment is missing, it will be imputed using the Visit 6 assessment.

For all clinical measures except SGI and CGI, the following two types of analyses will be performed. The first type will be based on the difference in intra-subject changes from baseline for Period 1 (placebo) versus Period 2 (dalfampridine-ER) using the 44 subjects randomized to Sequence A. The changes from baseline between the two treatments will be compared using a paired t-test. The second type of analysis will be based on between-treatment group comparisons of the changes from baseline within Period 1 only. The changes from baseline between the two treatments will be compared using a two-sample t-test.

For SGI and CGI, two types of analyses will be performed as well. However, the analyses will be performed using the results of the SGI and CGI at Visit 4 and Visit 7, and not on the changes from baseline. The statistical methods described above will also be used in the analysis of SGI and CGI.

### 6.16.9 REFERENCES FOR EXAMPLE 16

- Gubitz G. Acute stroke management and prevention of recurrences. In Evidence-Based Neurology: Management of neurological diseases. Blackwell Publishing, Malden (MA), 2007: pp 113-126.
- Demaerschalk, B, Hwang HM, Leung G. US cost burden of ischemic stroke: A systematic review. Am J Manag Care. 2010;16(7):525-33.
- American Heart Association. Heart Disease and Stroke Statistics-2008 Update. Dallas, TX: American Heart Association;2008:19.
- Lloyd-Jones D, Adams R, Carnethon M, et al. Heart Disease and Stroke Statistics 2009 Update: A Report from the American Heart Association Statistics Committee and Stroke Statistics Subcommittee. Circulation 209;119:480-486.
- Ovbiagele B, Lyden P, Saver J, et al. Disability status at one month is a reliable proxy for final ischemic stroke outcome. Neurology 2010;75:688-692.
- Carod-Artal J, Egido JA, Gonzalez JL, et al. Quality of Life among Stroke survivors evaluated 1 year after Stroke. Stroke. 2000;31:2995-3000.
- Arene N, Hidler J. Understanding motor impairment in the paretic lower limb after Stroke: A review of the literature. Top Stroke Rehabil. 2009 Sept-Oct;16(5):346-356.
- Dimyan M, Cohen L. Neuroplasticity in the context of motor rehabilitation after stroke. Nat Rev Neurol. 2011;7:76-85.
- Forrester L, Wheaton L, Luft A. Exercise-mediated locomotor recovery and lower-limb neuroplasticity after stroke. J Rehab Res Dev. 2008;45(2):205-220.
- Adams HP, Bendixen BH, Kapelle LJ, et al. Classification of subtype of acute ischemic stroke. Definitions for use in a multicenter clinical trial. TOAST. Trial of Org 10171 in acute stroke treatment. Stroke 1993;24:35-41.
- Adams HP, Del Zoppo G, Alberts M, et al. Guidelines for the early management of adults with ischemic stroke: A guideline from the American Heart Association/American Sroke Association Stroke Council, Clinical Cardiology Council, Cardiovascular Radiology and Intervention Council, and the Atherosclerotic Peripheral Vascular Disease and Quality of Care Outcomes in research Interdisciplinary Working Groups. Stroke 2007;38:1655-1711.
- Wechsler L. Imaging Evaluation of Acute Ischemic Stroke. Stroke 2011;42(suppl 1):S12-S15.
- Taylor TN, Davis PH, Torner JC, et al. Lifetime cost of stroke in the United States. Stroke 1996;27(9):1459-1466.
- Goodman AD, Brown TR, Cohen JA, et al. Dose comparison trial of sustained-release fampridine in multiple sclerosis. Neurology. 2008;71:1134-1141.
- Goodman AD, Brown TR, Krupp LB, et al. Sustained-release oral fampridine in multiple sclerosis: a randomized, double-blind, controlled trial. Lancet. 2009;373:732-38.
- Goodman AD, Brown TR, Edwards KR, et al. A Phase 2 trial of extended release oral dalfmapridine in multiple sclerosis. Ann Neurol. 2010;373:494-50.
- Menon B, Shorvon S. Ischaemic stroke in adults and epilepsy. Epilepsy Res. 2009;87:1-11.
- Labovitz D, Hauser A, Sacco R. Prevalence and predictors of early seizure and status epilepticus after first stroke. Neurology. 2001;57:200.
- Spozhmy P, Neiman E, Andriola M, et al. A practical review and approach to poststroke seizures. Rev Neurol Dis. 2011;8(1/2):10-15.
- Bladin C, Alexandrov A, Bellavance A, et al. Seizures after stroke: A prospective multicenter study. Arch Neurol. 2000;57:1617-1622.
- Szaflarski J, Rackley A, Kleindorfer D, et al. Incidence of seizures in the acute phase of stroke: A population-based study. Epilepsia. 2008;49(6):974-981.
- Krakow K, Sitzer M, Rosenow F, et al. Predictors of acute post-stroke seizures. Cerebrovasc Dis. 2010;30:584-589.
- Wei L, Yu SP, Gottron F, at al. Potassium channel blockers attenuate hypoxia and ischemia-induced neuronal death in vitro and in vivo. Stroke 2003;34:1281-1286.
- Huang H, Gao T, Gong L, et al. Potassium channel blocker TEA prevents CA1 hippocampal injury following transient forebrain ischemia in adult rats. Neurosci Lett. 2001;83-86.
- Bains J, Follwell M, Latchford K, at al. Slowly inactivating potassium conductance (ID): A potential target for stroke therapy. Stroke 2001;32:2624-2634.
- Fischer J, et al. The Multiple Sclerosis Functional Composite Administration and Scoring Manual. National Multiple Sclerosis Society. 2001; 1-41.
- Bohannon R. Comfortable and maximum walking speed of adults aged 20-79 years: reference values and determinants. Age and Ageing. 1997; 26: 15-19.
- Mathiowetz V, Volland G, Kashman N , et al. Adult norms for the Box and Block Test of manual dexterity. Am J Occup Ther. 1985; 36(6): 386-391.
- Mathiowetz V, Kashman N, Volland G, et al. Grip and pinch strength; normative data for adults. Arch Phys Med Rehabil. 1985; 66:69-72.
- Fugl-Meyer AR, Jääskö L, Leyman I, Olsson S, Steglind S. The post-stroke hemiplegic patient: a method for evaluation of physical performance. Scand J Rehabil Med. 1975; 7(1):13-31.
- Code of Federal Regulations, Title 21 Food and Drugs. In: Selected Regulations and Guidance for Drug Studies. Philadelphia, PA: Clinical Research Resources; Rev. April 1,2006.
- World Medical Association. Declaration of Helsinski: Ethical Principles for Medical Research Involving Human Subjects. Helsinki, Finland, June 1964.
- Brashear A, Zafonte R, Corcoran M, et al. Inter-and intrarater reliability of the Ashworth Scale and the Disability Assessment Scale in patients with upper-limb poststroke spasticity. Arch Phys Med Rehabil. 2002; 83(10): 1349-54.

### 6.17 Example 17: Effects of Oral Administration of 4-AP: Functional Recovery Following MCA occlusion (MCAO) in Rats. Blinded, vehicle-controlled dose escalation study.

4-AP was evaluated for its ability to promote functional sensorimotor improvement following ischemic stroke in rats with stable motor deficits at times remote from their ischemic events. The animal model of ischemic stroke, i.e., the MCAO model, used in this example is the same as the animal model described in Example 2.

In the MCAO model recovery begins to plateau by 4 weeks after MCAO, at which time there are still measurable deficits in sensorimotor function. However, 4 weeks after MCAO there may still be slow continued endogenous recovery. For these reasons, treatment in this example was initiated on Day 56 after MCAO, at a time point even more remote from the initial ischemic event to allow the animals to reach a more stable level of sensorimotor deficits after endogenous recovery.

### Experimental Design

In this experiment, Sprague Dawley rats were subjected to a surgery resulting in middle cerebral artery occlusion (MCAO), treated with vehicle (water) or 4-aminopyridine as described below, and subjected to behavioral assessments as described below.

Animals: 30 male Sprague Dawley Rats, 300-400 g (obtained from Charles River Laboratories, which arrived 7-10 days before surgery at 250-275 g) were used. Animals were randomly assigned to treatment groups.

Nomenclature: The nomenclature for the days of the study is as follows: Day 0 is the day of the MCAO, and the days following are numbered consecutively (Day 1, Day 2, Day 3, etc.); Day -1 represents the day prior to the MCAO.

Grouping details: The amount of time needed for some procedures in this study necessitated breaking up the 2 treatment groups (see Table 26 below), into 4 working groups. Six animals received stroke surgery per day. If an animal died during the 8-day surgical period of the study, it was replaced by a spare. If not, the animal was not replaced. Most animal deaths (<5% overall) occurred in the immediate post-op to 7 day period.

Anesthesia: Anesthesia was performed as described in Example 2, above.

Temperature: 37.0 ± 1 °C.

Surgical Procedure: Surgical procedure was performed as described in Example 2, above.

Post-Operative Monitoring: Post-Operative Monitoring was performed as described in Example 2, above.

Handling, surgery, and injections timetable: Handling, surgery, and injections timetable was the same as that described in Example 2, above.

Treatment and dosing: Rats were treated in accordance with the treatment schedule shown in Table 26. Dosing is shown in Table 27. 4-aminopyridine was dissolved in water for injection (WFI, Cellgro) and sterile filtered. Solutions of 0.25 mg/mL, 0.5 mg/mL and 1.0 mg/mL of 4-aminopyridine were delivered by gastric gavage at 2mL/kg for final doses of 0.5 mg/kg, 1 mg/kg or 2.0 mg/kg respectively. Vehicle control treatment was WFI delivered at 2 mL/kg by gastric gavage. Starting on Day 56 after MCAO, animals received gastric gavage of solutions (2 mL/kg) approximately 12 hours apart. The vehicle control group was treated with water for all doses on Days 56-65. For the treated group, six doses of 4-aminopyridine at 0.5 mg/kg were delivered over Days 56-59, followed by six doses at 1.0 mg/kg over Days 59-62 and six doses at 2.0 mg/kg over Days 62-65. Animals in all groups were not treated during Days 66-70. p.o.=par oral.

**Table 26:**

| | Endogenous recovery phase - no treatment (with behavior tests on Day -1, 1, 7, 14, 21, 28, 35, 42, 49, 56) | Treatment Day 56 (evening)-59 (morning) (behavior tests on Day 59). Par oral b.i.d. | Treatment Day 59 (evening)-62 (morning) (behavior tests on Day 62). Par oral b.i.d. | Treatment Day 62 (evening)-65 (morning) (behavior tests on Day 65 and 70). Par oral b.i.d. |
|---|---|---|---|---|
| Group 1 (n=15) | Days 1-56 | Vehicle (water) | Vehicle (water) | Vehicle (water) |
| Group 2 (n=15) | Days 1-56 | 4-AP Low Dose | 4-AP Medium Dose | 4-AP High Dose |

**Table 27:**

| **Treatment ID** | **Treatment** |
|---|---|
| V | Vehicle (water) |
| Low dose | 0.5 mg/kg, b.i.d, p.o. of 4-AP |
| Medium dose | 1 mg/kg, b.i.d., p.o. of 4-AP |
| High dose | 2.0 mg/kg, b.i.d, p.o. of 4-AP |

Treatment groups: Animals had MCAO surgery and were allowed to recover for 56 days. Animals were then randomized into 2 groups based on their baseline behavior. Par oral dosing b.i.d. was initiated in the evening of Day 56 after MCAO. Behavior testing during dosing periods was started 1 hour after dosing. Blood was collected via saphenous vein just prior to and during treatment phases just after behavioral testing (90 minutes post dosing). All dosing was via gastric gavage, volume not to exceed 2 mL/kg.

Blood sampling: 300 microliter blood sample was collected from the saphenous vein of each animal on Day 56 just prior to the first dose and then exactly 90 min after the 6^{th} dose at each dose level. Blood was collected, centrifuged, stored and analyzed as described in Example 2, above.

Behavioral test details: Behavioral evaluations were done by evaluators blinded to treatment assignment. Blinded assessments of sensorimotor function were performed just prior to MCAO surgery, 24 hours after MCAO surgery and weekly thereafter until the first phase of dosing using limb placing and body swing behavioral tests. As described above, behavioral assessments were timed exactly with dosing times. Animals were given the first dose, behavioral assessments were performed starting 60 minutes later. Animals were tested one hour after the 6^{th} dose of each dose level (on Days 59, 62 and 65) and at the end of the 5 day washout on Day 70.

Limb Placing: Evaluated at Day -1 (pre-operation), Day 1, Day 7, Day 14, Day 21, Day 28, Day 35, Day 42, Day 49, Day 56, Day 59, Day 62, Day 65, Day 70. The limb placing tests were divided into forelimb and hindlimb tests. The forelimb and hindlimb placing tests and the scoring for these tests are described in Example 2, above.

Body Swing: Evaluated at Day -1 (pre-operation), Day 1, Day 7, Day 14, Day 21, Day 28, Day 35, Day 42, Day 49, Day 56, Day 59, Day 62, Day 65, Day 70. The body swing test and the scoring for this test is described in Example 2, above.

Euthanasia and post-mortem processing: At day 70 after MCAO, rats were anesthetized as described in Example 2, above.

Infarct measurement: Infarct measurement was performed as described in Example 2, above.

Statistical Methods: Changes from pre-treatment baseline values (Day 56) were calculated for each behavioral score at each time point assessed after dosing. Mean behavioral parameter data were subject to Analysis of Variance (ANOVA). Infarct volume data were analyzed by ANOVA. All data were expressed as means ± SEM.

Regulatory Compliance: Regulatory Compliance for the study was the same as that described in Example 2, above.

Quality Assurance (QA): QA for the study was the same as that described in Example 2, above.

### Results

Both groups of animals (vehicle and 4-aminopyridine-treated) demonstrated a typical recovery response to the MCAO-induced ischemia with normal scores of 0 just prior to the surgery (Day -1) followed by a complete loss in function (score 12, forelimb; 6, hind limb) within 24 hours after the occlusion (Day 1). During the next 8 week, untreated phase, forelimb and hind limb scores improved to approximately 4.5 and 2.5 (respectively) and approached a plateau level of recovery (see Figures 11 and 12). In particular, after the complete loss of function, animals recovered partially and reached a plateau around Day 30. Animals remained at this level of function through Day 56 when treatment was initiated.

Sensorimotor function was evaluated using forelimb and hindlimb placing and body swing tests. The forelimb placing test shows the effect of the treatment on forelimb function (see Figure 11). The hindlimb placing test shows the effect of the treatment on hindlimb function (see Figure 12). The body swing test shows the effect of the treatment on global body control (see Figure 13).

The vehicle group demonstrated small and statistically insignificant changes in behavior compared to the last assessment prior to dose initiation. In contrast the animals that received 0.5 mg/kg 4-aminopyridine (low dose) significantly improved in forelimb placing (p < 0.001) compared to vehicle (see Figure 11, Day 59). The hind limb placing score improved with the low dose but did not reach significance (see Figure 12, Day 59). Increasing the dose of 4-aminopyridine to 1 mg/kg resulted in a measureable improvement in both the forelimb and hind limb tests (p < 0.001 and p < 0.05, respectively, Figures 11 and 12, Day 62) compared to vehicle. The final dose escalation to 2 mg/kg 4-aminopyridine was associated with significant improvements in both the forelimb and hind limb function (p<0.0001 and p<0.001, respectively, Figures 11 and 12, Day 65) compared to vehicle treated animals. When treatment was withdrawn for 5 days, the improvements partially declined in the raw scores, though the hind limb score remained greater than the vehicle treated group (p<0.05 , Figure 12, Day 70). It may be that the prolonged and consistent dose period requires additional time to wash out fully compared to the vehicle treated group. However, given the short serum half-life of 4-aminopyridine it appears more likely that there could be a training effect from the repeated testing that occurred in a relatively short period of time. Vehicle treated animals remained stable in their deficits, as only slight improvements were seen in vehicle treated animals during the entire course of the treatment phase.

Thus, Figure 11 indicates that the treatment with either low dose, medium dose or high dose 4-aminopyridine, 8 weeks post ischemic brain injury, is effective to improve forelimb function in rats. Figure 11 also indicates that the effect is dose-responsive. This effect is also reversible as it diminishes upon withdrawal of the drug. Figure 12 indicates that the treatment with low dose of 4-aminopyridine 8 weeks post-ischemic injury can be effective to improve hindlimb function in rats, and indicates that the treatment with medium or high dose 4-aminopyridine, 8 weeks post ischemic brain injury, is effective to improve hindlimb function in rats. Further, Figure 12 indicates that this effect is dose responsive as the treatment with a higher dose results in an improved behavioral score, relative to the treatment with a lower dose or vehicle control. Figure 12 also indicates that the effect is at least partially reversible.

The body swing performance has not been extensively characterized at the assessed time points. While there appears to be a treatment effect in the body swing performance at the first on drug assessment (Day 59) compared to the pretreatment score on Day 56, no conclusions can be drawn from the data as a whole in light of the divergence of the body swing asymmetry observed between the vehicle and 4-aminopyridine groups prior to treatment initiation (Figure 13). It is noted that the age and size of animals used in this example was considerably greater than in the study presented in Example 2, which may have played a role in the general motivation and performance ability of the animals in this particular test.

4-aminopyridine plasma levels: Blood samples drawn when the animals were receiving vehicle treatment had levels of 4-aminopyridine below the lower limit of quantitation for the method. Samples drawn when animals received 4-aminopyridine confirmed exposure at the time of behavioral testing appropriately related to dose level. 4-aminopyridine plasma levels are shown in Table 28.

**Table 28: 4-aminopyridine plasma levels**

| Treatment | Mean (SE) 4-aminopyridine Plasma Level (ng/mL) | | |
|---|---|---|---|
| | Dose Level (mg/kg) | | |
| | 0.5 | 1.0 | 2.0 |
| 4-aminopyridine | 68.3 (3.3) | 114.0 (5.5) | 184.7 (13.1) |
| Vehicle (water) | BLOQ* | BLOQ* | BLOQ* |

| | | | |
|---|---|---|---|
| SE, standard error; *BLOQ=below lower limit of quantitation (<1.0 ng/mL) | | | |

Accordingly, the data show significant reversible and dose dependent improvements in forelimb and hind limb sensorimotor function during times when 4-AP was at detectable plasma levels in the animals.

Table 29 shows that no differences in infarct volume were observed between vehicle (water) and 4-aminopyridine. SE = standard error

**Table 29:**

| Group | Mean (SE) Infarct Volume (%) |
|---|---|
| Vehicle (water) | 38.5 (2.4) |
| 4-AP | 40.0 (2.3) |

Infarct volume analysis of the brain tissue was included in the study as a typical outcome measure for preclinical stroke studies. No differences in infarct volume were observed between any groups within this study, and infarct volumes were also similar between the study presented in this example and in Example 2.

### References for Examples 2 and 17

1. Roger VL, Go AS, Lloyd-Jones DM, et al. Heart disease and stroke statistics--2012 update: a report from the American Heart Association. Circulation 2012;125:e2-e220.
2. Chida Y, Kokubo Y, Sato S, et al. The alterations of oligodendrocyte, myelin in corpus callosum, and cognitive dysfunction following chronic cerebral ischemia in rats. Brain Res 2011;1414:22-31.
3. Aboul-Enein F, Rauschka H, Kornek B, et al. Preferential loss of myelin-associated glycoprotein reflects hypoxia-like white matter damage in stroke and inflammatory brain diseases. J Neuropathol Exp Neurol 2003;62:25-33.
4. Ho PW, Reutens DC, Phan TG, et al. Is white matter involved in patients entered into typical trials of neuroprotection? Stroke 2005;36:2742-4.
5. Lindenberg R, Renga V, Zhu LL, Betzler F, Alsop D, Schlaug G. Structural integrity of corticospinal motor fibers predicts motor impairment in chronic stroke. Neurology 2010;74:280-7.
6. Liou LM, Chen CF, Guo YC, et al. Cerebral white matter hyperintensities predict functional stroke outcome. Cerebrovasc Dis 2010;29:22-7.
7. Pantoni L, Garcia JH, Gutierrez JA. Cerebral white matter is highly vulnerable to ischemia. Stroke 1996;27:1641-6.
8. Waxman SG, Kocsis JD, Stys PK. The axon: structure, function, and pathophysiology. New York: Oxford University Press; 1995.
9. Waxman SG. Ion channels and neuronal dysfunction in multiple sclerosis. Arch Neurol 2002;59:1377-80.
10. Nashmi R, Fehlings MG. Mechanisms of axonal dysfunction after spinal cord injury: with an emphasis on the role of voltage-gated potassium channels. Brain Res Brain Res Rev 2001;38:165-91.
11. Nashmi R, Fehlings MG. Changes in axonal physiology and morphology after chronic compressive injury of the rat thoracic spinal cord. Neuroscience 2001;104:235-51.
12. Targ EF, Kocsis JD. 4-Aminopyridine leads to restoration of conduction in demyelinated rat sciatic nerve. Brain Res 1985;328:358-61.
13. Sherratt RM, Bostock H, Sears TA. Effects of 4-aminopyridine on normal and demyelinated mammalian nerve fibres. Nature 1980;283:570-2.
14. Bostock H, Sears TA, Sherratt RM. The effects of 4-aminopyridine and tetraethylammonium ions on normal and demyelinated mammalian nerve fibres. J Physiol 1981;313:301-15.
15. Shi R, Blight AR. Differential effects of low and high concentrations of 4-aminopyridine on axonal conduction in normal and injured spinal cord. Neuroscience 1997;77:553-62.
16. Jensen JM, Shi R. Effects of 4-aminopyridine on stretched mammalian spinal cord: the role of potassium channels in axonal conduction. J Neurophysiol 2003;90:2334-40.
17. Blight AR. Effect of 4-aminopyridine on axonal conduction-block in chronic spinal cord injury. Brain Res Bull 1989;22:47-52.
18. Hayes KC. The use of 4-aminopyridine (fampridine) in demyelinating disorders. CNS Drug Rev 2004;10:295-316.
19. Cardenas DD, Ditunno J, Graziani V, et al. Phase 2 trial of sustained-release fampridine in chronic spinal cord injury. Spinal Cord 2007;45:158-68.
20. Goodman AD, Brown TR, Cohen JA, et al. Dose comparison trial of sustained-release fampridine in multiple sclerosis. Neurology 2008;71:1134-41.
21. Goodman AD, Brown TR, Edwards KR, et al. A phase 3 trial of extended release oral dalfampridine in multiple sclerosis. Ann Neurol 2010;68:494-502.
22. Belavic JM. Dalfampridine (Ampyra) for multiple sclerosis. Nurse Pract 2010;35:7-9.
23. Guide to the care and use of laboratory animals. 2011.
24. Tamura A, Gotoh O, Sano K. [Focal cerebral infarction in the rat: I. Operative technique and physiological monitorings for chronic model]. No To Shinkei 1986;38:747-51.
25. De Ryck M, Van Reempts J, Duytschaever H, Van Deuren B, Clincke G. Neocortical localization of tactile/proprioceptive limb placing reactions in the rat. Brain Res 1992;573:44-60.
26. Borlongan CV, Sanberg PR. Elevated body swing test: a new behavioral parameter for rats with 6-hydroxydopamine-induced hemiparkinsonism. J Neurosci 1995;15:5372-8.
27. Cramer SC. Repairing the human brain after stroke: I. Mechanisms of spontaneous recovery. Ann Neurol 2008;63:272-87.
28. Dewar D, Underhill SM, Goldberg MP. Oligodendrocytes and ischemic brain injury. J Cereb Blood Flow Metab 2003;23:263-74.
29. Petito CK, Olarte JP, Roberts B, Nowak TS, Jr., Pulsinelli WA. Selective glial vulnerability following transient global ischemia in rat brain. J Neuropathol Exp Neurol 1998;57:231-8.
30. Waxman SG. Demyelination in spinal cord injury and multiple sclerosis: what can we do to enhance functional recovery? J Neurotrauma 1992;9 Suppl 1:S105-17.
31. Waxman SG, Utzschneider DA, Kocsis JD. Enhancement of action potential conduction following demyelination: experimental approaches to restoration of function in multiple sclerosis and spinal cord injury. Prog Brain Res 1994;100:233-43.
32. Hayes KC, Kakulas BA. Neuropathology of human spinal cord injury sustained in sports-related activities. J Neurotrauma 1997;14:235-48.
33. Kakulas BA. A review of the neuropathology of human spinal cord injury with emphasis on special features. J Spinal Cord Med 1999;22:119-24.
34. Wu ZZ, Li DP, Chen SR, Pan HL. Aminopyridines potentiate synaptic and neuromuscular transmission by targeting the voltage-activated calcium channel beta subunit. J Biol Chem 2009;284:36453-61.
35. Blight AR, Toombs JP, Bauer MS, Widmer WR. The effects of 4-aminopyridine on neurological deficits in chronic cases of traumatic spinal cord injury in dogs: a phase I clinical trial. J Neurotrauma 1991;8:103-19.
36. Goodman AD, Hyland M. Dalfampridine in multiple sclerosis. Drugs Today (Bare) 2010;46:635-9.
37. Hayes KC, Katz MA, Devane JG, et al. Pharmacokinetics of an immediate-release oral formulation of Fampridine (4-aminopyridine) in normal subjects and patients with spinal cord injury. J Clin Pharmacol 2003;43:379-85.
38. Donovan WH, Halter JA, Graves DE, et al. Intravenous infusion of 4-AP in chronic spinal cord injured subjects. Spinal Cord 2000;38:7-15.
39. Brus-Ramer M, Carmel JB, Martin JH. Motor cortex bilateral motor representation depends on subcortical and interhemispheric interactions. J Neurosci 2009;29:6196-206.
40. Cramer SC, Nelles G, Benson RR, et al. A functional MRI study of subjects recovered from hemiparetic stroke. Stroke 1997;28:2518-27.
41. Dijkhuizen RM, Ren J, Mandeville JB, et al. Functional magnetic resonance imaging of reorganization in rat brain after stroke. Proc Natl Acad Sci U S A 2001;98:12766-71.

## Claims

1. An aminopyridine or a pharmaceutically acceptable salt thereof for use in treating a stroke-related sensorimotor impairment in a human patient who has suffered a stroke, wherein the aminopyridine has the following structure: 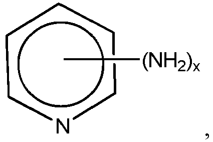 wherein x is 1 or 2.

2. The aminopyridine or pharmaceutically acceptable salt thereof for the use of claim 1, wherein the treating comprises administration of the aminopyridine or the pharmaceutically acceptable salt thereof during a stable chronic phase post-stroke.

3. The aminopyridine or pharmaceutically acceptable salt thereof for the use of claim 1, wherein the treating comprises administration of the aminopyridine or the pharmaceutically acceptable salt thereof at or after 6 months from the time the patient had a stroke.

4. The aminopyridine or pharmaceutically acceptable salt thereof for the use of any one of claims 1 to 3, wherein the stroke-related sensorimotor impairment is an impairment in walking, impairment in limb function, impairment in lower extremity function, impairment in upper extremity function, impairment in hand function, impairment in fine hand coordination, impairment in grip strength, impairment in balance or coordination, impairment in global body control, dysarthria, impairment in jaw function, impairment in chewing, or impairment in jaw articulation.

5. The aminopyridine or pharmaceutically acceptable salt thereof for the use of claim 4, wherein the stroke-related sensorimotor impairment is an impairment in walking, for example wherein the impairment in walking is decreased walking speed, such as a decreased walking speed shown by use of the Timed 25 Foot Walk (T25FW) test.

6. The aminopyridine or pharmaceutically acceptable salt thereof for the use of any one of claims 1 to 5, wherein the treating comprises administration of the aminopyridine or the pharmaceutically acceptable salt thereof beginning at least 6 months from the time the patient had a stroke, in another embodiment at least 8 months from the time the patient had a stroke.

7. The aminopyridine or pharmaceutically acceptable salt thereof for the use of any one of claims 1 to 5, wherein the treating comprises administration of the aminopyridine or the pharmaceutically acceptable salt thereof beginning at least 1 week from the time the patient had a stroke, at least 4 weeks from the time the patient had a stroke, at least 8 weeks from the time the patient had a stroke, or at least 4 months from the time the patient had a stroke.

8. The aminopyridine or pharmaceutically acceptable salt thereof for the use of any one of claims 1 to 5, wherein the treating comprises administration of the aminopyridine or the pharmaceutically acceptable salt thereof beginning between 2 and 7 days from the time the patient had a stroke, in another embodiment the treating comprises administration of the aminopyridine or the pharmaceutically acceptable salt thereof beginning within 6 hours or within 2 days from the time the patient had a stroke.

9. The aminopyridine or pharmaceutically acceptable salt thereof for the use of any preceding claim, wherein the aminopyridine is a mono-aminopyridine.

10. The aminopyridine or pharmaceutically acceptable salt thereof for the use of any preceding claim, wherein the aminopyridine is 4-aminopyridine.

11. The aminopyridine or pharmaceutically acceptable salt thereof for the use of any preceding claim, wherein the aminopyridine or the pharmaceutically acceptable salt thereof is in a sustained release composition, for example wherein said sustained release composition provides a Tₘₐₓ of about 2 hours to about 6 hours in a human.

12. The aminopyridine or pharmaceutically acceptable salt thereof for the use of any preceding claim, wherein the aminopyridine or the pharmaceutically acceptable salt thereof is to be administered orally, for example wherein the aminopyridine or the pharmaceutically acceptable salt thereof is formulated in a form of a tablet.

13. The aminopyridine or pharmaceutically acceptable salt thereof for the use of any preceding claim,
wherein the aminopyridine or the pharmaceutically acceptable salt thereof is to be administered to the patient once daily or twice daily; and/or
wherein the aminopyridine is for twice daily oral administration in a sustained release composition in an amount in the range of 4 to 17.5 mg, for example 12.5 mg; 5 to 15 mg, for example 5 mg; 5 to 10 mg, for example 10 mg; or 7.5 to 10 mg, for example 7.5 mg; and/or
wherein the aminopyridine is for once daily oral administration in a sustained release composition in an amount in the range of 8 to 30 mg; 10 to 30 mg, for example 10 mg; 10 to 20 mg; or 15 to 20 mg, for example 15 mg; and/or
wherein the aminopyridine or the pharmaceutically acceptable salt thereof is in an amount that obtains a Cₘᵢₙₛₛ or average Cₘᵢₙₛₛ of at least about 11, 12, 13, 14, 15, 16, 17, 18, 19 or 20 ng/ml when administered to a human; and/or
wherein the aminopyridine or the pharmaceutically acceptable salt thereof is in an amount that obtains a Cₘᵢₙₛₛ or average Cₘᵢₙₛₛ in a range of about 12 ng/ml to 20 ng/ml when administered to a human.

14. The aminopyridine or pharmaceutically acceptable salt thereof for the use of any preceding claim, wherein the aminopyridine or the pharmaceutically acceptable salt thereof is comprised in a composition that does not further comprise choline or a source of choline.

15. The aminopyridine or pharmaceutically acceptable salt thereof for the use of any preceding claim, wherein the stroke is an ischemic stroke.

16. The aminopyridine or pharmaceutically acceptable salt thereof for the use of any one of claims 1 to 3, wherein the aminopyridine is 4-aminopyridine in a sustained release composition for oral administration, for example in the amount of 10 mg for twice daily administration, and wherein the use is for improving walking in a human patient who has suffered an ischemic stroke and has an impairment in walking, for example wherein the use in improving walking is use in increasing walking speed.

17. The aminopyridine or pharmaceutically acceptable salt thereof for the use of any preceding claim, wherein the aminopyridine or pharmaceutically acceptable salt thereof is the aminopyridine and not the pharmaceutically acceptable salt thereof.

## Patentansprüche

1. Aminopyridin oder pharmazeutisch unbedenkliches Salz davon zur Verwendung bei der Behandlung einer mit Schlaganfall in Zusammenhang stehenden sensorimotorischen Störung in einem humanen Patienten, der einen Schlaganfall erlitten hat, wobei das Aminopyridin die folgende Struktur aufweist: 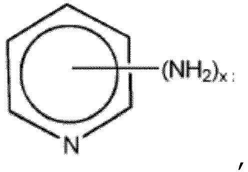 wobei x für 1 oder 2 steht.

2. Aminopyridin oder pharmazeutisch unbedenkliches Salz davon zur Verwendung nach Anspruch 1, wobei die Behandlung die Verabreichung des Aminopyridins oder des pharmazeutisch unbedenklichen Salzes davon während einer stabilen chronischen Phase nach dem Schlaganfall umfasst.

3. Aminopyridin oder pharmazeutisch unbedenkliches Salz davon zur Verwendung nach Anspruch 1, wobei die Behandlung die Verabreichung des Aminopyridins oder des pharmazeutisch unbedenklichen Salzes davon zu dem Zeitpunkt, an dem der Patient einen Schlaganfall erlitten hat, oder 6 Monate danach umfasst.

4. Aminopyridin oder pharmazeutisch unbedenkliches Salz davon zur Verwendung nach einem der Ansprüche 1 bis 3, wobei es sich bei der mit einem Schlaganfall in Zusammenhang stehenden sensorimotorischen Störung um eine Gehstörung, eine Störung der Funktion der Gliedmaßen, eine Störung der Funktion der unteren Extremitäten, eine Störung der Funktion der oberen Extremitäten, eine Störung der Handfunktion, eine Störung der feinen Handkoordination, eine Störung der Griffstärke, eine Störung von Gleichgewicht oder Koordination, eine Störung der allgemeinen Körperkontrolle, Dysarthrie, eine Störung der Kieferfunktion, eine Störung beim Kauen oder eine Störung bei der Kieferartikulation handelt.

5. Aminopyridin oder pharmazeutisch unbedenkliches Salz davon zur Verwendung nach Anspruch 4, wobei es sich bei der mit einem Schlaganfall in Zusammenhang stehenden sensorimotorischen Störung um eine Gehstörung handelt, wobei es sich bei der Gehstörung beispielsweise um eine verminderte Gehgeschwindigkeit wie eine durch Anwendung des Timed 25 Foot Walk (T25FW)-Tests aufgezeigte verminderte Gehgeschwindigkeit handelt.

6. Aminopyridin oder pharmazeutisch unbedenkliches Salz davon zur Verwendung nach einem der Ansprüche 1 bis 5, wobei die Behandlung die Verabreichung des Aminopyridins oder des pharmazeutisch unbedenklichen Salzes davon beginnend mindestens 6 Monate von dem Zeitpunkt, an dem der Patient einen Schlaganfall erlitten hat, gemäß einer anderen Ausführungsform mindestens 8 Monate von dem Zeitpunkt, an dem der Patient einen Schlaganfall erlitten hat, umfasst.

7. Aminopyridin oder pharmazeutisch unbedenkliches Salz davon zur Verwendung nach einem der Ansprüche 1 bis 5, wobei die Behandlung die Verabreichung des Aminopyridins oder des pharmazeutisch unbedenklichen Salzes davon beginnend mindestens eine Woche von dem Zeitpunkt, an dem der Patient einen Schlaganfall erlitten hat, mindestens 4 Wochen von Zeitpunkt, an dem der Patient einen Schlaganfall erlitten hat, mindestens 8 Wochen von dem Zeitpunkt, an dem der Patient einen Schlaganfall erlitten hat, oder mindestens 4 Monate von dem Zeitpunkt, an dem der Patient einen Schlaganfall erlitten hat, umfasst.

8. Aminopyridin oder pharmazeutisch unbedenkliches Salz davon zur Verwendung nach einem der Ansprüche 1 bis 5, wobei die Behandlung die Verabreichung des Aminopyridins oder des pharmazeutisch unbedenklichen Salzes davon beginnend zwischen 2 und 7 Tagen von dem Zeitpunkt, an dem der Patient einen Schlaganfall erlitten hat, umfasst; gemäß einer anderen Ausführungsform umfasst die Behandlung die Verabreichung des Aminopyridins oder des pharmazeutisch unbedenklichen Salzes davon beginnend innerhalb von 6 Stunden oder innerhalb von 2 Tagen von dem Zeitpunkt, an dem der Patient einen Schlaganfall erlitten hat.

9. Aminopyridin oder pharmazeutisch unbedenkliches Salz davon zur Verwendung nach einem der vorhergehenden Ansprüche, wobei es sich bei dem Aminopyridin um ein Monoaminopyridin handelt.

10. Aminopyridin oder pharmazeutisch unbedenkliches Salz davon zur Verwendung nach einem der vorhergehenden Ansprüche, wobei es sich bei dem Aminopyridin um 4-Aminopyridin handelt.

11. Aminopyridin oder pharmazeutisch unbedenkliches Salz davon zur Verwendung nach einem der vorhergehenden Ansprüche, wobei das Aminopyridin oder das pharmazeutisch unbedenkliche Salz davon in einer Zusammensetzung mit anhaltender Freisetzung vorliegt, wobei die Zusammensetzung mit anhaltender Freisetzung beispielsweise in einem Menschen eine Tₘₐₓ von etwa 2 Stunden bis etwa 6 Stunden bereitstellt.

12. Aminopyridin oder pharmazeutisch unbedenkliches Salz davon zur Verwendung nach einem der vorhergehenden Ansprüche, wobei das Aminopyridin oder das pharmazeutisch unbedenkliche Salz davon oral zu verabreichen ist, wobei das Aminopyridin oder das pharmazeutisch unbedenkliche Salz davon beispielsweise in Form einer Tablette formuliert ist.

13. Aminopyridin oder pharmazeutisch unbedenkliches Salz davon zur Verwendung nach einem der vorhergehenden Ansprüche,
wobei das Aminopyridin oder das pharmazeutisch unbedenkliche Salz davon dem Patienten einmal täglich oder zweimal täglich zu verabreichen ist und/oder wobei das Aminopyridin für die zweimal tägliche orale Verabreichung in einer Zusammensetzung mit anhaltender Freisetzung in einer Menge im Bereich von 4 bis 17,5 mg, zum Beispiel 12,5 mg, 5 bis 15 mg, zum Beispiel 5 mg, 5 bis 10 mg, zum Beispiel 10 mg, oder 7,5 bis 10 mg, zum Beispiel 7,5 mg, bestimmt ist und/oder
wobei das Aminopyridin für die einmal tägliche orale Verabreichung in einer Zusammensetzung mit anhaltender Freisetzung in einer Menge im Bereich von 8 bis 30 mg, 10 bis 30 mg, zum Beispiel 10 mg, 10 bis 20 mg oder 15 bis 20 mg, zum Beispiel 15 mg, bestimmt ist und/oder wobei das das Aminopyridin oder das pharmazeutisch unbedenkliche Salz davon in einer Menge vorliegt, mit der sich bei der Verabreichung an einen Menschen eine Cₘᵢₙₛₛ oder durchschnittliche Cₘᵢₙₛₛ von mindestens etwa 11, 12, 13, 14, 15, 16, 17, 18, 19 oder 20 ng/ml erzielen lässt und/oder
wobei das Aminopyridin oder das pharmazeutisch unbedenkliche Salz davon in einer Menge vorliegt, mit der sich bei der Verabreichung an einen Menschen eine Cₘᵢₙₛₛ oder durchschnittliche Cₘᵢₙₛₛ im Bereich von etwa 12 ng/ml bis 20 ng/ml erzielen lässt.

14. Aminopyridin oder pharmazeutisch unbedenkliches Salz davon zur Verwendung nach einem der vorhergehenden Ansprüche, wobei das Aminopyridin oder das pharmazeutisch unbedenkliche Salz davon in einer Zusammensetzung umfasst ist, die nicht weiterhin Cholin oder eine Cholinquelle umfasst.

15. Aminopyridin oder pharmazeutisch unbedenkliches Salz davon zur Verwendung nach einem der vorhergehenden Ansprüche, wobei es sich bei dem Schlaganfall um einen ischämischen Schlaganfall handelt.

16. Aminopyridin oder pharmazeutisch unbedenkliches Salz davon zur Verwendung nach einem der Ansprüche 1 bis 3, wobei es sich bei dem Aminopyridin um 4-Aminopyridin in einer Zusammensetzung mit anhaltender Freisetzung zur oralen Verabreichung handelt, beispielsweise in einer Menge von 10 mg für die zweimal tägliche Verabreichung, und wobei die Anwendung zur Verbesserung des Gehens bei einem humanen Patienten, der einen ischämischen Schlaganfall erlitten hat und eine Gehstörung aufweist, bestimmt ist, wobei es sich bei der Anwendung zum Verbessern des Gehens beispielsweise um eine Anwendung zum Erhöhen der Gehgeschwindigkeit handelt.

17. Aminopyridin oder pharmazeutisch unbedenkliches Salz davon zur Verwendung nach einem der vorhergehenden Ansprüche, wobei das Aminopyridin oder das pharmazeutisch unbedenkliche Salz davon das Aminopyridin und nicht das pharmazeutisch unbedenkliche Salz davon ist.

## Revendications

1. Aminopyridine ou sel pharmaceutiquement acceptable de celle-ci pour utilisation dans le traitement d'un trouble sensorimoteur lié à un accident vasculaire cérébral chez un patient humain qui a souffert d'un accident vasculaire cérébral, l'aminopyridine ayant la structure suivante : 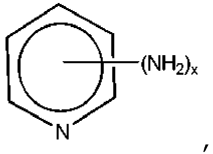 dans laquelle x est 1 ou 2.

2. Aminopyridine ou sel pharmaceutiquement acceptable de celle-ci pour l'utilisation selon la revendication 1, le traitement comprenant l'administration de l'aminopyridine ou du sel pharmaceutiquement acceptable de celle-ci pendant une phase chronique stable post-accident vasculaire cérébral.

3. Aminopyridine ou sel pharmaceutiquement acceptable de celle-ci pour l'utilisation selon la revendication 1, le traitement comprenant l'administration de l'aminopyridine ou du sel pharmaceutiquement acceptable de celle-ci 6 mois ou plus de 6 mois après le moment auquel le patient a subi un accident vasculaire cérébral.

4. Aminopyridine ou sel pharmaceutiquement acceptable de celle-ci pour l'utilisation selon l'une quelconque des revendications 1 à 3, le trouble sensorimoteur lié à un accident vasculaire cérébral étant une altération de la marche, une altération de la fonction des membres, une altération de la fonction des extrémités inférieures, une altération de la fonction des extrémités supérieures, une altération de la fonction des mains, une altération de la coordination fine des mains, une altération de la force de préhension, une altération de l'équilibre ou de la coordination, une altération du contrôle global du corps, une dysarthrie, une altération de la fonction des mâchoires, une altération de la mastication ou une altération de l'articulation des mâchoires.

5. Aminopyridine ou sel pharmaceutiquement acceptable de celle-ci pour l'utilisation selon la revendication 4, le trouble sensorimoteur lié à un accident vasculaire cérébral étant une altération de la marche, par exemple, l'altération de la marche étant une vitesse de la marche réduite, telle qu'une vitesse de la marche réduite mise en évidence au moyen du test de marche de 25 pieds (T25FW).

6. Aminopyridine ou sel pharmaceutiquement acceptable de celle-ci pour l'utilisation selon l'une quelconque des revendications 1 à 5, le traitement comprenant l'administration de l'aminopyridine ou du sel pharmaceutiquement acceptable de celle-ci commençant au moins 6 mois après le moment auquel le patient a subi un accident vasculaire cérébral, dans un autre mode de réalisation au moins 8 mois après le moment auquel le patient a subi un accident vasculaire cérébral.

7. Aminopyridine ou sel pharmaceutiquement acceptable de celle-ci pour l'utilisation selon l'une quelconque des revendications 1 à 5, le traitement comprenant l'administration de l'aminopyridine ou du sel pharmaceutiquement acceptable de celle-ci commençant au moins 1 semaine après le moment auquel le patient a subi un accident vasculaire cérébral, au moins 4 semaines après le moment auquel le patient a subi un accident vasculaire cérébral, au moins 8 semaines après le moment auquel le patient a subi un accident vasculaire cérébral, ou au moins 4 mois après le moment auquel le patient a subi un accident vasculaire cérébral.

8. Aminopyridine ou sel pharmaceutiquement acceptable de celle-ci pour l'utilisation selon l'une quelconque des revendications 1 à 5, le traitement comprenant l'administration de l'aminopyridine ou du sel pharmaceutiquement acceptable de celle-ci commençant entre 2 et 7 jours après le moment auquel le patient a subi un accident vasculaire cérébral, dans un autre mode de réalisation le traitement comprenant l'administration de l'aminopyridine ou du sel pharmaceutiquement acceptable de celle-ci commençant dans les 6 heures ou dans les 2 jours après le moment auquel le patient a subi un accident vasculaire cérébral.

9. Aminopyridine ou sel pharmaceutiquement acceptable de celle-ci pour l'utilisation selon l'une quelconque des revendications précédentes, l'aminopyridine étant une mono-aminopyridine.

10. Aminopyridine ou sel pharmaceutiquement acceptable de celle-ci pour l'utilisation selon l'une quelconque des revendications précédentes, l'aminopyridine étant la 4-aminopyridine.

11. Aminopyridine ou sel pharmaceutiquement acceptable de celle-ci pour l'utilisation selon l'une quelconque des revendications précédentes, l'aminopyridine ou le sel pharmaceutiquement acceptable de celle-ci étant dans une composition à libération prolongée, par exemple, ladite composition à libération prolongée produisant un Tₘₐₓ d'environ 2 heures à environ 6 heures chez un humain.

12. Aminopyridine ou sel pharmaceutiquement acceptable de celle-ci pour l'utilisation selon l'une quelconque des revendications précédentes, l'aminopyridine ou le sel pharmaceutiquement acceptable de celle-ci étant destinée à être administrée par voie orale, par exemple, l'aminopyridine ou le sel pharmaceutiquement acceptable de celle-ci étant formulée sous la forme d'un comprimé.

13. Aminopyridine ou sel pharmaceutiquement acceptable de celle-ci pour l'utilisation selon l'une quelconque des revendications précédentes,
l'aminopyridine ou le sel pharmaceutiquement acceptable de celle-ci étant destinée à être administrée au patient une fois par jour ou deux fois par jour ; et/ou
l'aminopyridine étant pour administration orale deux fois par jour dans une composition à libération prolongée en une quantité dans la plage de 4 à 17,5 mg, par exemple 12,5 mg ; 5 à 15 mg, par exemple 5 mg ; 5 à 10 mg, par exemple 10 mg ; ou 7,5 à 10 mg, par exemple 7,5 mg ; et/ou
l'aminopyridine étant pour administration orale une fois par jour dans une composition à libération prolongée en une quantité dans la plage de 8 à 30 mg ; 10 à 30 mg, par exemple 10 mg ; 10 à 20 mg ; ou 15 à 20 mg, par exemple 15 mg ; et/ou
l'aminopyridine ou le sel pharmaceutiquement acceptable de celle-ci étant en une quantité qui permet d'obtenir une Cₘᵢₙₛₛ ou une Cₘᵢₙₛₛ moyenne d'au moins environ 11, 12, 13, 14, 15, 16, 17, 18, 19 ou 20 ng/ml lorsqu'elle est administrée à un humain ; et/ou
l'aminopyridine ou le sel pharmaceutiquement acceptable de celle-ci étant en une quantité qui permet d'obtenir une Cₘᵢₙₛₛ ou une Cₘᵢₙₛₛ moyenne dans une plage d'environ 12 ng/ml à 20 ng/ml lorsqu'elle est administrée à un humain.

14. Aminopyridine ou sel pharmaceutiquement acceptable de celle-ci pour l'utilisation selon l'une quelconque des revendications précédentes, l'aminopyridine ou le sel pharmaceutiquement acceptable de celle-ci étant comprise dans une composition qui ne comprend pas en outre de la choline ou une source de choline.

15. Aminopyridine ou sel pharmaceutiquement acceptable de celle-ci pour l'utilisation selon l'une quelconque des revendications précédentes, l'accident vasculaire cérébral étant un accident vasculaire cérébral ischémique.

16. Aminopyridine ou sel pharmaceutiquement acceptable de celle-ci pour l'utilisation selon l'une quelconque des revendications 1 à 3, l'aminopyridine étant la 4-aminopyridine dans une composition à libération prolongée pour administration orale, par exemple en une quantité de 10 mg pour administration deux fois par jour, et l'utilisation étant pour améliorer la marche chez un patient humain qui a souffert d'un accident vasculaire cérébral ischémique et présente une altération de la marche, par exemple, l'utilisation dans l'amélioration de la marche étant une utilisation dans l'augmentation de la vitesse de la marche.

17. Aminopyridine ou sel pharmaceutiquement acceptable de celle-ci pour l'utilisation selon l'une quelconque des revendications précédentes, l'aminopyridine ou le sel pharmaceutiquement acceptable de celle-ci étant l'aminopyridine et non le sel pharmaceutiquement acceptable de celle-ci.
